# EUROPEAN PATENT SPECIFICATION

(11) **EP 2 906 241 B1**
(45) Date of publication and mention of the grant of the patent: **08.01.2020**
(21) Application number: 13845573.8
(22) Date of filing: 11.10.2013
(51) Int. Cl.: A61K 39/395, A61K 31/7088, A61P 35/00, A61P 31/12, A61P 37/00

(54) **ENHANCEMENT OF THE IMMUNE RESPONSE**
VERBESSERUNG DER IMMUNREAKTION
RENFORCEMENT DE LA RÉPONSE IMMUNITAIRE

(30) Priority: 12.10.2012 US 201261713203 P
(43) Date of publication of application: 19.08.2015
(73) Proprietor: The Brigham and Women's Hospital, Inc., Boston, MA 02115 (US)
(72) Inventor: BLUMBERG, Richard S., Waltham Massachusetts 02451 (US); HUANG, Yu-Hwa, Boston Massachusetts 02130 (US)
(74) Representative: Sweetinburgh, Mark Roger
(86) International application number: PCT/US2013/064506
(87) International publication number: WO 2014/059251

(56) References cited:
- US-A1- 2009 226 435
- US-B2- 7 132 255
- US-B2- 8 198 412
- C.-J. CHEN ET AL: "The Cell-Cell Adhesion Molecule Carcinoembryonic Antigen-Related Cellular Adhesion Molecule 1 Inhibits IL-2 Production and Proliferation in Human T Cells by Association with Src Homology Protein-1 and Down-Regulates IL-2 Receptor", THE JOURNAL OF IMMUNOLOGY, vol. 172, no. 6, 15 March 2004 (2004-03-15), pages 3544-3552, XP055265350, US ISSN: 0022-1767, DOI: 10.4049/jimmunol.172.6.3544
- T. NAGAISHI ET AL: "Role of CEACAM1 as a Regulator of T Cells", ANNALS OF THE NEW YORK ACADEMY OF SCIENCES, vol. 1072, no. 1, 1 August 2006 (2006-08-01), pages 155-175, XP055265409, US ISSN: 0077-8923, DOI: 10.1196/annals.1326.004
- L. M. FRANCISCO ET AL: "PD-L1 regulates the development, maintenance, and function of induced regulatory T cells", MOLECULAR IMMUNOLOGY, vol. 206, no. 13, 14 December 2009 (2009-12-14), pages 3015-3029, XP055070413, ISSN: 0161-5890, DOI: 10.1016/j.molimm.2007.08.013
- SÜ LEYMAN ERGÜ ET AL: "CEA-Related Cell Adhesion Molecule 1: A Potent Angiogenic Factor and a Major Effector of Vascular Endothelial Growth Factor", MOLECULAR CELL, vol. 5, 1 February 2000 (2000-02-01), pages 311-320, XP055265510,
- STUART RODNEY W ET AL: "Targeting T cell costimulation in autoimmune disease", EXPERT OPINION ON THERAPEUTIC TARGETS, INFORMA HEALTHCARE, GB, vol. 6, no. 3, 1 June 2002 (2002-06-01), pages 275-289, XP009125144, ISSN: 1744-7631, DOI: 10.1517/14728222.6.3.275
- ALI NA, GAUGHAN AA, OROSZ CG, BARAN CP, MCMAKEN S, WANG Y, ET AL: "Latency Associated Peptide Has In Vitro and In Vivo Immune Effects Independent of TGF-beta1", PLOS ONE, vol. 3, no. 4, E1914, 2 April 2008 (2008-04-02), pages 1-9, XP002756628, DOI: 10.1371/journal.pone.0001914
- CURRAN MICHAEL A. ET AL.: 'PD-1 and CTLA-4 combination blockade expands infiltrating T cells and reduces regulatory T and myeloid cells within B 16 melanoma tumors.' PNAS vol. 107, no. 9, 2010, pages 4275 - 4280, XP055067204
- MELERO IGNACIO ET AL.: 'Immunostimulatory monoclonal antibodies for cancer therapy.' NATURE REVIEWS I CANCER vol. 7, 2007, pages 95 - 106, XP002716002
- GRAY JULIET C. ET AL.: 'Optimising anti-tumour CD8 T-cell responses using combinations of immunomodulatory antibodies.' EUR. J. IMMUNOL vol. 38, 2008, , pages 2499 - 2511, XP055151985
- CHEN MEI-LING ET AL.: 'Latency-Associated Peptide Identifies a Novel CD4+CD25+Regulatory T Cell Subset with TGF-beta Mediated Function and Enhanced Suppression of Experimental Autoimmune Encephalomyelitis.' J IMMUNOL vol. 180, no. 11, 2008, pages 7327 - 7337, XP055246475
- SIVAN SAPOZNIK ET AL: "Novel Anti-Melanoma Immunotherapies: Disarming Tumor Escape Mechanisms", CLINICAL & DEVELOPMENTAL IMMUNOLOGY, vol. 1, no. 1, 1 January 2012 (2012-01-01) , pages 1-9, XP055249166, US ISSN: 1740-2522, DOI: 10.1155/2012/818214

### SEQUENCE LISTING

<110> THE BRIGHAM AND WOMEN'S HOSPITAL, INC.
<120> ENHANCEMENT OF THE IMMUNE RESPONSE
<130> 043214-075201-PCT
<140>
   <141>
<150> 61/713,203
   <151> 2012-10-12
<160> 38
<170> PatentIn version 3.5
<210> 1
   <211> 288
   <212> PRT
   <213> Homo sapiens
<400> 1
   Met Gln Ile Pro Gln Ala Pro Trp Pro Val Val Trp Ala Val Leu Gln
   1               5                   10                  15
   Leu Gly Trp Arg Pro Gly Trp Phe Leu Asp Ser Pro Asp Arg Pro Trp
               20                  25                  30
   Asn Pro Pro Thr Phe Ser Pro Ala Leu Leu Val Val Thr Glu Gly Asp
           35                  40                  45
   Asn Ala Thr Phe Thr Cys Ser Phe Ser Asn Thr Ser Glu Ser Phe Val
       50                  55                  60
   Leu Asn Trp Tyr Arg Met Ser Pro Ser Asn Gln Thr Asp Lys Leu Ala
   65                  70                  75                  80
   Ala Phe Pro Glu Asp Arg Ser Gln Pro Gly Gln Asp Cys Arg Phe Arg
                   85                  90                  95
   Val Thr Gln Leu Pro Asn Gly Arg Asp Phe His Met Ser Val Val Arg
               100                 105                 110
   Ala Arg Arg Asn Asp Ser Gly Thr Tyr Leu Cys Gly Ala Ile Ser Leu
           115                 120                 125
   Ala Pro Lys Ala Gln Ile Lys Glu Ser Leu Arg Ala Glu Leu Arg Val
       130                 135                 140
   Thr Glu Arg Arg Ala Glu Val Pro Thr Ala His Pro Ser Pro Ser Pro
   145                 150                 155                 160
   Arg Pro Ala Gly Gln Phe Gln Thr Leu Val Val Gly Val Val Gly Gly
                   165                 170                 175
   Leu Leu Gly Ser Leu Val Leu Leu Val Trp Val Leu Ala Val Ile Cys
               180                 185                 190
   Ser Arg Ala Ala Arg Gly Thr Ile Gly Ala Arg Arg Thr Gly Gln Pro
           195                 200                 205
   Leu Lys Glu Asp Pro Ser Ala Val Pro Val Phe Ser Val Asp Tyr Gly
       210                 215                 220
   Glu Leu Asp Phe Gln Trp Arg Glu Lys Thr Pro Glu Pro Pro Val Pro
   225                 230                 235                 240
   Cys Val Pro Glu Gln Thr Glu Tyr Ala Thr Ile Val Phe Pro Ser Gly
                   245                 250                 255
   Met Gly Thr Ser Ser Pro Ala Arg Arg Gly Ser Ala Asp Gly Pro Arg
               260                 265                 270
   Ser Ala Gln Pro Leu Arg Pro Glu Asp Gly His Cys Ser Trp Pro Leu
           275                 280                 285
<210> 2
   <211> 6
   <212> PRT
   <213> Artificial Sequence
<220>
   <223> Description of Artificial Sequence: Synthetic peptide
<400> 2
   Ser Ser His Gly Met Ser
   1               5
<210> 3
   <211> 17
   <212> PRT
   <213> Artificial Sequence
<220>
   <223> Description of Artificial Sequence: Synthetic peptide
<400> 3
   Thr Ile Ser Ser Gly Gly Thr Tyr Thr Tyr Tyr Pro Asp Ser Val Lys
   1               5                   10                  15
   Gly
<210> 4
   <211> 12
   <212> PRT
   <213> Artificial Sequence
<220>
   <223> Description of Artificial Sequence: Synthetic peptide
<400> 4
   His Asp Phe Asp Tyr Asp Ala Ala Trp Phe Ala Tyr
   1               5                   10
<210> 5
   <211> 10
   <212> PRT
   <213> Artificial Sequence
<220>
   <223> Description of Artificial Sequence: Synthetic peptide
<400> 5
   Ser Ala Asn Ser Ser Val Ser Tyr Met Tyr
   1               5                   10
<210> 6
   <211> 7
   <212> PRT
   <213> Artificial Sequence
<220>
   <223> Description of Artificial Sequence: Synthetic peptide
<400> 6
   Leu Thr Ser Asn Leu Ala Ser
   1               5
<210> 7
   <211> 9
   <212> PRT
   <213> Artificial Sequence
<220>
   <223> Description of Artificial Sequence: Synthetic peptide
<400> 7
   Gln Gln Trp Ser Ser Asn Pro Pro Thr
   1               5
<210> 8
   <211> 6
   <212> PRT
   <213> Artificial Sequence
<220>
   <223> Description of Artificial Sequence: Synthetic peptide
<400> 8
   Ser Ser His Gly Met Ser
   1               5
<210> 9
   <211> 6
   <212> PRT
   <213> Artificial Sequence
<220>
   <223> Description of Artificial Sequence: Synthetic peptide
<400> 9
   Ser Phe Tyr Gly Met Ser
   1               5
<210> 10
   <211> 6
   <212> PRT
   <213> Artificial Sequence
<220>
   <223> Description of Artificial Sequence: Synthetic peptide
<400> 10
   Ser Asp Tyr Tyr Leu Tyr
   1               5
<210> 11
   <211> 17
   <212> PRT
   <213> Artificial Sequence
<220>
   <223> Description of Artificial Sequence: Synthetic peptide
<400> 11
   Thr Ile Ser Ser Gly Gly Thr Tyr Thr Tyr Tyr Pro Asp Ser Val Lys
   1               5                   10                  15
   Gly
<210> 12
   <211> 17
   <212> PRT
   <213> Artificial Sequence
<220>
   <223> Description of Artificial Sequence: Synthetic peptide
<400> 12
   Thr Phe Ser Gly Gly Gly Asn Tyr Thr Tyr Tyr Pro Asp Ser Val Lys
   1 5 10 15
   Gly
<210> 13
   <211> 17
   <212> PRT
   <213> Artificial Sequence
<220>
   <223> Description of Artificial Sequence: Synthetic peptide
<400> 13
   Thr Ile Ser Val Gly Gly Gly Asn Thr Ser Tyr Pro Asp Ser Val Lys
   1 5 10 15
   Gly
<210> 14
   <211> 12
   <212> PRT
   <213> Artificial Sequence
<220>
   <223> Description of Artificial Sequence: Synthetic peptide
<400> 14
   His Asp Phe Asp Tyr Asp Ala Ala Trp Phe Ala Tyr
   1 5 10
<210> 15
   <211> 11
   <212> PRT
   <213> Artificial Sequence
<220>
   <223> Description of Artificial Sequence: Synthetic peptide
<400> 15
   His Gly Gly Leu Pro Phe Tyr Ala Met Asp Tyr
   1 5 10
<210> 16
   <211> 11
   <212> PRT
   <213> Artificial Sequence
<220>
   <223> Description of Artificial Sequence: Synthetic peptide
<400> 16
   Gly Leu Thr Thr Gly Pro Ala Trp Phe Ala Tyr
   1 5 10
<210> 17
   <211> 10
   <212> PRT
   <213> Artificial Sequence
<220>
   <223> Description of Artificial Sequence: Synthetic peptide
<400> 17
   Ser Ala Asn Ser Ser Val Ser Tyr Met Tyr
   1 5 10
<210> 18
   <211> 12
   <212> PRT
   <213> Artificial Sequence
<220>
   <223> Description of Artificial Sequence: Synthetic peptide
<400> 18
   Ser Val Ser Ser Ser Ile Ser Ser Ser Asn Leu His
   1 5 10
<210> 19
   <211> 17
   <212> PRT
   <213> Artificial Sequence
<220>
   <223> Description of Artificial Sequence: Synthetic peptide
<400> 19
   Lys Ser Ser Gln Ser Leu Leu Asn Ser Ser Asn Gln Lys Asn Tyr Leu
   1 5 10 15
   Ala
<210> 20
   <211> 11
   <212> PRT
   <213> Artificial Sequence
<220>
   <223> Description of Artificial Sequence: Synthetic peptide
<400> 20
   Arg Ala Ser Gln Lys Ile Ser Gly Tyr Leu Ser
   1 5 10
<210> 21
   <211> 7
   <212> PRT
   <213> Artificial Sequence
<220>
   <223> Description of Artificial Sequence: Synthetic peptide
<400> 21
   Leu Thr Ser Asn Leu Ala Ser
   1               5
<210> 22
   <211> 12
   <212> PRT
   <213> Artificial Sequence
<220>
   <223> Description of Artificial Sequence: Synthetic peptide
<400> 22
   Ser Val Ser Ser Ser Ile Ser Ser Ser Asn Leu His
   1               5                   10
<210> 23
   <211> 7
   <212> PRT
   <213> Artificial Sequence
<220>
   <223> Description of Artificial Sequence: Synthetic peptide
<400> 23
   Phe Ala Ser Thr Arg Glu Ser
   1               5
<210> 24
   <211> 7
   <212> PRT
   <213> Artificial Sequence
<220>
   <223> Description of Artificial Sequence: Synthetic peptide
<400> 24
   Ala Ala Ser Thr Leu Asp Ser
   1               5
<210> 25
   <211> 9
   <212> PRT
   <213> Artificial Sequence
<220>
   <223> Description of Artificial Sequence: Synthetic peptide
<400> 25
   Gln Gln Trp Ser Ser Asn Pro Pro Thr
   1               5
<210> 26
   <211> 9
   <212> PRT
   <213> Artificial Sequence
<220>
   <223> Description of Artificial Sequence: Synthetic peptide
<400> 26
   Gln Gln Trp Ser Ser His Pro Phe Thr
   1               5
<210> 27
   <211> 9
   <212> PRT
   <213> Artificial Sequence
<220>
   <223> Description of Artificial Sequence: Synthetic peptide
<400> 27
   Gln Gln His Tyr Ser Thr Pro Trp Thr
   1               5
<210> 28
   <211> 10
   <212> PRT
   <213> Artificial Sequence
<220>
   <223> Description of Artificial Sequence: Synthetic peptide
<400> 28
   Leu Gln Tyr Ala Ser Ser Leu Met Tyr Thr
   1               5                   10
<210> 29
   <211> 351
   <212> PRT
   <213> Homo sapiens
<400> 29
   Met Gly His Leu Ser Ala Pro Leu His Arg Val Arg Val Pro Trp Gln
   1               5                   10                  15
   Gly Leu Leu Leu Thr Ala Ser Leu Leu Thr Phe Trp Asn Pro Pro Thr
               20                  25                  30
   Thr Ala Gln Leu Thr Thr Glu Ser Met Pro Phe Asn Val Ala Glu Gly
           35                  40                  45
   Lys Glu Val Leu Leu Leu Val His Asn Leu Pro Gln Gln Leu Phe Gly
       50                  55                  60
   Tyr Ser Trp Tyr Lys Gly Glu Arg Val Asp Gly Asn Arg Gln Ile Val
   65                  70                  75                  80
   Gly Tyr Ala Ile Gly Thr Gln Gln Ala Thr Pro Gly Pro Ala Asn Ser
                   85                  90                  95
   Gly Arg Glu Thr Ile Tyr Pro Asn Ala Ser Leu Leu Ile Gln Asn Val
               100                 105                 110
   Thr Gln Asn Asp Thr Gly Phe Tyr Thr Leu Gln Val Ile Lys Ser Asp
           115                 120                 125
   Leu Val Asn Glu Glu Ala Thr Gly Gln Phe His Val Tyr Pro Glu Leu
       130                 135                 140
   Pro Lys Pro Ser Ile Ser Ser Asn Asn Ser Asn Pro Val Glu Asp Lys
   145                 150                 155                 160
   Asp Ala Val Ala Phe Thr Cys Glu Pro Glu Thr Gln Asp Thr Thr Tyr
                   165                 170                 175
   Leu Trp Trp Ile Asn Asn Gln Ser Leu Pro Val Ser Pro Arg Leu Gln
               180                 185                 190
   Leu Ser Asn Gly Asn Arg Thr Leu Thr Leu Leu Ser Val Thr Arg Asn
           195                 200                 205
   Asp Thr Gly Pro Tyr Glu Cys Glu Ile Gln Asn Pro Val Ser Ala Asn
       210                 215                 220
   Arg Ser Asp Pro Val Thr Leu Asn Val Thr Tyr Gly Pro Asp Thr Pro
   225                 230                 235                 240
   Thr Ile Ser Pro Ser Asp Thr Tyr Tyr Arg Pro Gly Ala Asn Leu Ser
                   245                 250                 255
   Leu Ser Cys Tyr Ala Ala Ser Asn Pro Pro Ala Gln Tyr Ser Trp Leu
               260                 265                 270
   Ile Asn Gly Thr Phe Gln Gln Ser Thr Gln Glu Leu Phe Ile Pro Asn
   275 280 285
   Ile Thr Val Asn Asn Ser Gly Ser Tyr Thr Cys His Ala Asn Asn Ser
   290 295 300
   Val Thr Gly Cys Asn Arg Thr Thr Val Lys Thr Ile Ile Val Thr Glu
   305 310 315 320
   Ser Pro Val Leu Gly Glu Asp Glu Ala Val Pro Gly Gln His His Pro
   325 330 335
   Gln His Lys Pro Cys Gln Glu Gly Gly Cys Trp Asp Val Leu Val
   340 345 350
<210> 30
   <211> 321
   <212> PRT
   <213> Homo sapiens
<400> 30
   Met Gly His Leu Ser Ala Pro Leu His Arg Val Arg Val Pro Trp Gln
   1 5 10 15
   Gly Leu Leu Leu Thr Ala Ser Leu Leu Thr Phe Trp Asn Pro Pro Thr
   20 25 30
   Thr Ala Gln Leu Thr Thr Glu Ser Met Pro Phe Asn Val Ala Glu Gly
   35 40 45
   Lys Glu Val Leu Leu Leu Val His Asn Leu Pro Gln Gln Leu Phe Gly
   50 55 60
   Tyr Ser Trp Tyr Lys Gly Glu Arg Val Asp Gly Asn Arg Gln Ile Val
   65 70 75 80
   Gly Tyr Ala Ile Gly Thr Gln Gln Ala Thr Pro Gly Pro Ala Asn Ser
   85 90 95
   Gly Arg Glu Thr Ile Tyr Pro Asn Ala Ser Leu Leu Ile Gln Asn Val
   100 105 110
   Thr Gln Asn Asp Thr Gly Phe Tyr Thr Leu Gln Val Ile Lys Ser Asp
   115 120 125
   Leu Val Asn Glu Glu Ala Thr Gly Gln Phe His Val Tyr Pro Glu Leu
   130 135 140
   Pro Lys Pro Ser Ile Ser Ser Asn Asn Ser Asn Pro Val Glu Asp Lys
   145                 150                 155                 160
   Asp Ala Val Ala Phe Thr Cys Glu Pro Glu Thr Gln Asp Thr Thr Tyr
                   165                 170                 175
   Leu Trp Trp Ile Asn Asn Gln Ser Leu Pro Val Ser Pro Arg Leu Gln
               180                 185                 190
   Leu Ser Asn Gly Asn Arg Thr Leu Thr Leu Leu Ser Val Thr Arg Asn
           195                 200                 205
   Asp Thr Gly Pro Tyr Glu Cys Glu Ile Gln Asn Pro Val Ser Ala Asn
       210                 215                 220
   Arg Ser Asp Pro Val Thr Leu Asn Val Thr Tyr Gly Pro Asp Thr Pro
   225                 230                 235                 240
   Thr Ile Ser Pro Ser Asp Thr Tyr Tyr Arg Pro Gly Ala Asn Leu Ser
                   245                 250                 255
   Leu Ser Cys Tyr Ala Ala Ser Asn Pro Pro Ala Gln Tyr Ser Trp Leu
               260                 265                 270
   Ile Asn Gly Thr Phe Gln Gln Ser Thr Gln Glu Leu Phe Ile Pro Asn
           275                 280                 285
   Ile Thr Val Asn Asn Ser Gly Ser Tyr Thr Cys His Ala Asn Asn Ser
       290                 295                 300
   Val Thr Gly Cys Asn Arg Thr Thr Val Lys Thr Ile Ile Val Thr Gly
   305                 310                 315                 320
   Lys
<210> 31
   <211> 417
   <212> PRT
   <213> Homo sapiens
<400> 31
   Met Gly His Leu Ser Ala Pro Leu His Arg Val Arg Val Pro Trp Gln
   1               5                   10                  15
   Gly Leu Leu Leu Thr Ala Ser Leu Leu Thr Phe Trp Asn Pro Pro Thr
               20                  25                  30
   Thr Ala Gln Leu Thr Thr Glu Ser Met Pro Phe Asn Val Ala Glu Gly
           35                  40                  45
   Lys Glu Val Leu Leu Leu Val His Asn Leu Pro Gln Gln Leu Phe Gly
       50                  55                  60
   Tyr Ser Trp Tyr Lys Gly Glu Arg Val Asp Gly Asn Arg Gln Ile Val
   65                  70                  75                  80
   Gly Tyr Ala Ile Gly Thr Gln Gln Ala Thr Pro Gly Pro Ala Asn Ser
                   85                  90                  95
   Gly Arg Glu Thr Ile Tyr Pro Asn Ala Ser Leu Leu Ile Gln Asn Val
               100                 105                 110
   Thr Gln Asn Asp Thr Gly Phe Tyr Thr Leu Gln Val Ile Lys Ser Asp
           115                 120                 125
   Leu Val Asn Glu Glu Ala Thr Gly Gln Phe His Val Tyr Pro Glu Leu
       130                 135                 140
   Pro Lys Pro Ser Ile Ser Ser Asn Asn Ser Asn Pro Val Glu Asp Lys
   145                 150                 155                 160
   Asp Ala Val Ala Phe Thr Cys Glu Pro Glu Thr Gln Asp Thr Thr Tyr
                   165                 170                 175
   Leu Trp Trp Ile Asn Asn Gln Ser Leu Pro Val Ser Pro Arg Leu Gln
               180                 185                 190
   Leu Ser Asn Gly Asn Arg Thr Leu Thr Leu Leu Ser Val Thr Arg Asn
           195                 200                 205
   Asp Thr Gly Pro Tyr Glu Cys Glu Ile Gln Asn Pro Val Ser Ala Asn
       210                 215                 220
   Arg Ser Asp Pro Val Thr Leu Asn Val Thr Tyr Gly Pro Asp Thr Pro
   225                 230                 235                 240
   Thr Ile Ser Pro Ser Asp Thr Tyr Tyr Arg Pro Gly Ala Asn Leu Ser
                   245                 250                 255
   Leu Ser Cys Tyr Ala Ala Ser Asn Pro Pro Ala Gln Tyr Ser Trp Leu
               260                 265                 270
   Ile Asn Gly Thr Phe Gln Gln Ser Thr Gln Glu Leu Phe Ile Pro Asn
           275                 280                 285
   Ile Thr Val Asn Asn Ser Gly Ser Tyr Thr Cys His Ala Asn Asn Ser
       290                 295                 300
   Val Thr Gly Cys Asn Arg Thr Thr Val Lys Thr Ile Ile Val Thr Glu
   305                 310                 315                 320
   Leu Ser Pro Val Val Ala Lys Pro Gln Ile Lys Ala Ser Lys Thr Thr
                   325                 330                 335
   Val Thr Gly Asp Lys Asp Ser Val Asn Leu Thr Cys Ser Thr Asn Asp
               340                 345                 350
   Thr Gly Ile Ser Ile Arg Trp Phe Phe Lys Asn Gln Ser Leu Pro Ser
           355                 360                 365
   Ser Glu Arg Met Lys Leu Ser Gln Gly Asn Thr Thr Leu Ser Ile Asn
       370                 375                 380
   Pro Val Lys Arg Glu Asp Ala Gly Thr Tyr Trp Cys Glu Val Phe Asn
   385                 390                 395                 400
   Pro Ile Ser Lys Asn Gln Ser Asp Pro Ile Met Leu Asn Val Asn Cys
                   405                 410                 415
   Lys
<210> 32
   <211> 525
   <212> PRT
   <213> Homo sapiens
<400> 32
   Met Gly His Leu Ser Ala Pro Leu His Arg Val Arg Val Pro Trp Gln
   1               5                   10                  15
   Gly Leu Leu Leu Thr Ala Ser Leu Leu Thr Phe Trp Asn Pro Pro Thr
               20                  25                  30
   Thr Ala Gln Leu Thr Thr Glu Ser Met Pro Phe Asn Val Ala Glu Gly
           35                  40                  45
   Lys Glu Val Leu Leu Leu Val His Asn Leu Pro Gln Gln Leu Phe Gly
       50                  55                  60
   Tyr Ser Trp Tyr Lys Gly Glu Arg Val Asp Gly Asn Arg Gln Ile Val
   65                  70                  75                  80
   Gly Tyr Ala Ile Gly Thr Gln Gln Ala Thr Pro Gly Pro Ala Asn Ser
                   85                  90                  95
   Gly Arg Glu Thr Ile Tyr Pro Asn Ala Ser Leu Leu Ile Gln Asn Val
               100                 105                 110
   Thr Gln Asn Thr Gly Phe Tyr Thr Leu Gln Val Ile Lys Ser Asp Leu
           115                 120                 125
   Val Asn Glu Glu Ala Thr Gly Gln Phe His Val Tyr Pro Glu Leu Pro
       130                 135                 140
   Lys Pro Ser Ile Ser Ser Asn Asn Ser Asn Pro Val Glu Asp Lys Asp
   145                 150                 155                 160
   Ala Val Ala Phe Thr Cys Glu Pro Glu Thr Gln Asp Thr Thr Tyr Leu
                   165                 170                 175
   Trp Trp Ile Asn Asn Gln Ser Leu Pro Val Ser Pro Arg Leu Gln Leu
               180                 185                 190
   Ser Asn Gly Asn Arg Thr Leu Thr Leu Leu Ser Val Thr Arg Asn Asp
           195                 200                 205
   Thr Gly Pro Tyr Glu Cys Glu Ile Gln Asn Pro Val Ser Ala Asn Arg
       210                 215                 220
   Ser Asp Pro Val Thr Leu Asn Val Thr Tyr Gly Pro Asp Thr Pro Thr
   225                 230                 235                 240
   Ile Ser Pro Ser Asp Thr Tyr Tyr Arg Pro Gly Ala Asn Leu Ser Leu
                   245                 250                 255
   Ser Cys Tyr Ala Ala Ser Asn Pro Pro Ala Gln Tyr Ser Trp Leu Ile
               260                 265                 270
   Asn Gly Thr Phe Gln Gln Ser Thr Gln Glu Leu Phe Ile Pro Asn Ile
           275                 280                 285
   Thr Val Asn Asn Ser Gly Ser Tyr Thr Cys His Ala Asn Asn Ser Val
       290                 295                 300
   Thr Gly Cys Asn Arg Thr Thr Val Lys Thr Ile Ile Val Thr Glu Leu
   305                 310                 315                 320
   Ser Pro Val Val Ala Lys Pro Gln Ile Lys Ala Ser Lys Thr Thr Val
                   325                 330                 335
   Thr Gly Asp Lys Asp Ser Val Asn Leu Thr Cys Ser Thr Asn Asp Thr
               340                 345                 350
   Gly Ile Ser Ile Arg Trp Phe Phe Lys Asn Gln Ser Leu Pro Ser Ser
           355                 360                 365
   Glu Arg Met Lys Leu Ser Gln Gly Asn Thr Thr Leu Ser Ile Asn Pro
       370                 375                 380
   Val Lys Arg Glu Asp Ala Gly Thr Tyr Trp Cys Glu Val Phe Asn Pro
   385                 390                 395                 400
   Ile Ser Lys Asn Gln Ser Asp Pro Ile Met Leu Asn Val Asn Tyr Asn
                   405                 410                 415
   Ala Leu Pro Gln Glu Asn Gly Leu Ser Pro Gly Ala Ile Ala Gly Ile
               420                 425                 430
   Val Ile Gly Val Val Ala Leu Val Ala Leu Ile Ala Val Ala Leu Ala
           435                 440                 445
   Cys Phe Leu His Phe Gly Lys Thr Gly Arg Ala Ser Asp Gln Arg Asp
       450                 455                 460
   Leu Thr Glu His Lys Pro Ser Val Ser Asn His Thr Gln Asp His Ser
   465                 470                 475                 480
   Asn Asp Pro Pro Asn Lys Met Asn Glu Val Thr Tyr Ser Thr Leu Asn
                   485                 490                 495
   Phe Glu Ala Gln Gln Pro Thr Gln Pro Thr Ser Ala Ser Pro Ser Leu
               500                 505                 510
   Thr Ala Thr Glu Ile Ile Tyr Ser Glu Val Lys Lys Gln
           515                 520                 525
<210> 33
   <211> 390
   <212> PRT
   <213> Homo sapiens
<400> 33
<210> 34
   <211> 249
   <212> PRT
   <213> Homo sapiens
<400> 34
   Leu Ser Thr Cys Lys Thr Ile Asp Met Glu Leu Val Lys Arg Lys Arg
   1               5                   10                  15
   Ile Glu Ala Ile Arg Gly Gln Ile Leu Ser Lys Leu Arg Leu Ala Ser
               20                  25                  30
   Pro Pro Ser Gln Gly Glu Val Pro Pro Gly Pro Leu Pro Glu Ala Val
           35                  40                  45
   Leu Ala Leu Tyr Asn Ser Thr Arg Asp Arg Val Ala Gly Glu Ser Ala
       50                  55                  60
   Glu Pro Glu Pro Glu Pro Glu Ala Asp Tyr Tyr Ala Lys Glu Val Thr
   65                  70                  75                  80
   Arg Val Leu Met Val Glu Thr His Asn Glu Ile Tyr Asp Lys Phe Lys
                   85                  90                  95
   Gln Ser Thr His Ser Ile Tyr Met Phe Phe Asn Thr Ser Glu Leu Arg
               100                 105                 110
   Glu Ala Val Pro Glu Pro Val Leu Leu Ser Arg Ala Glu Leu Arg Leu
           115                 120                 125
   Leu Arg Leu Lys Leu Lys Val Glu Gln His Val Glu Leu Tyr Gln Lys
       130                 135                 140
   Tyr Ser Asn Asn Ser Trp Arg Tyr Leu Ser Asn Arg Leu Leu Ala Pro
   145                 150                 155                 160
   Ser Asp Ser Pro Glu Trp Leu Ser Phe Asp Val Thr Gly Val Val Arg
                   165                 170                 175
   Gln Trp Leu Ser Arg Gly Gly Glu Ile Glu Gly Phe Arg Leu Ser Ala
               180                 185                 190
   His Cys Ser Cys Asp Ser Arg Asp Asn Thr Leu Gln Val Asp Ile Asn
           195                 200                 205
   Gly Phe Thr Thr Gly Arg Arg Gly Asp Leu Ala Thr Ile His Gly Met
       210                 215                 220
   Asn Arg Pro Phe Leu Leu Leu Met Ala Thr Pro Leu Glu Arg Ala Gln
   225                 230                 235                 240
   His Leu Gln Ser Ser Arg His Arg Arg
                   245
<210> 35
   <211> 20
   <212> DNA
   <213> Artificial Sequence
<220>
   <223> Description of Artificial Sequence: Synthetic primer
<400> 35
   gcgagatctc acagagcaca 20
<210> 36
   <211> 20
   <212> DNA
   <213> Artificial Sequence
<220>
   <223> Description of Artificial Sequence: Synthetic primer
<400> 36
   gctgggaatt gaagttcagg 20
<210> 37
   <211> 20
   <212> DNA
   <213> Artificial Sequence
<220>
   <223> Description of Artificial Sequence: Synthetic primer
<400> 37
   ctggcatcgt gattggagtt 20
<210> 38
   <211> 18
   <212> DNA
   <213> Artificial Sequence
<220>
   <223> Description of Artificial Sequence: Synthetic primer
<400> 38
   cagaaggagc cagatccg 18

## Description

### BACKGROUND

The immune system exists to protect the body from foreign invaders and diseased cells. A healthy immune system can recognize foreign or aberrant cells and target them for destruction. As part of the natural control of the immune system, components of one's immune system (e.g. T cells) that recognize healthy host cells are destroyed or forced to enter an inactive state, a phenomenon referred to as "T cell tolerance." This prevents the immune system from attacking the healthy cells of the host organism itself.

However, in the case of chronic diseases, the prolonged response of the immune system to the chronic disease can lead to counterproductive T cell tolerance. T cells that are recognizing and attacking foreign or diseased cells can be "turned off" via T cell tolerance. This obviously reduces the host's ability to respond to and recover from the chronic disease.

Chronic immune disorders can wreak havoc, particularly those associated with T-cell tolerance, such as cancers. According to the most recent data from the World Health Organization, ten million people around the world were diagnosed with the cancer in 2000, and six million died from it. Moreover, statistics indicate that the cancer incidence rate is on the rise around the globe. In America, for example, projections suggest that fifty percent of those alive today will be diagnosed with some form of cancer at some point in their lives. Hence, there remains an urgent need for compositions and approaches to treating T-cell tolerance mediated immune disorders.

### SUMMARY

According to the present invention, there is provided a therapeutically effective amount of an anti-CEACAM1 antibody and an anti-PD1 antibody for use in therapy.

Also provided is a composition comprising an anti-CEACAM1 antibody and an anti-PD1 antibody for use in therapy.

Provided herein are compositions for use in enhancing the immune response and/or reducing T cell tolerance in a subject. As described herein, the inventors have discovered that certain subpopulations of T cells can coexpress two or more of CEACAM1, PD1, and/or LAP, which promote T-cell tolerance through separate signaling pathways. The discovery that T cells can coexpress these molecules underlies the technology described herein, which relates to administering inhibitors of two or more of these molecules in order to achieve a greater degree of reduction in T cell tolerance than is possible by inhibiting only one pathway.

Accordingly, the compositions and methods described herein increase the T-cell mediated immune response in a subject (i.e. reduce T-cell tolerance) by concurrently inhibiting the activity of the signaling pathways controlled by at least two of CEACAM1, PD1, and/or LAP. Relieving the suppression of the immune system is highly desirable in, for example, an immunocompromised subject or a subject suffering from cancer.

In one aspect, described herein is a composition for use in enhancing the immune response or decreasing T cell tolerance in a subject, the composition comprising an anti-CEACAM1 antibody and an anti-PD1 antibody. In some embodiments, the composition can comprise an anti-CEACAM1 antibody, an anti-PD1 antibody, and a LAP inhibitory agent.

Also described herein is a composition comprising a bispecific agent comprising a first portion which is a CEACAM1 inhibitory agent and a second portion which is a PD1 inhibitory agent.

In some embodiments, the anti-CEACAM1 antibody can bind a CEACAM1 molecule having the sequence set forth in SEQ ID NO: 32 or an allelic or splice variant of SEQ ID NO: 32. In some embodiments, the anti-CEACAM1 antibody can bind a CEACAM1 ligand interaction site. In some embodiments, the anti-CEACAM1 antibody can bind to an amino acid residue corresponding to any of amino acid residues 1-429 of SEQ ID NO: 32. In some embodiments, the anti-CEACAM1 antibody can bind an extracellular domain of CEACAM1. In some embodiments, the anti-CEACAM1 antibody can bind the homophilic interaction domain of CEACAM1 and inhibits homophilic interaction of CEACAM1 molecules.

In some embodiments, the anti-PD1 antibody can bind a PD1 molecule having the sequence set forth in SEQ ID NO:1, or an allelic or splice variant of SEQ ID NO: 1. In some embodiments, the anti-PD1 antibody can bind a PD1 ligand interaction site. In some embodiments, the anti-PD1 antibody can bind to an amino acid residue corresponding to any of amino acid residues 41-136 of SEQ ID NO: 1.

In some embodiments, the LAP inhibitory agent can bind a LAP molecule having the sequence set forth in SEQ ID NO: 34 or an allelic or splice variant of SEQ ID NO: 34. In some embodiments, the LAP inhibitory agent can bind a LAP ligand interaction site. In some embodiments, the LAP inhibitory agent can bind to an amino acid residue corresponding to amino acid residue 218 of SEQ ID NO: 33.

In one aspect, described herein is a composition for use in treating a chronic disease. In some embodiments, the chronic disease can be selected from the group consisting of: cancer; a persistent infection; and a chronic viral infection.

### BRIEF DESCRIPTION OF THE DRAWINGS

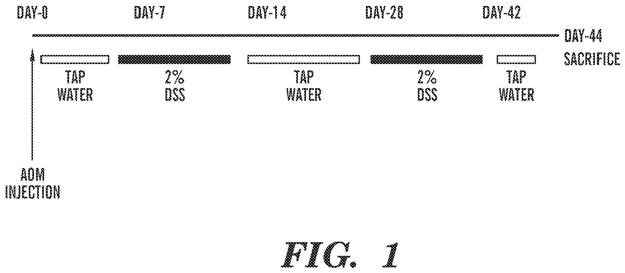

Figure 1 depicts a schematic of the AOM-DSS model in WT mice.

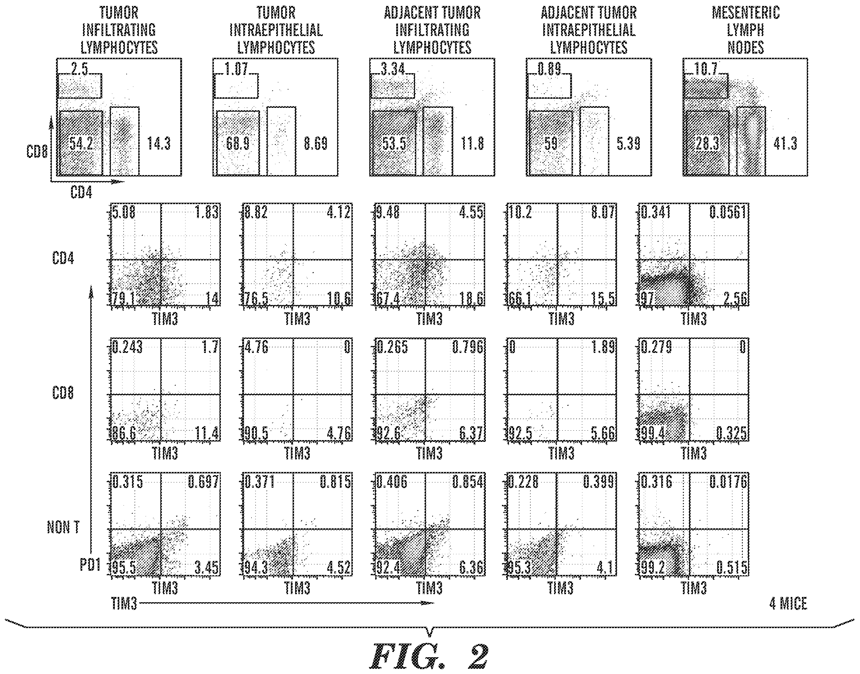

Figure 2 depicts the results of FACS analysis examining expression of PD1 and TIM-3 in CD4 and CD8 T-cells.

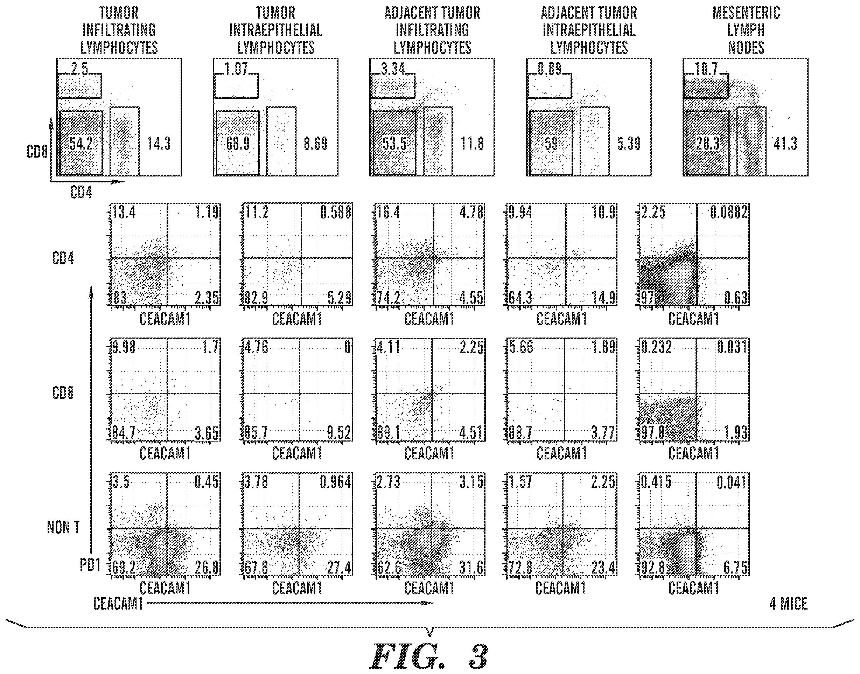

Figure 3 depicts the results of FACS analysis examining expression of PD1 and CEACAM1 in CD4 and CD8 T-cells.

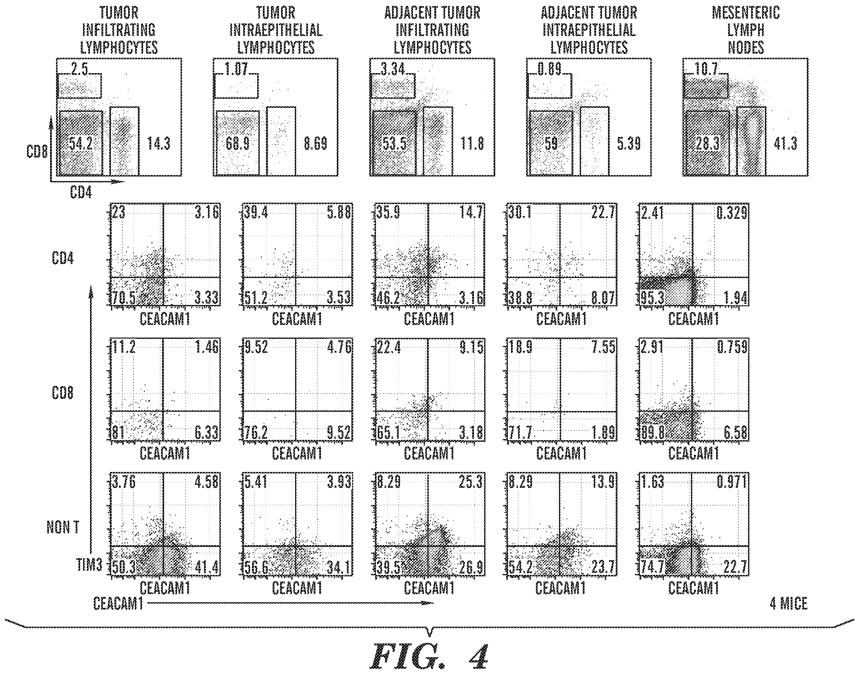

Figure 4 depicts the results of FACS analysis examining expression of CEACAM1 and TIM-3 in CD4 and CD8 T-cells.

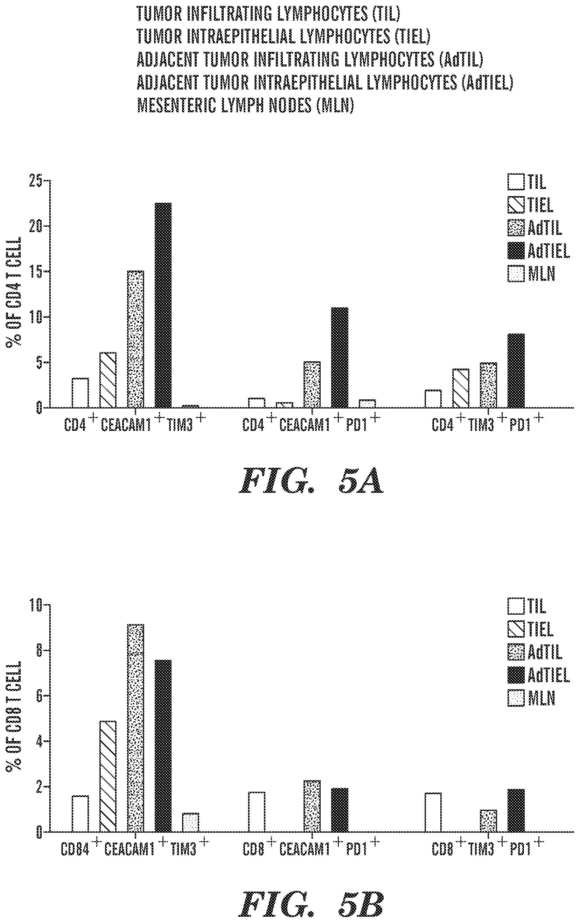

Figures 5A-5B depict graphs demonstrating CEACAM1 and PD1 are co-expressed on the cell surface of chronically activated CD4⁺ (Figure 5A) and CD8⁺ (Figure 5B) tumor infiltrating T lymphocytes.

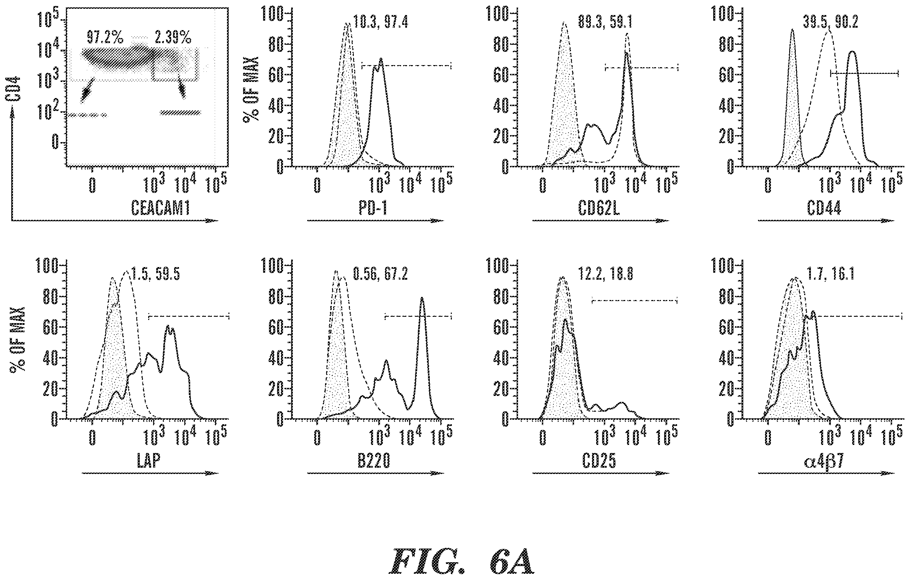

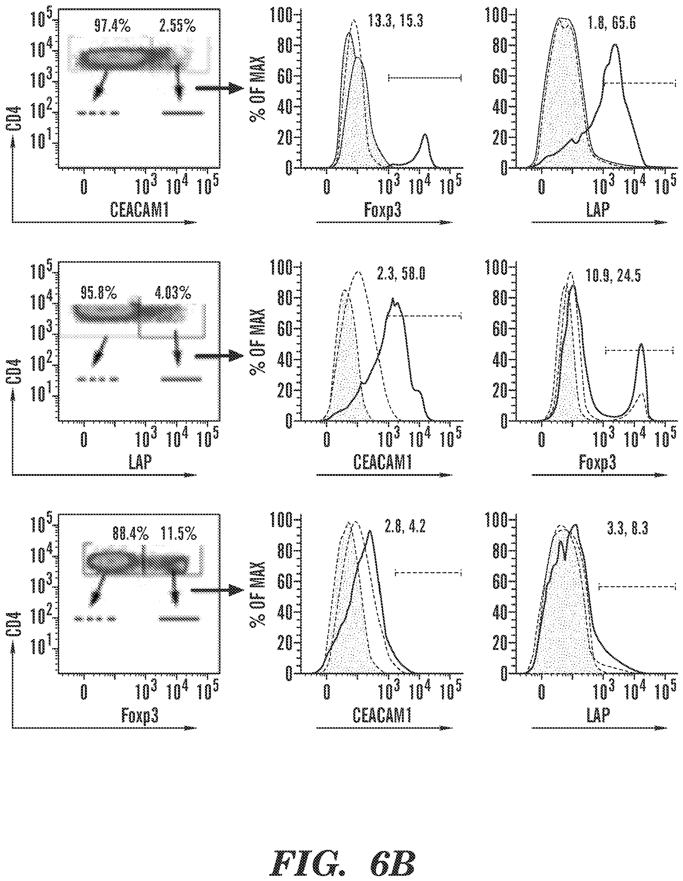

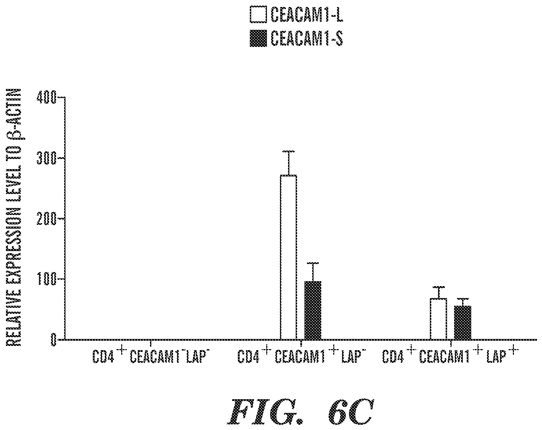

Figures 6A-6C demonstrate that CEACAM1-4S is preferentially expressed on specific subsets of both circulating and intestinal CD4+ T cells. Figure 6A depicts graphs of FACS analysis demonstrating that expression of CEACAM1 is associated with the enrichment of specific cell surface molecules. Percentages on histogram gates indicate the frequency of cells positive for the given marker in the CD4+CEACAM1- and CD4+CEACAM1+ cell populations, respectively, of MLN CD3+ T cells from WT mice. Figure 6B depicts graphs of FACS analysis demonstrating that CEACAM1 expression co-segregates with latency associated peptide (LAP) expression and is unrelated to Foxp3 expression in CD4+ T cells from the MLN of naive WT mice. Figure 6C depicts a chart demonstrating that the CEACAM1-S isoform is enriched on CD4+CEACAM1+ T cells which express LAP. T cells were isolated from pooled spleen and lymph node (LN) in Figure 6C. Figures 6A-6C show representative data from 3 independent experiments with n = 5-6 mice per group.

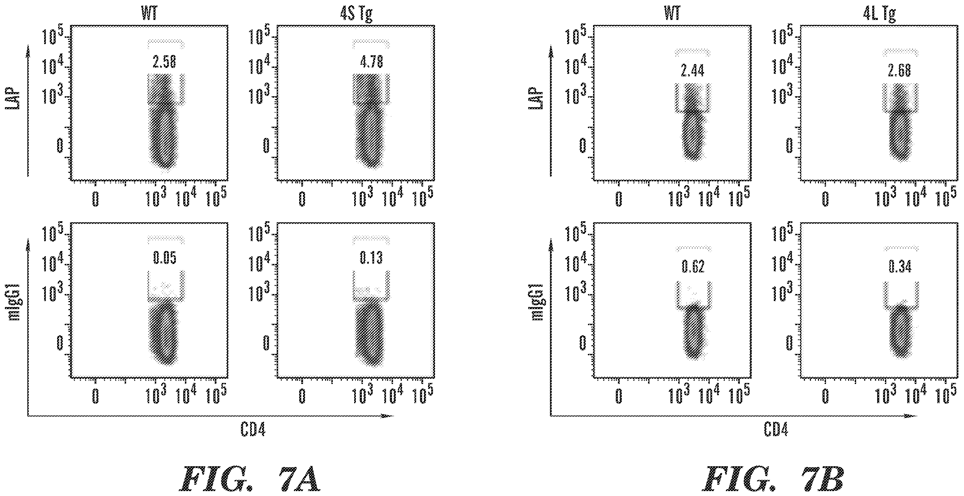

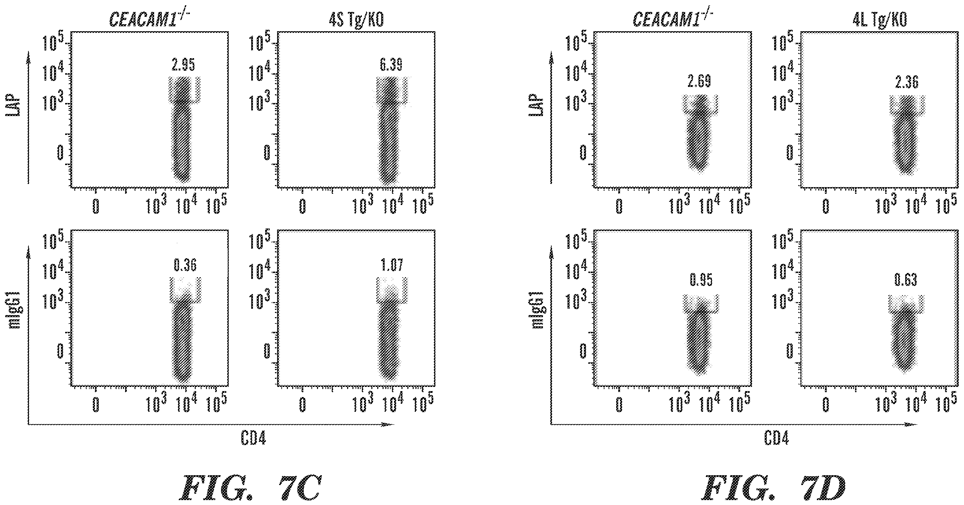

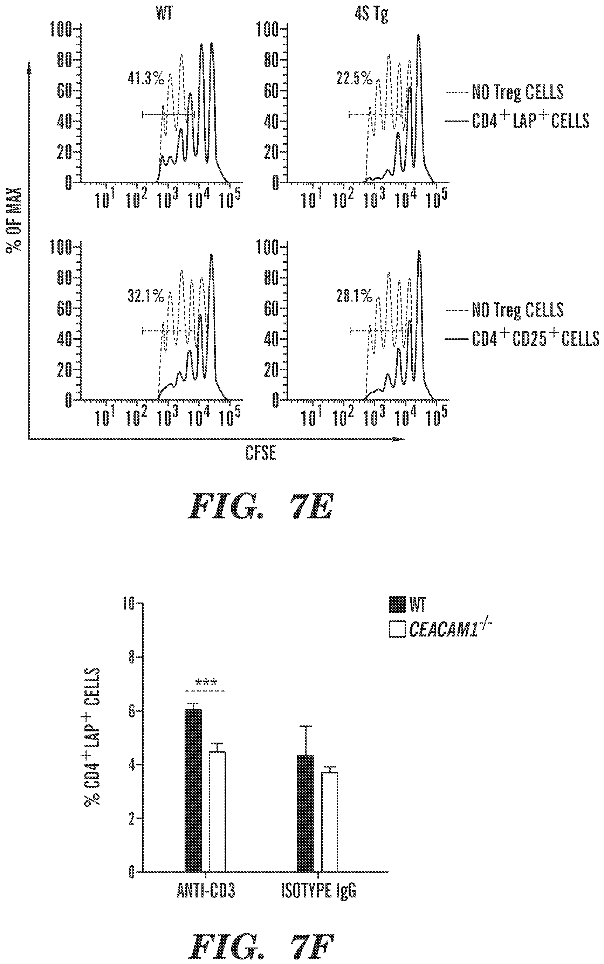

Figures 7A-7F demonstrate that CEACAM1-S directly enhances the generation of CD4+LAP+. Figures 7A-7D depict graphs of FACS analysis results demonstrating that the percentage of CD4+LAP+ cells is increased in MLN of naive CEACAM1-4S transgenic (4S Tg) mice over-expressing this isoform specifically in T cells (Figure 7A) but not CEACAM1-4L (4L Tg) mice (Figure 7B). *CEACAM1*^{*-*/*-*}-4S Tg mice (Figure 7C) but not *CEACAM1*^{*-*/*-*}-4L Tg mice (Figure 7D) are enriched in CD4+LAP+ T cells compared to their littermate controls. Figure 7E depicts graphs of FACS analysis results demonstrating that overexpression of CEACAM1-4S enhances the regulatory function of CD4+LAP+ Treg cells in vitro as shown by the decrease in proliferation of responder T cells when co-cultured with CD4+LAP+ Treg cells from pooled spleen and LN of 4S Tg mice. Figure 7F depicts a chart demonstrating that oral feeding of anti-CD3 antibody significantly increases the percentage of CD4+LAP+ Treg cells in the MLN of WT but not *CEACAM1*^{*-*/*-*}mice. Mice were fed 5 µg anti-CD3 (or isotype control) for each of 5 consecutive days and CD4+LAP+ T cell frequency was assessed after 7 days.

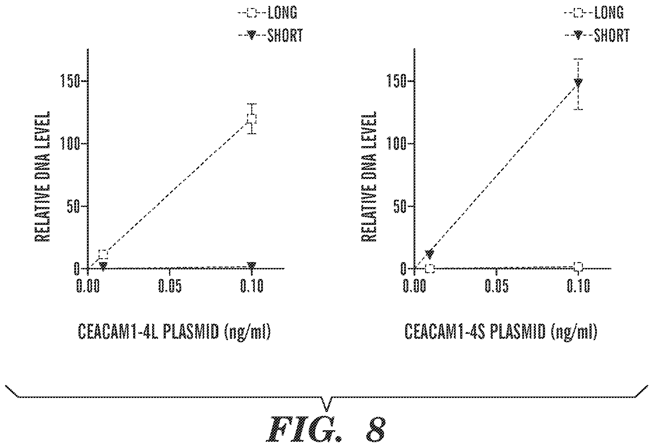

Figure 8 depicts the results of a novel CEACAM1 isoform-discriminating qPCR assay which depicts the specificity and efficiency of qPCR primers for mouse CEACAM1-L and -S isoforms amplification verified using mCEACAM1-4L and 4S plasmids. This novel assay was used in Figure 6C.

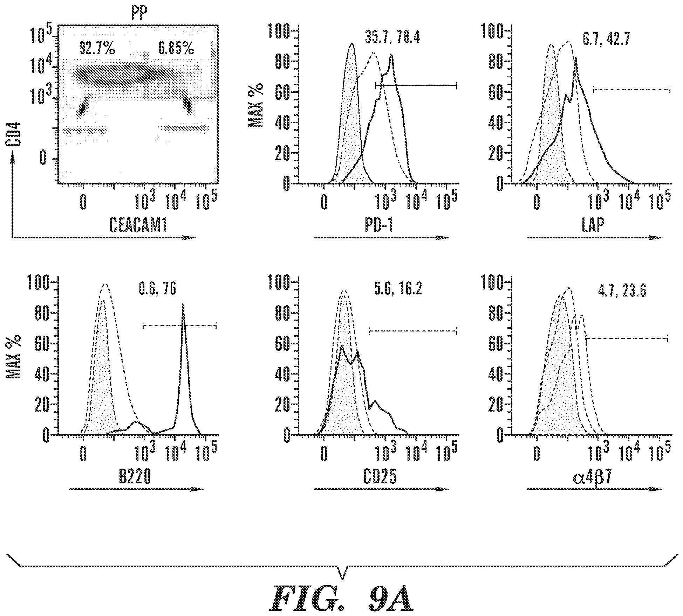

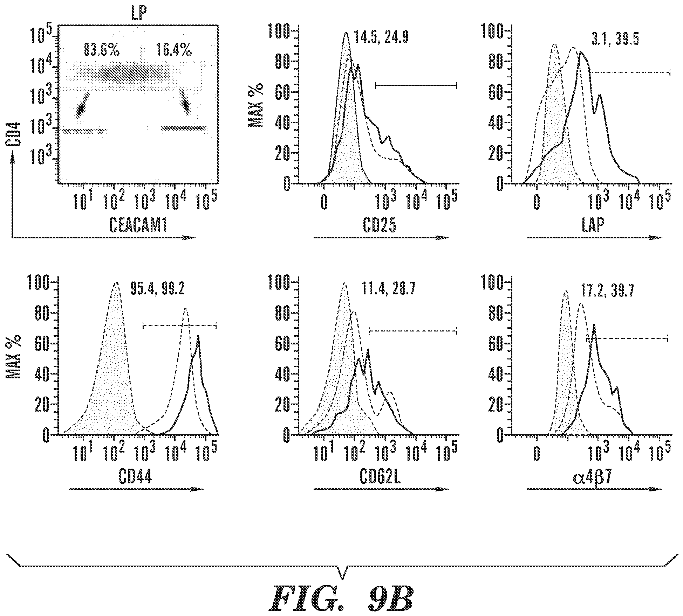

Figures 9A-9B demonstrate that CEACAM1-S expression on CD4+ T cells is associated with the expression of molecules which characterize specific subsets of T cells in the Peyer's patches (PP) and lamina propria (LP). Figures 9A and 9B depict graphs of FACS analysis characterizing cell surface molecule expression on CD4+CEACAM1+ and CD4+CEACAM1- cells in PP and LP of WT mice. Percentages on histogram gates indicate the frequency of cells positive for the given marker in the CD4+CEACAM1- and CD4+CEACAM1+ cell populations, respectively. Representative data from 3 independent experiments with n = 5-6 mice per group.

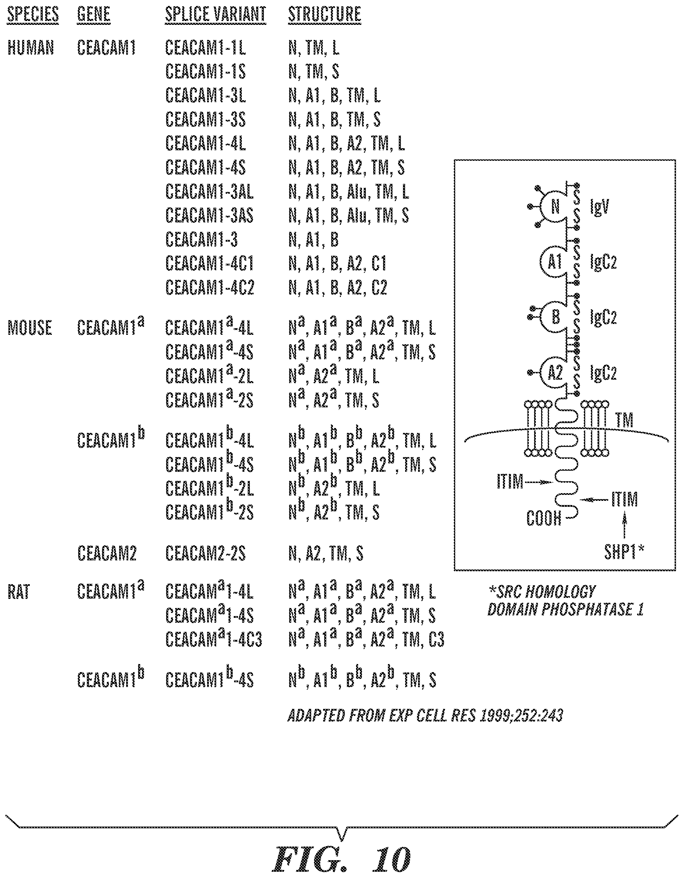

Figure 10 depicts the isoforms of CEACAM1 created by alternate splicing.

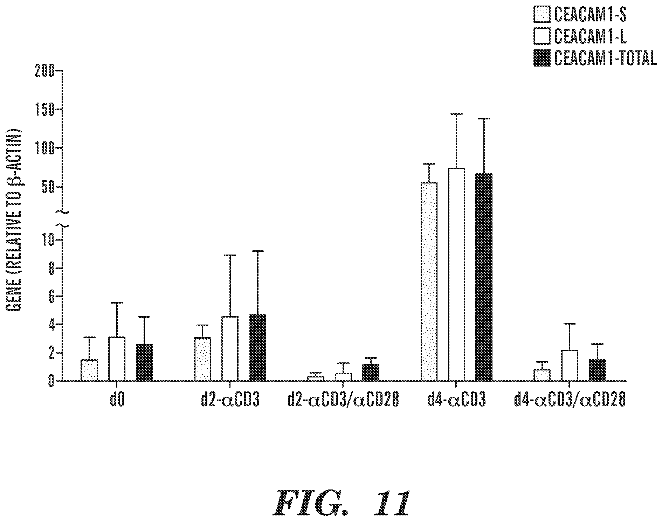

Figure 11 depicts a graph of CEACAM1-S and CEACAM1-L expression using the qPCR assay in Figure 8. Total T cells from spleen were stimulated with plate-bound coated anti-CD3 or anti-CD3 plus CD28 for 2 and subsequently 4 days. CEACAM1-S, -L and total CEACAM1 were assessed by qPCR. Figure 11 shows that both of the major CEACAM1 isoforms (S and L) which are low in resting T cells are transcriptionally regulated by ligation of T cells through the TCR/CD3 complex (signal 1) but negatively regulated when the TCR/CD3 complex is co-engaged by the classical co-stimulatory signal (signal 2) provided by CD28.

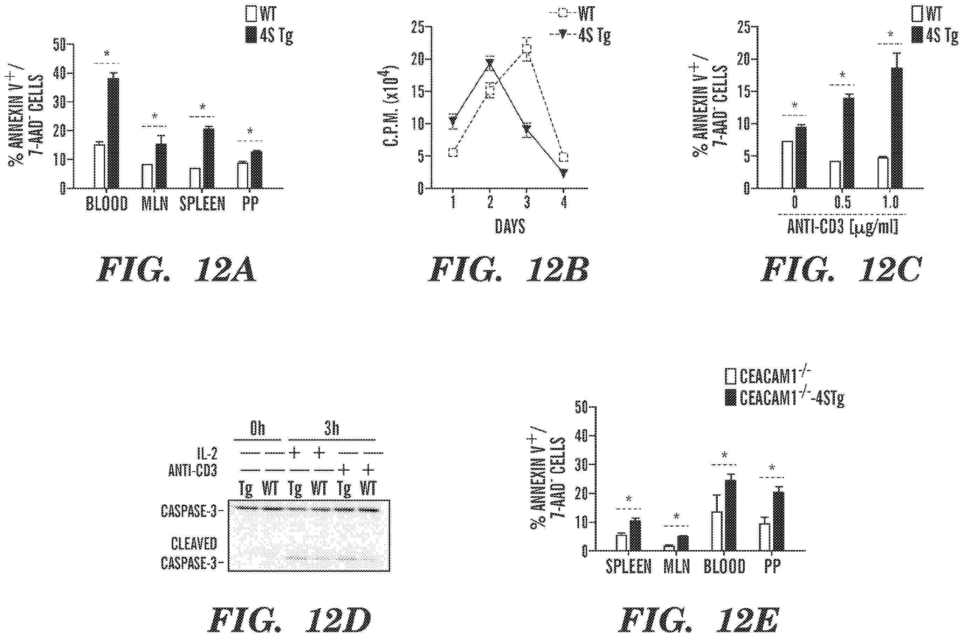

Figures 12A-12E demonstrate that CEACAM1-4S is associated with activation of primary T cells and sensitization to activation induced cell death. Figure 12A depicts a graph demonstrating that compared to WT littermates, 4S Tg mice exhibit higher percentages of CD3⁺ Annexin V⁺/7AAD⁻ apoptotic cells. (n = 5 mice per group) Figures 12B-12C depict graphs of the results of experiments in which CD3⁺ T cells isolated from spleen of 4S Tg mice and their WT littermates were cultured with anti-CD3 antibody at indicated concentrations, or 1 µg/ml anti-CD3 over the indicated time course. Proliferation assays were performed by [³H]-thymidine incorporation (Figure 12B) and apoptosis was determined using annexin-V and 7-AAD staining (Figure 12C). (n = 5 mice per group) Figure 12D depicts an immunoblot. CD3⁺ T cells were isolated from the spleen of 4S Tg or WT mice, and cultured with IL-2 (100 U/ml) or anti-CD3 (1 µg/ml) coated plates. Cell lysates were prepared at the indicated times, and caspase-3 and its activated form were determined using immunoblot. Figure 12E depicts a graph demonstrating that compared to *Ceacam1*^{*-*/*-*} mice, *Ceacam1*^{*-*/*-*}-4S Tg mice have higher percentages of annexin V⁺/7AAD⁻ apoptotic cells (n = 5 mice per group).

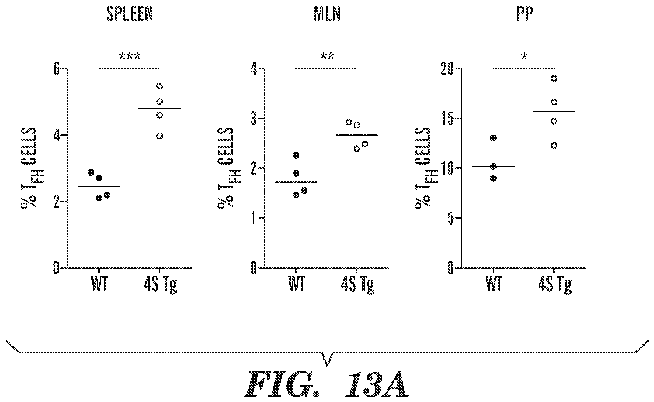

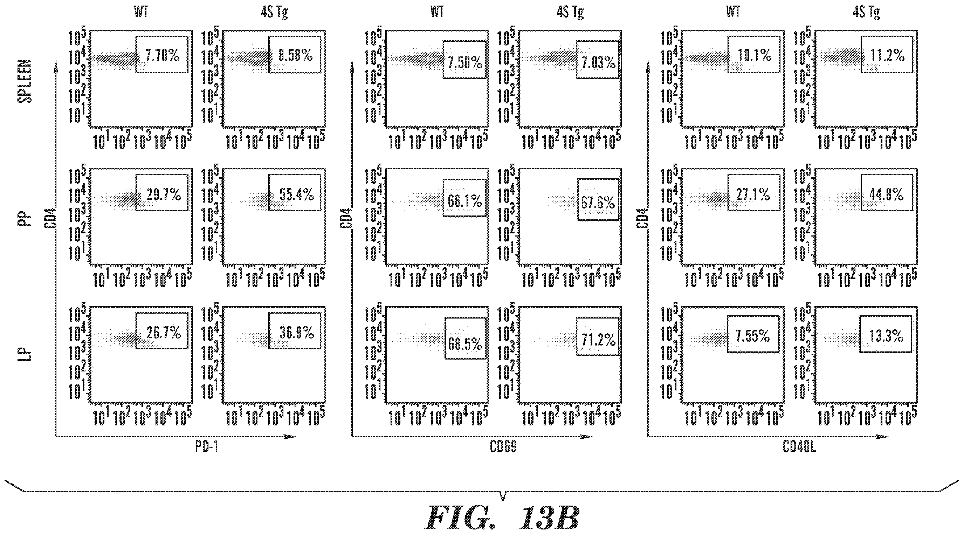

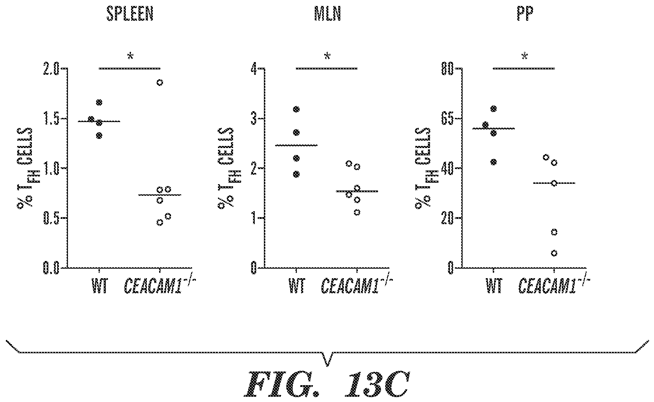

Figures 13A-13C demonstrate that CEACAM1-S directly enhances the generation of PD-1 bearing Tfollicular helper (T_{FH}) regulatory cells. Figures 13A and 13C depict graphs of the percentage of CD4⁺PD1⁺CXCR5⁺T_{FH} cells in the spleen, MLN and PP is increased in 4S Tg mice (Figure 13A) and decreased in *Ceacam1*^{*-*/*-*} mice (Figure 13C) when compared to their littermate controls. Figure 13B depicts plots of the results of FACS analysis. CD4+ T cells within the PP or LP, but not in the spleen of 4S Tg mice exhibit increased levels of CD69, PD-1 and CD40L relative to their WT littermate controls. All data are representative of 3 independent experiments with n = 3-6 mice per group.

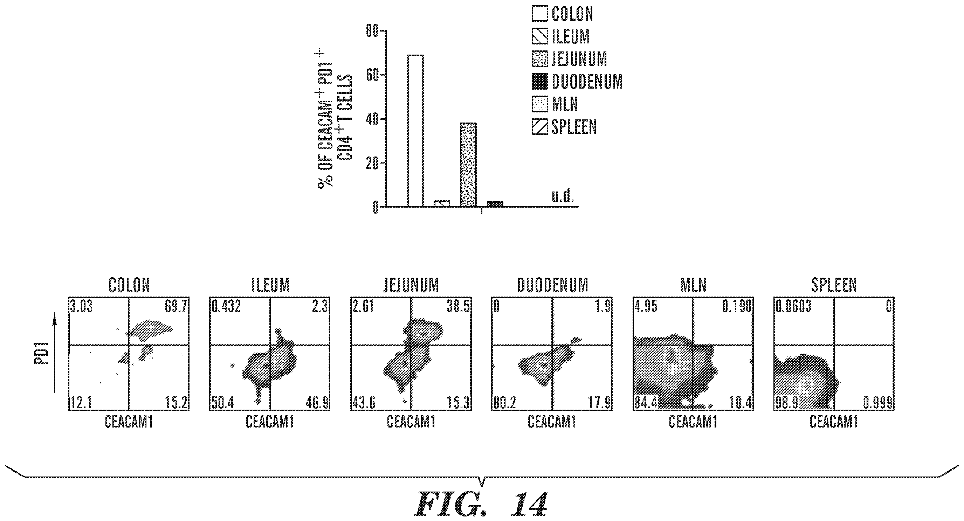

Figure 14 demonstrates co-expression of CEACAM1 and PD1 on T cells under physiologic conditions. Lamina propria lymphocytes were isolated from colon, ileum, jejunum and duodenum as well as mesenteric lymph nodes (MLN) and spleen and after gating on CD3-positive cells stained for co-expression of PD1 and CEACAM1. The top panel depicts a graph of the summary of quantity of positive cells and the bottom panel depicts the histograms of the flow cytometry.

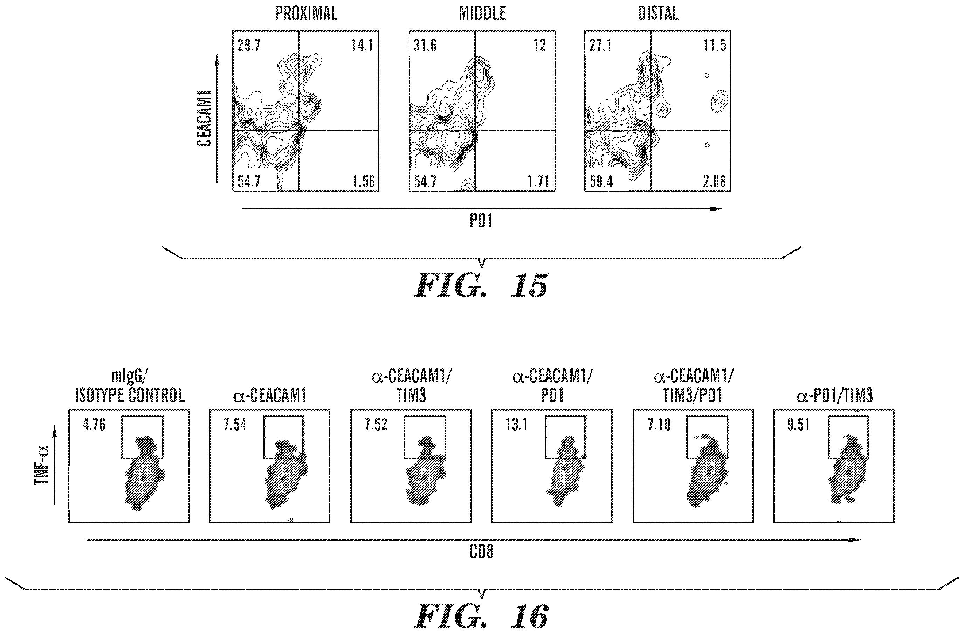

Figure 15 demonstrates co-expression of CEACAM1 and PD1 in lamina propria lymphocytes infiltrating the colon of mice with colitis induced by adoptive transfer of naive CD4+ T cells in Rag-deficient and CEACAM1-deficient animals. Co-expression of CEACAM1 and PD1 is shown after gating on CD3+ lymphocytes obtained from proximal, middle and distal colon.
Figure 16 demonstrates that blockade of CEACAM1 and PD1 increases TNF-a production from tumor infiltrating lymphocytes (TIL). TILs were isolated from subcutaneous tumors associated with CT26 colorectal carcinoma cell line. TILs were stimulated with anti-CD3 and anti-CD28 monoclonal antibodies in the presence of isotype control antibodies or antibodies that block different co-inhibitory cell surface molecules. Note the synergistic increase of TNF-a production by TILs treated with anti-CEACAM1 and anti-PDl antibodies.

### DETAILED DESCRIPTION

T-cell tolerance functions, in part, to provide a population of immune system cells that recognize self-major histocompatibility complex (MHC) molecules but do not recognize self-peptides. In chronic diseases (e.g. chronic infections or cancer), T cell tolerance occurs when the subject is still in need of a robust immune response. Previous studies have shown that carcinoembryonic antigen related cell adhesion molecule 1 (CEACAM1), programmed cell death 1 (PD1) and TGFβ1 latency associated peptide (LAP) are important components of immune regulation which increase T cell tolerance. These molecules have been observed to be expressed on separate subpopulations of activated T-cells, especially after prolonged activation. Thus, while CEACAM1, PD1 and LAP have been shown to play in T cell immune regulation, it had not been established or suggested that there exists a population of T cells which expresses detectable levels of two or more of CEACAM1, PD1, and/or LAP prior to the discoveries described herein.

As described herein, the inventors have found that certain subpopulations of T cells co-express detectable levels of (i) CEACAM1 and LAP, (ii) CEACAM1 and PD1, (iii) PD1 and LAP, or (iv) CEACAM1, PD1, and LAP. The discovery of such cells underlies the concept described herein of reducing T cell tolerance by inhibiting at least two of these T cell tolerance-promoting molecules. The ability to target multiple pathways which contribute to T cell tolerance can provide a greater effect upon T cell tolerance, e.g. by synergistic effects, leading to a therapeutic decrease in T cell tolerance. Inhibition of two or more of CEACAM1, PD1, and/or LAP can therefore enhance the responsiveness of the immune system.

As used herein, "CEACAM1" (also known as CD66a or biliary glycoprotein) refers to a polypeptide having a single Ig variable domain-like amino terminus, from one to three Ig constant domain-like regions, and a single membrane-spanning segment followed by either a short (CEACAM1-S) or long (CEACAM1-L) cytoplasmic domain (Hinoda et al., 85 PNAS 6959 (1988)). The N-terminal domain has been shown to facilitate homophilic intercellular binding that influences a broad spectrum of cellular processes related to cellular activation and/or cell cycle progression; and is also targeted by the heterophilic adhesins of viral (murine hepatitis virus) and bacterial (*Neisseria gonorrhoeae* and *N. meningitidis, Moraxella catarrhalis,* and *Haemophilus influenzae*) pathogens, allowing their infection of the diverse array of CEACAM1-expressing human cells and tissues *in vivo.*

When expressed, CEACAM1 is characterized by significant alternate RNA splicing leading to eleven isoforms in humans and at least four isoforms in mice. These isoforms differ in the length of the cytoplasmic tail and the number of extracellular Ig-like domains and are named accordingly. As noted, the majority of CEACAM1 isoforms possess either a long (CEACAM1-L) CT or a short (CEACAM1-S) cytoplasmic tails (Azuz-Lieberman et al., 17 Intl. Immunol. 837 (2005); Gray-Owen & Blumberg, 2006; Moller et al., 65 Int. J. Cancer 740 (1996); Nakajima et al., 168 J. Immunol. 1028 (2002); Singer et al., 168 J. Immunol. 5139 (2002)). The long cytoplasmic tail (∼72 amino acids in humans) contains two immune-receptor tyrosine-based inhibitory motifs (ITIMs) (Chen et al., 172 J. Immunol. 3535 (2004)). These isoforms are inhibitory for T-cell responses, which inhibition generally involves the ITIM domains and Src homology 2 domain phosphatase 1 (SHP-1). ITIM phosphorylation and, consequently, its association with SHP-1 requires p56 Lck kinase and the ability to bind homophilically. CEACAM1 recruits SHP-1 to the T-cell receptor (TCR) signalsome where SHP-1 blocks ZAP-70 activation via dephosphorylation of CD3-ζ, ZAP-70, or both. Indeed, masking the homophilic binding site with a specific Fab causes increased cytotoxicity and lymphocyte degranulation (Chen et al., 180 J. Immunol. 6085 (2008)). CEACAM1-S isoforms are inhibitory by inducing weak co-stimulatory signals that sensitize cells to activation induced cell death and the emergence of populations of regulatory cells such as CD4+LAP+ T cells (Chen et al., 172 J. Immunol. 3535 (2004)).

Clinical evidence shows that high-level CEACAM1 expression on tumors and tumor-infiltrating lymphocytes correlates with poor prognosis and high risk of metastasis. CEACAM1 functions as a regulatory co-receptor for both lymphoid and myeloid cell types, and is constitutively expressed in a wide range of tissues and cell types. Its expression on natural killer (NK) cells and T-cells is, however, mainly induced by cytokines and membrane-activating receptor activation. As described herein, previous studies have determined that CEACAM1 is a ligand for itself (homophilic ligation), and is involved in heterophilic ligation with galectin 3 and selectins. CEACAM1 expression, which is low on resting T-cells, is transcriptionally regulated by ligation of T-cells through the TCR/CD3 complex (signal 1) but negatively regulated when the TCR/CD3 complex is co-engaged by the classical co-stimulatory signal (signal 2) provided by CD28 and induces both major classes of CEACAM1 isoforms (CEACAM1 short and CEACAM-long). Given the fact that TCR/CD3 ligation alone (in the absence of costimulation) is a common mechanism for the induction of T-cell tolerance, the strong induction of CEACAM1 by such stimulation may, without wishing to be bound or limited by theory, be part of the tolerogenic program.

Further, in regard to the tertiary structure of CEACAM1, the two major isoforms, CEACAM1-4L and CEACAM1-4S, which differ only in their cytoplasmic domains, have extracellular domains (ectodomains) comprised of four glycosylated Ig domains. CEACAM1-induced cell signaling is regulated by its intercellular homophilic binding at the cell surface, which is mediated by the N-terminal Ig domain (D1) in a reciprocal D1-D1 interaction. The basic structure of the IgV N-terminal domain of CEACAM1 is a tertiary fold of a stacked pair of β-pleated sheets. There are nine component β strands, with strands A, B, E, and D lying in one sheet and strands C, C', C", F, and G being antiparallel in the other sheet. The GFCC' face of the N-terminal domain of CEACAM1 is known to be crucial for mediating homophilic adhesion. Homophilic and heterophilic interactions have been observed for other adhesion receptor-ligand pairs; such as of CD2 with CD58; ICAM-1 with ICAM-1, LFA-1, rhinoviruses or *Plasmodium falciparum*-infected erythrocytes; or cadherins with cadherins (Watt et al., 98 Blood 1469 (2001)). These interactions indicated that the GFCC' faces of the immunoglobulin family members may have evolved, without wishing to be bound or limited by theory, as a sticky patch to recognize a variety of protein-protein interactions (Springer et al., 6 Ann. Rev. Cell Biol. 359 (1990)).

In some embodiments, an anti-CEACAM1 antibody can inhibit one or more isoforms of CEACAM1 which can promote T-cell tolerance. In some embodiments, an anti-CEACAM1 antibody can inhibit one or more isoforms of CEACAM1 which can promote T-cell tolerance and not other isoforms of CEACAM1. Isoforms of CEACAM1 which can promote T-cell tolerance include, but are not limited to the isoforms depicted in Figure 11, e.g. CEACAM1-1L, CEACAM1-1S, CEACAM1-3L, CEACAM1-3S, CEACAM1-4L, CEACAM1-4S, CEACAM1-3AL, CEACAM1-3AS, CEACAM1-3, CEACAM1-4C1, and CEACAM1-4C2 including both soluble and transmembrane isoforms and variants, substitution variants, and conservative substitution variants thereof. Further discussion of CEACAM1 isoforms can be found, e.g. in Beauchemin et al. Exp Cell Res 1999:252-243.

A peptide region responsible for CEACAM1 heterophilic interactions, for example with *Neisseria* Opa proteins, is also on the GFCC'C" face and overlaps partially with the homophilic binding site. Fedarovich et al., D62 Acta Cryst. 971 (2006). In comparison, binding of a murine CEACAM1 to murine coronavirus requires a uniquely folded CC' loop, in which amino acids 34 to 52 play a crucial role (Tan et al., 21 EMBO J. 2076 (2002); Watt et al., 2001). Additionally, amino acids between residues 27 to 42 (particularly D27L28F29) and S32, Y34, V39, Q44, Q89, and 191 on the GFCC'C" face form differential adhesiotopes for the binding of *H. influenzae,* and the *N. gonorhheae* and *N. meningiditis* Opa proteins. These adhesiotopes are likely a groove; formed by homophilic *cis* binding that involves V39 and D40 CC' loop residues, or formed after disruption of CEACAM1 *cis* dimerization by cytokine (e.g., TNFα) activation that precedes CEACAM1/pathogen binding (Watt et al., 2001).

Moreover, the IgC2 domain of CEACAM1 has also been implicated in coronavirus and *H. influenzae* receptor activity. Immobilized CEACAM1, in which the tertiary structure of this highly flexible molecule is restrained, also exhibits decreased adhesion, further implicating the cytoplasmic regions of the molecule (e.g., intracellular dimerization) in both homophilic and heterophilic interactions and signaling. Further, the formation of homodimers in *cis* has been characterized for multiple splice variants (isoforms), even those lacking IgC2 domains.

The lack of intradomain disulfide bridges in the N-terminal D1 domain renders the CEACAM1 ectodomain highly flexible. CEACAM1exists in the cell membrane in microclusters; whereas homophilic binding triggers reorganization that results in two different kinds of dimers, as well as trimers and higher-order oligomers. Because of the hinge regions between the Ig domains, antiparallel *trans*-dimers (C-dimers) and parallel cis-dimers (A-dimers) can be formed. The N-terminal D1 domain participates in both C- and A-dimerization, while the D2-D4 domains are involved only in A-dimerization. Divalent cations decreased ectodomain flexibility and enhanced formation of multimeric complexes, which are further implicated in CEACAM1-mediated cell adhesion. Importantly, the dimerization of the ectodomains is transduced by the transmembrane domains to the cytoplasmic domains, thus, in turn, directing intracellular signaling. Another binding site might be implicated across the ABED face, depending on the level and flexibility of glycosylations on the CEACAM1 molecule (Klaile et al., 187 J. Cell Biol. 553 (2009).

CEACAM1 function is also likely impacted by glycosylation. Carbohydrates account for up to sixty percent of the weight of CEACAM1 expressed on the cell surface. The role of this enormous carbohydrate content allows for the construction of "subspecies" of CEACAM1 isoforms. For example, the ability of CEACAM1 to express sialyl Lewis x modifications can affect leukocyte homing (Chen et al., 86 J. Leuk. Biol. 195 (2009).

In some embodiments, an anti-CEACAM1 antibody can bind a CEACAM1 molecule having the sequence set forth in SEQ ID NO: 32 or an allelic or splice variant of SEQ ID NO: 32. Allelic and splice variants of SEQ ID NO: 32 can include those variants described in, e.g. Gaur et al. Molecular Cancer 2008 7:46 Barnett et al. Mol Cell Biol 1993, 13:1273-1282; and Barnett et al. J Cell Biol 1989, 108:267-276.

In some embodiments, an anti-CEACAM1 antibody can bind one or more of the extracellular domains of CEACAM1, e.g. any of amino acids 1-429 of SEQ ID NO: 32. In some embodiments, an anti-CEACAM1 antibody can bind the Ig V-set domain of CEACAM1, e.g. any of amino acids 35-140 of SEQ ID NO: 32. In some embodiments, an anti-CEACAM1 antibody can bind an Ig C-set domain of CEACAM1, e.g. any of amino acids 140-415 of SEQ ID NO: 32. The extracellular domain structure of CEACAM1 variants is further described, for example, in Watt et al. Immunobiology 2001 98:1469-1479.

In some embodiments, an anti-CEACAM1 antibody can bind a CEACAM1 ligand interaction domain. In some embodiments, a CEACAM1 ligand interaction domain can comprise extracellular residues of a CEACAM1 polypeptide. By way of non-limiting example, the homophilic ligand interaction domain of CEACAM1 can comprise amino acids 73, 74, 98, and 116 of SEQ ID NO: 32 and the heterophilic ligand interaction domain of CEACAM1 can comprise amino acids 61, 62, 63, 66, 68,73, 78, 123, and 125 of SEQ ID NO: 32. Residues important for ligand binding in a number of CEACAM1 variants are further described, for example, in Watt et al. Immunobiology 2001 98:1469-1479. In some embodiments, an anti-CEACAM1 antibody can bind to (or physically interact with) one or more of these homophilic ligand interaction amino acids, e.g. amino acids 73, 74, 98, and 116 of SEQ ID NO: 32. In some embodiments, an anti-CEACAM1 antibody can bind to (or physically interact with) one or more of these heterophilic ligand interaction amino acids, e.g. amino acids 61, 62, 63, 66, 68,73, 78, 123, and 125 of SEQ ID NO: 32. In this context, physical interaction can encompass steric hindrance of the interaction of one or more of these sites with a ligand involved in tolerogenesis.

In some embodiments, the anti-CEACAM1 antibody can bind the homophilic interaction domain of CEACAM1 and inhibit homophilic interaction of CEACAM1 molecules. In some embodiments, an anti-CEACAM1 antibody can reduce homophilic binding of CEACAM1. *Trans*-homophilic CEACAM1 binding induces *cis*-dimerization by an allosteric mechanism transmitted by the N terminal immunoglobulin-like domain. CEACAM1-L homodimer formation is reduced by coexpression of CEACAM1-S and modulated by antibody ligation. Transmembrane signaling by CEACAM1 can operate by alteration of the monomer/dimer equilibrium. Müller et al., 187 J. Cell Biol. 569 (2009). Exemplary agents that selectively decrease homophilic CEACAM1 binding are described, for example, in U.S. Patents No. 7,132,255 and No. 6,852,320, and the references cited therein.

Monomeric soluble forms of CEACAM1 have been demonstrated to inhibit CEACAM1 signaling (see, e.g. Markel. EJI 2004 34:2138-2148).

The longest isoform, CEACAM1-4L (isoform 1) has 526 amino acids, having the sequence:

The foregoing amino acid sequences include a leader sequence (34 amino acids), and the mature peptide starting at QLT. The DNA and amino acid sequences of CEACAM1 isoforms of various species are available on-line from the NCBI, including human (ID: 634), mouse (ID: 26365), rat (ID: 81613), cow (ID: 404118), zebra fish (ID: 114465), dog (ID: 612435), goat (ID: 100384959), and orangutan (ID: 100172828).

For example, the secreted human CEACAM1 isoform 2 (UniProtKB P13688-2) has the amino acids:

Secreted human CEACAM1 isoform 3 (UniProtKB P13688-3) has the amino acids:

Secreted human CEACAM1 isoform 4 (UniProtKB P13688-4) has the amino acids:

PD1 (or CD279) is a 288 amino acid type I transmembrane protein composed of one immunoglobulin (Ig) superfamily domain, a 20 amino acid stalk, a transmembrane domain, and an intracellular domain of approximately 95 residues containing an immunoreceptor tyrosine-based inhibitory motif (ITIM), as well as an immunoreceptor tyrosine-based switch motif (ITSM). PD1 is encoded by the *Pdcd1* and *PDCD1* genes on chromosome 1 in mice and chromosome 2 in humans respectively. In both species, *Pdcd1* is encoded by 5 exons. Exon 1 encodes a short signal sequence, whereas exon 2 encodes an Ig domain. The stalk and transmembrane domains make up exon 3, and exon 4 codes for a short 12 amino acid sequence that marks the beginning of the cytoplasmic domain. Exon 5 contains the C-terminal intracellular residues and a long 3'UTR (Keir ME et al., 2008. Annu Rev Immunol. 26:677-704). PD1 is a member of the B7 family of receptors.

Splice variants of PD1 have been cloned from activated human T cells. These transcripts lack exon 2, exon 3, exons 2 and 3, or exons 2 through 4. All these variants, except for the splice variant lacking exon 3 only (PD1Δex3), are expressed at levels similar to full-length PD1 in resting peripheral blood mononuclear cells (PBMCs). All variants are significantly induced upon activation of human T cells with anti-CD3 and anti-CD28 (Keir ME et al., 2008. Annu Rev Immunol. 26:677-704).

Accordingly, the term " PD1" as used herein, refers to the 288 amino acid polypeptide having the amino acid sequence of:
MQIPQAPWPVVWAVLQLGWRPGWFLDSPDRPWNPPTFSPALLVVTEGDNATFTCSFSNTSES FVLNWYRMSPSNQTDKLAAFPEDRSQPGQDCRFRVTQLPNGRDFHMSVVRARRNDSGTYLC GAISLAPKAQIKESLRAELRVTERRAEVPTAHPSPSPRPAGQFQTLVVGVVGGLLGSLVLLVW VLAVICSRAARGTIGARRTGQPLKEDPSAVPVFSVDYGELDFQWREKTPEPPVPCVPEQTEYA TIVFPSGMGTSSPARRGSADGPRSAQPLRPEDGHCSWPL (**SEQ ID NO:1**), as described by, *e.g.,* NP_005009, together with any naturally occurring allelic, splice variants, and processed forms thereof. Typically, PD1 refers to human PD1. The term "PD1" is also used to refer to truncated forms or fragments of the PD1 polypeptide. Reference to any such forms of PD1 can be identified in the application, e.g., by "PD1 (42-136)." For example, the mature PD1 peptide is referred to herein as PD1 (21-288), and PD1 IgV domain as PD1 (42-136). Specific residues of PD1 can be referred to as, for example, "PD1 (68)."

PD1 has been shown to be expressed on T cells, B cells, natural killer T cells, activated monocytes, and dendritic cells (DCs). PD1 is not expressed on resting T cells but is inducibly expressed after activation. Ligation of the T cell receptor or B cell receptor can upregulate PD1 on T and B lymphocytes. In normal human reactive lymphoid tissue, PD1 is expressed on germinal center-associated T cells. PD1 compartmentalization in intracellular stores has been described in a regulatory T cell population. PD1 is inducibly expressed on APCs on myeloid CD11c+ DCs and monocytes in humans (Keir ME et al., 2008. Annu Rev Immunol. 26:677-704.

PD1 has two known ligands, PD-L1 and PD-L2, which are also members of the B7 family. The binding interface of PD1 to PD-L1 is via its IgV-like domain *(i.e.,* PD1 (42-136)). Residues important for binding of PD1 to its ligands include residues 64, 66, 68, 73, 74, 75, 76, 78, 90, 122, 124, 126, 128, 130, 131, 132, 134, and 136. PD-L1/CD274 has been shown to be constitutively expressed on mouse T and B cells, DCs, macrophages, mesenchymal stem cells, and bone marrow-derived mast cells. CD274/PD-L1 expression is also found on a wide range of nonhematopoietic cells and is upregulated on a number of cell types after activation. PD-L1 is expressed on almost all murine tumor cell lines, including PA1 myeloma, P815 mastocytoma, and B16 melanoma upon treatment with IFN-γ. Loss or inhibition of phosphatase and tensin homolog (PTEN), a cellular phosphatase that modifies phosphatidylinositol 3-kinase (PI3K) and Akt signaling, increases post-transcriptional PD-L1 expression in cancers (Keir ME et al., 2008. Annu Rev Immunol. 26:677-704). Residues of PD-L1 important for binding to PD1 include PD-L1 (67), PD-L1 (121), PD-L1 (122), PD-L1 (123), PD-L1 (123), PD-L1 (124), and PD-L1 (126).

PD-L2 expression is more restricted than PD-L1 expression. PD-L2 is inducibly expressed on DCs, macrophages, and bone marrow-derived mast cells. PD-L2 is also expressed on 50% to 70% of resting peritoneal B1 cells, but not on conventional B2 B cells. PD-L2 can also be induced on monocytes and macrophages by GM-CSF, IL-4, and IFN-γ. PD-L2 expression has also been observed on tumor lines.

Also described herein is an anti-PD-L1 antibody reagent and an anti-PD-L2 antibody reagent. In the present context, an anti-PD-L1 antibody reagent or anti-PD-L2 antibody reagent is one that binds to the target ligand and prevents binding of the ligand to PD1.

PD1 and its ligands have been shown to have important roles in regulating immune defenses against microbes that cause acute and chronic infections. The PD1: PD-L pathways appear to play important roles in the outcome of infection, and the regulation of the delicate balance between effective antimicrobial immune defenses and immune-mediated tissue damage. Accordingly, in some embodiments of the aspects described herein, an anti PD1 antibody inhibits or blocks binding of PD1 to its ligands.

A number of microorganisms that cause chronic infection appear to have exploited the PD1: PD-L pathways to evade the immune responses and establish persistent infection. Studies in the lymphocytic choriomeningitis virus (LCMV) model of chronic viral infection were the first to show a role for the PD1:PD-L pathway during chronic infection (Barber DL et al. 2006. Nature 439:682-87). Viruses that cause chronic infections can render virus-specific T cells nonfunctional and thereby silence the antiviral T cell response (Wherry EJ and Ahmed R. 2004. J. Virol. 78:5535-45). Functional dysregulation, also termed herein as "exhaustion," of CD8 T cells is an important reason for ineffective viral control during chronic infections and is characteristic of chronic LCMV infection in mice, as well as of HIV, HBV, HCV, and HTLV infection in humans and SIV infection in primates.

In chronic viral infections in humans, several groups have shown that PD1 expression is high on HIV-specific (Petrovas C et al. 2006. J. Exp. Med. 203:2281-92; Day CL et al. 2006. Nature 443:350-54; Trautmann L et al. 2006. Nat. Med. 12:1198-202), HBV-specific (Boettler T et al. 2006. J. Virol. 80:3532-40; Boni C et al. 2007. J. Virol. 81:4215-25), and HCV-specific T cells (Bengsch B. et al., 2010 PLoS Pathog. 6(6); Urbani S et al. 2006. J. Virol. 80:11398-403). Blocking PD1:PD-L interactions *in vitro* has been shown to reverse the exhaustion of HIV-specific, HBV-specific (Boni C et al. 2007. J. Virol. 81:4215-25), HCV-specific, and SIV-specific (Velu V et al. 2007. J. Virol.81:5819-28) CD8 and CD4 T cells and restores proliferation and cytokine production (Petrovas C et al. 2006. J. Exp. Med. 203:2281-92; Day CL et al. 2006. Nature 443:350-54; Trautmann L et al. 2006. Nat. Med. 12:1198-202; Urbani S et al. 2006. J. Virol. 80:11398-403). Recent work shows that the HCV core, a nucleocapsid protein, can upregulate PD1 and PD-L1 expression on healthy donor T cells and that upregulation of PD1 is mediated by interaction of the HCV core with the complement receptor C1QBP (Yao ZQ et al. 2007. Viral Immunol. 20:276-87).

The PD1:PD-L pathway also can play a key role in the chronicity of bacterial infections. *Helicobacter pylori* causes chronic gastritis and gastroduodenal ulcers and is a risk factor for development of gastric cancer. During *H. pylori* infection, T cell responses are insufficient to clear infection, leading to persistent infection. Gastric epithelial cells express MHC class II molecules and are thought to have important APC (antigen-presenting cell) function during H. pylori infection. Anti-PD-L1 blocking antibodies enhance T cell proliferation and IL-2 production in cultures of gastric epithelial cells exposed to *H. pylori* and CD4 T cells, suggesting that the PD1:PD-L1 pathway can play an important role in inhibiting T cell responses during *H. pylori* infection (Das S et al. 2006. J. Immunol. 176:3000-9.

Parasitic worms also have exploited the PD1:PD-L pathways to induce macrophages with strong suppressive function. During *Taenia crassiceps* infection in mice, a high percentage of CD4 T cells express PD1, and PD-L1 and PD-L2 are upregulated on activated macrophages. Blockade of PD-L1, PD-L2, or PD1 significantly decreased suppression of *in vitro* T cell proliferation by macrophages from *Taenia*-infected mice (Terrazas LI et al. 2005. Int. J. Parasitol. 35:1349-58). Similarly, during *Schistosoma mansoni* infection in mice, macrophages express high levels of PD-L1 and more modest levels of PD-L2. Anti-PD-Ll completely abrogated the ability of these macrophages to suppress T cell proliferation *in vitro,* whereas anti-PD-L2 had no effect (Keir ME et al., 2008. Annu Rev Immunol. 26:677-704). The PD1:PD-L pathways have also been shown to have distinct roles in the immune response to the protozoan parasite *Leishmania mexicana* (Keir ME et al., 2008. Annu Rev Immunol. 26:677-704).

Tumors express antigens that can be recognized by host T cells, but immunologic clearance of tumors is rare. Part of this failure is due to immune suppression by the tumor microenvironment. Recent work has indicated that the PD1: PD-L pathways are involved in suppression of anti-cancer/tumor immune responses. PD1 expression is upregulated on tumor infiltrating lymphocytes, and this can contribute to tumor immunosuppression. PD-L1 expression has been shown in situ on a wide variety of solid tumors, including breast, lung, colon, ovarian, melanoma, bladder, liver, salivary, stomach, gliomas, thyroid, thymic epithelial, head, and neck. In addition, in ovarian cancer, PD-L1 expression is inversely correlated with intraepithelial, but not stromal, infiltrating CD8 T cells, suggesting that PD-L1 inhibits the intratumor migration of CD8 T cells. Also, studies relating PD-L1 expression on tumors to disease outcome show that PD-L1 expression strongly correlates with unfavorable prognosis in kidney, ovarian, bladder, breast, gastric, and pancreatic cancer but not small cell lung cancer (Keir ME et al., 2008. Annu Rev Immunol. 26:677-704).

The PD1 pathway can also play a role in hematologic malignancies. PD1 is highly expressed on the T cells of angioimmunoblastic lymphomas, and PD-L1 is expressed on the associated follicular dendritic cell network. In nodular lymphocyte-predominant Hodgkin lymphoma, the T cells associated with lymphocytic and/or histiocytic (L&H) cells express PD1. PD1 and PD-L1 are expressed on CD4 T cells in HTLV-1-mediated adult T cell leukemia and lymphoma. PD-L2 has been identified as being highly expressed in mantle cell lymphomas. PD-L1 is expressed on multiple myeloma cells but not on normal plasma cells, and T cell expansion in response to myeloma cells is enhanced *in vitro* by PD-L1 blockade. PD-L1 is expressed on some primary T cell lymphomas, particularly anaplastic large cell T lymphomas (Keir ME et al., 2008. Annu Rev Immunol. 26:677-704).

In some embodiments, an anti-PDl antibody can bind a PD1 molecule having the sequence set forth in SEQ ID NO: 1 or an allelic or splice variant of SEQ ID NO: 1. Splice variants of PD1 have been described, for example in Keir et al. Annual Review of Immunology 2008 26:677-704 and Nielsen et al. Cell Immunol 2005 235:109-116.

In some embodiments, an anti-PDl antibody can bind to a PD1 ligand interaction site. In some embodiments, specific binding to a PD1 ligand interaction site modulates interaction of PD1 with PD-L1. In some embodiments, specific binding to a PD1 ligand interaction site modulates interaction of PD1 with PD-L2. In some embodiments, specific binding to a PD1 ligand interaction site modulates interaction of PD1 with PD-L1 and PD-L2. In some embodiments, a ligand interaction site of PD1 can comprise any of amino acid residues 41-136 of SEQ ID NO:1. In some embodiments, a ligand interaction site on PD1 can comprise any of the amino acid residues selected from the group consisting of amino acids 64, 66, 68, 73, 74, 75, 76, 78, 90, 122, 124, 126, 128, 130, 131, 132, 134, and 136 of SEQ ID NO:1. In some embodiments, the ligand interaction site on PD1 comprises any of the amino acid residues selected from the group consisting of amino acids 78, 126, and 136 of SEQ ID NO:1. In some embodiments, an anti-PDl antibody can bind to, or physically interact with any of the amino acid residues selected from the group consisting of amino acids 64, 66, 68, 73, 74, 75, 76, 78, 90, 122, 124, 126, 128, 130, 131, 132, 134, and 136 of SEQ ID NO:1. In some embodiments, an anti-PDl antibody can bind to, or physically interact with, any of the amino acid residues selected from the group consisting of amino acids 41-136 of SEQ ID NO:1. In some embodiments, an anti-PD1 antibody can bind to, or physically interact with, any of the amino acid residues selected from the group consisting of amino acids 78, 126, and 136 of SEQ ID NO:1. In this context, physical interaction can encompass steric hindrance of the interaction of one or more of these sites with a ligand involved in tolerogenesis.

The anti-PDl antibody reagents described herein include PD1 binding site sequences from monoclonal antibodies specific for human PD1, such as, MDX-1106 (ONO-4538), a fully human IgG4 anti-PDl blocking antibody (Journal of Clinical Oncology, 2008 Vol 26, No 15S); CT-011 (CureTech, LTD, previously CT-AcTibody or BAT), a humanized monoclonal IgG1 antibody (Benson DM et al., Blood. 2010 May 11), or those obtained from, clone NAT (Abcam), clone EH12.2H7 (Biolegend), clone J116 (eBioscience), clone MIH4 (eBioscience), clone J105 (eBioscience), or clone 192106 (R& D systems).

Also described herein are compositions and methods which relate to LAP inhibitory agents. As used herein, "latency associated peptide" or "LAP" refers to the amino -terminal domain of the TGFβ3 precursor peptide. When LAP is cleaved from the precursor peptide, it can remain in noncovalent association with TGFβ3, forming the latent complex. LAP can exist as a homodimer of LAP molecules. Sequences of LAP polypeptides are known for a number of species, e.g. human LAP (amino acids 30-278 of SEQ ID NO: 33). Agents that physically interact with, bind to, or sterically occlude ligand binding to these interaction sites can be used to inhibit LAP activity, and particularly LAP activity involved in the establishment of maintenance of tolerogenesis.

LAP contains important residues necessary for the interaction with binding partners, e.g. TGFβ. Cysteines 224 and 226 are important in the intermolecular disulphide bond between two LAPs. Their mutation to serine renders the molecule "active" (Sanderson et al., Proc. Natl. Acad. Sci. USA, 92, 2572-2576 (1995); Brunneret al, Mol. Endocrinol. 6, 1691-1700 (1992); Brunner et al, J. Biol. Chem, 264, 13660-13664 (1989). The RGD motif (245-247) facilitates the interaction with integrins (Munger et al, Mol Biol Cell, 9:2627-2638 (1998; DerynckR, TIBS, 19, 548-553 (1994). Cysteine 33 is important for the disulphide bridge with the third cysteine-rich repeat of latent TGFβ binding protein (LTBP) (Saharinen et al. The EMBO Journal, 15, 245-253 (1996)). Nucleic acid encoding TGFβ is described in U.S. Pat. No. 5,801,231.

In some embodiments, a LAP inhibitory agent can bind a LAP molecule having the sequence set forth in SEQ ID NO: 34 or an allelic or splice variant of SEQ ID NO: 34.

LAP inhibitory antibody reagents are known in the art. See, e.g. Ali et al. PLOS ONE 2008 :e1914. Further examples of anti-LAP antibody reagents are described in U.S. Patent No. 8,198,412 and U.S. Patent Publication No. 2008/0206219.

In some embodiments, a LAP inhibitory agent can bind a LAP ligand interaction site, e.g. a site that interacts with mature TGFβ. Non-limiting examples of such sites include R218 (see, e.g. McGowan et al. The Journal of Clinical Endocrinology and Metabolism 2003 88:3321-6).

Described herein are compositions and methods relating thereto which comprise an inhibitor of LAP (i.e. a LAP inhibitory agent). As used herein, an "inhibitory agent" is an agent that reduces the level and/or activity of a target as compared to the level and/or activity in the absence of the agent by a statistically significant amount. For the avoidance of doubt, reduction by at least 10%, at least 20%, at least 30%, at least 40%, at least 50%, at least 60%, at least 70%, at least 80%, at least 90%, at least 95% or more would be considered inhibitory. The level of a target can be the level of a polypeptide target, the level of an isoform or variant of a polypeptide target, or the level of an RNA transcript encoding the polypeptide target. The activity of a polypeptide target can include, but is not limited to, the ability of the target to bind a normal ligand, the ability of the target to interact with other polypeptides (e.g. downstream signaling partners), and/or the ability of the target polypeptide to effect a downstream response (e.g. phosphorylation levels or gene expression).

A target-specific inhibitory agent (i.e. an inhibitory agent specific for LAP) can include, by way of non-limiting examples, a protein, an antibody, an antibody reagent which binds specifically to the target and inhibits its signaling activity, or a small interfering RNA specific for or targeted to the target-encoding mRNA. As used herein, an "inhibitory agent" can refer to a protein-binding agent that permits inhibition of LAP as described herein. Such agents include, but are not limited to, antibodies ("antibodies" includes antigen-binding portions of antibodies such as epitope- or antigen-binding peptides, paratopes, functional CDRs; recombinant antibodies; chimeric antibodies; tribodies; midibodies; or antigen-binding derivatives, analogs, variants, portions, or fragments thereof), protein-binding agents, small molecules, recombinant protein, peptides, aptamers, avimers and protein-binding derivatives, portions or fragments thereof. Antisense oligonucleotides represent another class of agents that are useful in the compositions and methods described herein. This class of agents and methods for preparing and using them are all well-known in the art, as are ribozyme and miRNA molecules. See, e.g., WO2008/060617 for a thorough discussion.

As used herein, the term "specific binding" refers to a chemical interaction between two molecules, compounds, cells and/or particles wherein the first entity binds to the second, target entity with greater specificity and affinity than it binds to a third entity which is a non-target. In some embodiments, specific binding can refer to an affinity of the first entity for the second target entity which is at least 10 times, at least 50 times, at least 100 times, at least 500 times, at least 1000 times or greater than the affinity for the third nontarget entity

In some embodiments, an inhibitory agent can be an inhibitory nucleic acid, an inhibitory peptide, an inhibitory small molecule, an inhibitory antibody reagent, or inhibitory mimics (e.g. peptidomimetics) or combinations thereof. In some embodiments, an inhibitory agent can be a mimic or variant of the natural ligand of a target, e.g. a fragment or variant of TGFβ that will interact with LAP and prevent the binding of LAP to mature TGFβ.

In some embodiments, an inhibitory agent can be a dominant negative variant of LAP.

In some embodiments, an inhibitory agent (e.g. an antibody reagent) specific for a LAP can specifically bind to the extracellular region of the target. In some embodiments, an inhibitory agent (e.g. an antibody reagent) specific for LAP can specifically bind to a ligand interaction region of the target. In some embodiments, the inhibitory agent can reduce or inhibit the interaction of a ligand with the target. Specific regions of LAP that can be targeted by an inhibitory agent are described elsewhere herein.

As used herein, the term "antibody reagent" refers to a polypeptide that includes at least one immunoglobulin variable domain or immunoglobulin variable domain sequence and which specifically binds a given antigen (e.g. LAP). In some embodiments, an antibody reagent can specifically bind to LAP. In some embodiments, an antibody reagent can inhibit the signaling activity of the target molecule, e.g. inhibit signaling activity of LAP. Methods for measuring the signaling activity of the target molecule are discussed elsewhere herein.

An antibody reagent can comprise an antibody or a polypeptide comprising an antigen-binding domain of an antibody. In some embodiments, an antibody reagent can comprise a monoclonal antibody or a polypeptide comprising an antigen-binding domain of a monoclonal antibody. For example, an antibody can include a heavy (H) chain variable region (abbreviated herein as VH), and a light (L) chain variable region (abbreviated herein as VL). In another example, an antibody includes two heavy (H) chain variable regions and two light (L) chain variable regions. The term "antibody reagent" encompasses antigen-binding fragments of antibodies (e.g., single chain antibodies, Fab and sFab fragments, F(ab')2, Fd fragments, Fv fragments, scFv, and single domain antibody (dAb) fragments (see, e.g. de Wildt et al., Eur J. Immunol. 1996; 26(3):629-39) as well as complete antibodies. An antibody can have the structural features of IgA, IgG, IgE, IgD, IgM (as well as subtypes and combinations thereof). Antibodies can be from any source, including mouse, rabbit, pig, rat, and primate (human and non-human primate) and primatized antibodies. Antibodies also include midibodies, humanized antibodies, chimeric antibodies, and the like.

The VH and VL regions can be further subdivided into regions of hypervariability, termed "complementarity determining regions" ("CDR"), interspersed with regions that are more conserved, termed "framework regions" ("FR"). The extent of the framework region and CDRs has been precisely defined (see, Kabat, E. A., et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917. Each VH and VL is typically composed of three CDRs and four FRs, arranged from amino-terminus to carboxy-terminus in the following order: FR1, CDR1, FR2, CDR2, FR3, CDR3, and FR4.

The terms "antigen-binding fragment" or "antigen-binding domain", which are used interchangeably to refer to one or more fragments of a full length antibody that retain the ability to specifically bind to a target of interest. Examples of binding fragments encompassed within the term "antigen-binding fragment" include (i) a Fab fragment, a monovalent fragment consisting of the VL, VH, CL and CHI domains; (ii) a F(ab')2 fragment, a bivalent fragment including two Fab fragments linked by a disulfide bridge at the hinge region; (iii) an Fd fragment consisting of the VH and CHI domains; (iv) an Fv fragment consisting of the VL and VH domains of a single arm of an antibody, (v) a dAb fragment (Ward et al., (1989) Nature 341:544-546, which consists of a VH or VL domain; and (vi) an isolated complementarity determining region (CDR) that retains specific antigen-binding functionality. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules known as single chain Fv (scFv). See e.g., U.S. Pat. Nos. 5,260,203, 4,946,778, and 4,881,175; Bird et al. (1988) Science 242:423-426; and Huston et al. (1988) Proc. Natl. Acad. Sci. USA 85:5879-5883. Antibody fragments can be obtained using any appropriate technique including conventional techniques known to those of skill in the art. The term "monospecific antibody" refers to an antibody that displays a single binding specificity and affinity for a particular target, e.g., epitope. This term includes a "monoclonal antibody" or "monoclonal antibody composition," which as used herein refer to a preparation of antibodies or fragments thereof of single molecular composition, irrespective of how the antibody was generated.

The term "specificity" refers to the number of different types of antigens or antigenic determinants to which a particular antibody or antigen-binding portion thereof can bind. The specificity of an antibody or antigen-binding portion thereof, alone or in the context of a bispecific or multispecific polypeptide agent, can be determined based on affinity and/or avidity. The affinity, represented by the equilibrium constant for the dissociation (K_{D}) of an antigen with an antigen-binding protein (such as a bispecific or multispecific polypeptide agent), is a measure for the binding strength between an antigenic determinant and an antigen-binding site on the antigen-binding protein: the lesser the value of the K_{D}, the stronger the binding strength between an antigenic determinant and the antigen-binding molecule. Alternatively, the affinity can also be expressed as the affinity constant (K_{A}), which is 1/ K_{D}). As will be clear to the skilled person, affinity can be determined in a manner known per se, depending upon the specific antigen of interest. Accordingly, a bispecific or multispecific polypeptide agent as defined herein is said to be "specific for" a first target or antigen compared to a another target or antigen when it binds to the first antigen with an affinity (as described above, and suitably expressed, for example as a K_{D} value) that is at least 10x, such as at least 100x, and preferably at least 1000x, and up to 10000x or more better than the affinity with which said amino acid sequence or polypeptide binds to the other target or polypeptide. For example, when a bispecific or multispecific polypeptide agent is "specific for" a target or antigen compared to another target or antigen, it is directed against said target or antigen, but not directed against such other target or antigen.

Avidity is the measure of the strength of binding between an antigen-binding molecule (such as a bispecific polypeptide agent described herein) and the pertinent antigen. Avidity is related to both the affinity between an antigenic determinant and its antigen binding site on the antigen-binding molecule, and the number of pertinent binding sites present on the antigen-binding molecule. Typically, antigen-binding proteins (such as a bispecific polypeptide agent described herein) will bind to their cognate or specific antigen with a dissociation constant (K_{D} of 10⁻⁵ to 10⁻¹² moles/liter or less, such as 10⁻⁷ to 10⁻¹² moles/liter or less or 10⁻⁸ to 10⁻¹² moles/liter (i.e., with an association constant (K_{A}) of 10⁵ to 10¹² liter/moles or more, and such as 10⁷ to 10¹² liter/moles or 10⁸ to 10¹² liter/moles). Any K_{D} value greater than 10⁻⁴ mol/liter (or any K_{A} value lower than 10⁴ M⁻¹) is generally considered to indicate non-specific binding. The K_{D} for biological interactions which are considered meaningful (e.g., specific) are typically in the range of 10⁻¹⁰ M (0.1 nM) to 10⁻⁵ M (10000 nM). The stronger an interaction is, the lower is its K_{D}. A binding site on a bispecific or multispecific polypeptide agent described herein may bind to the desired antigen with an affinity less than 500 nM, less than 200 nM, or less than 10 nM, such as less than 500 pM. Specific binding of an antigen-binding protein to an antigen or antigenic determinant can be determined in any suitable manner known per se, including, for example, Scatchard analysis and/or competitive binding assays, such as radioimmunoassays (RIA), enzyme immunoassays (EIA) and sandwich competition assays, and the different variants thereof known per se in the art; as well as other techniques as mentioned herein.

Accordingly, as used herein, "selectively binds" or "specifically binds" refers to the ability of a polypeptide domain described herein to bind to a target, such as a molecule present on the cell-surface, with a K_{D} of 10⁻⁵ M (10000 nM) or less, e.g., 10⁻⁶ M or less, 10⁻⁷ M or less, 10⁻⁸ M or less, 10⁻⁹ M or less, 10⁻¹⁰ M or less, 10⁻¹¹ M or less, or 10⁻¹² M or less. For example, if a polypeptide agent described herein binds to CEACAM1 with a K_{D} of 10⁻⁵ M or lower, but not to another CEACAM molecule, such as CEACAM5, or a related homologue, then the agent is said to specifically bind CEACAM1. Specific binding can be influenced by, for example, the affinity and avidity of the polypeptide agent and the concentration of polypeptide agent. The person of ordinary skill in the art can determine appropriate conditions under which the polypeptide agents described herein selectively bind the targets using any suitable methods, such as titration of a polypeptide agent in a suitable cell binding assay.

In some embodiments, humanized or composite human antibody reagents can be used in the compositions and methods described herein. For example, in some embodiments of the compositions and methods described herein, a particular isolated recombinant antibody or antigen-binding portion thereof that binds specifically to CEACAM1 can comprise a heavy chain complementarity determining region (CDR) 1 consisting of the amino acid residues SSHGMS (SEQ ID NO: 2), a heavy chain CDR2 consisting of the amino acid residues TISSGGTYTYYPDSVKG (SEQ ID NO: 3), a heavy chain CDR3 consisting of the amino acid residues HDFDYDAAWFAY (SEQ ID NO: 4), a light chain CDR1 consisting of the amino acid residues SANSSVSYMY (SEQ ID NO: 5), a light chain CDR2 consisting of the amino acid residues LTSNLAS (SEQ ID NO: 6), and a light chain CDR3 consisting of the amino acid residues QQWSSNPPT (SEQ ID NO: 7).

Alternatively, in some embodiments of the compositions and methods described herein, the CEACAM1-binding antibody or epitope-binding peptide can be constructed to have CDR regions selected from the following: a heavy chain CDR 1 consisting of the amino acid residues SSHGMS (SEQ ID NO:8), SFYGMS (SEQ ID NO: 9), or SDYYLY (SEQ ID NO:10); a heavy chain CDR2 consisting of the amino acid residues TISSGGTYTYYPDSVKG (SEQ ID NO:11), TFSGGGNYTYYPDSVKG (SEQ ID NO:12) or TISVGGGNTSYPDSVKG (SEQ ID NO: 13); a heavy chain CDR3 consisting of the amino acid residues HDFDYDAAWFAY (SEQ ID NO: 14), or HGGLPFYAMDY (SEQ ID NO: 15), or GLTTGPAWFAY (SEQ ID NO: 16); a light chain CDR1 consisting of the amino acid residues SANSSVSYMY (SEQ ID NO: 17), SVSSSISSSNLH (SEQ ID NO: 18), KSSQSLLNSSNQKNYLA (SEQ ID NO: 19), or RASQKISGYLS (SEQ ID NO: 20); a light chain CDR2 consisting of the amino acid residues LTSNLAS (SEQ ID NO: 21), SVSSSISSSNLH (SEQ ID NO: 22), FASTRES (SEQ ID NO: 23), or AASTLDS (SEQ ID NO: 24); and a light chain CDR3 consisting of the amino acid residues QQWSSNPPT (SEQ ID NO: 25), QQWSSHPFT (SEQ ID NO: 26), QQHYSTPWT (SEQ ID NO: 27) or LQYASSLMYT (SEQ ID NO: 28). See also U.S. Patent Pub. No. 2004/0047858, for example.

Other anti-CEACAM1 monoclonal antibodies useful in the compositions and methods described herein include AgB10, that inhibits CEACAM1-mediated EAE suppression in mice (Fujita et al., 175 Am. J. Pathol. 1116 (2009)); a CEACAM1 antibody (Chen et al., 2004); murine D14HD11 (Yu et al., 281 J. Biol. Chem. 39179 (2006)); murine CEACAM1-specific CC1 (Iijima et al., 199 J. Exp. Med. 471 (2004)); mouse anti-human CEACAM1 MRG-1 (Ortenberg 2012); mouse anti-human CEACAM1 N-domain specific antibodies 5F4, 34B1, and 26H7 (IgG1) (Morales et al., 1999) each of which recognizes the N-terminal domain of CEACAM1 (Watt et al., 2001); and 12-140-4,4/3/17, COL-4, YG-C28F2, D14HD11, B18.7.7, D11-AD11, HEA 81, CLB-gran-10, F34-187, T84.1, B6.2, and B1.1. Monoclonal antibodies 34B1, 26H7, and 5F4 are also discussed in U.S. Patent Pub. No. 2004/0047858, *Therapeutic anti-BGP(C-CAM1) antibodies and uses thereof,* U.S. Patent No. 7,132,255; WO 99/52552, and Morales et al., 163 J. Immunol. 1363 (1999). Further assessment of specificity was published in Watt et al., 2001, which described that monoclonal antibody 5F4 binds to a domain within the N-Domain of CEACAM1 that is involved in homophilic interactions between the N-Domains of different CEACAM1 molecules.

Other CEACAM1-Fc fusion proteins have been described, including, for example, the extracellular portion of CEACAM1 fused to human IgFc in a mammalian expression vector (Markel et al., 110 J. Clin. Invest. 943 (2002)); pCEP4-N-CEACAM1-Fc see Gallagher, 71 J. Virol. 3129 (1997)); as well as commercially available sources of CEACAM1 fusion proteins, as known to one of ordinary skill in the art.

Exemplary agents that bind CEACAM1, and methods for identifying such agents and whether such agents enhance or suppress T-cell activity are found, for example, in U.S. Patents No. 7,132,255 and No. 6,852,320, and the references cited therein.

Also described herein are bispecific inhibitory agents, e.g. bispecific polypeptide agents. Bispecific agents comprise a molecule which is able to physically contact and inhibit at least two of CEACAM1, PD1, and/or LAP simultaneously. As used herein, the term "bispecific polypeptide agent" refers to a polypeptide that comprises a first polypeptide domain which has a binding site that has binding specificity for a first target, and a second polypeptide domain which has a binding site that has binding specificity for a second target, i.e., the agent has specificity for two targets, e.g., CEACAM1 and PD1. The first target and the second target are not the same (i.e., are different targets (e.g., proteins)). In some examples, the different targets can be co-expressed on the same cell. In some examples, a bispecific polypeptide agent can bind targets present on a single cell (heterophilic binding in *cis*)*,* and/or bind one target on one cell and the other on another cell (heterophilic binding in *trans*)*.* Accordingly, a bispecific polypeptide agent as described herein can selectively and specifically bind to a cell that expresses the first target and the second target. One example of a bispecific polypeptide agent is a bispecific antibody construct. Bispecific antibody constructs comprising antigen-binding portions of antibodies specific for two different antigens, e.g., PD1 and CEACAM1, can be readily constructed by one of skill in the art. Generally, sequences encoding the antigen-binding domain of a first antibody characterized and known to bind a desired epitope on one antigen can be joined, either directly, or through any of a variety of linkers as known to the ordinarily skilled artisan, to sequences encoding the antigen-binding domain of a second antibody characterized and known to bind a desired epitope on a second antigen. Such sequences can be inserted into an appropriate vector and introduced to a cell to produce the bispecific antibody polypeptide by methods known to those of ordinary skill in the art.

Also described herein is a trispecific agent, i.e. an agent having specificity for all of CEACAM1, PD1, and LAP. As used herein, the term "trispecific polypeptide agent" refers to a polypeptide that comprises at least a first polypeptide domain having a binding site that has binding specificity for a first target, a second polypeptide domain having a binding site that has binding specificity for a second target, and a third polypeptide domain having a binding site that has binding specificity for a third target e.g., CEACAM1, PD1, and LAP. The first, second, and third target are not the same (i.e., are different targets (e.g., proteins)), but can all be present (e.g., co-expressed) on a cell.

A multispecific polypeptide agent as described herein can bind one or more additional targets, i.e., a multispecific polypeptide can bind at least two, at least three, at least four, at least five, at least six, or more targets, wherein the multispecific polypeptide agent has at least two, at least, at least three, at least four, at least five, at least six, or more target-specific binding sites respectively. A multispecific polypeptide agent has the potential to bind a cell that expresses all the targets the agent is specific for more strongly (e.g., with greater avidity) than a cell that expresses only one target, or less targets than the agent is specific for. A non-limiting example of a multispecific polypeptide agent is a multispecific antibody or antigen-binding fragment thereof. For the avoidance of doubt, a bispecific polypeptide agent or a trispecific polypeptide agent is a type or subset of multispecific polypeptide agent.

It is noted that a multispecific antibody reagent, e.g. a bispecific antibody reagent, can preferentially bind to a cell expressing each of the target antigens for which the reagent is specific. While, for example, a bispecific antibody reagent can bind to two separate cells, each of which expresses one of the target antigens, it can be more specific for cells which express both of the target antigens on the same cell surface.

In some embodiments, the antibody reagents provided herein, or used in the methods described herein, can further comprise post-translational modifications. Exemplary post-translational polypeptide modifications include phosphorylation, acetylation, methylation, ADP-ribosylation, ubiquitination, glycosylation, carbonylation, sumoylation, biotinylation or addition of a polypeptide side chain or of a hydrophobic group. As a result, the modified polypeptides can contain non-amino acid elements, such as lipids, poly- or mono-saccharide, and phosphates. Such molecules can also be referred to as derivatives.

Reagents for use in the compositions and methods described herein include those that are conjugated or associated with polymers, such as PEG, that can enhance the half-life of the peptide *in vivo.* PEG modification is well-known in the art. See, e.g., WO2008/060617.

As used herein, the term "target" refers to a biological molecule (e.g., peptide, polypeptide, protein, lipid, carbohydrate) to which a polypeptide domain or other moiety (e.g. a small molecule or aptamer among others) which has a binding site can selectively bind. The target can be, for example, an intracellular target (e.g., an intracellular protein target) or a cell surface target (e.g., a membrane protein, a receptor protein).

As used herein an "antibody" refers to IgG, IgM, IgA, IgD or IgE molecules or antigen-specific antibody fragments thereof (including, but not limited to, a Fab, F(ab')₂, Fv, disulphide linked Fv, scFv, single domain antibody, closed conformation multispecific antibody, disulphide-linked scfv, diabody), whether derived from any species that naturally produces an antibody, or created by recombinant DNA technology; whether isolated from serum, B-cells, hybridomas, transfectomas, yeast or bacteria.

As described herein, an "antigen" is a molecule that is bound by a binding site on a polypeptide agent. Typically, antigens are bound by antibody ligands and are capable of raising an antibody response *in vivo.* An antigen can be a polypeptide, protein, nucleic acid or other molecule or portion thereof. Generally, the bispecific or multispecific polypeptide agents described herein are selected for target specificity against two or more particular antigens *(i.e.,* CEACAM1, PD1, and/or LAP). In the case of conventional antibodies and fragments thereof, the antibody binding site as defined by the variable loops (LI, L2, L3 and HI, H2, H3) is capable of binding to the antigen. The term "antigenic determinant" refers to an epitope on the antigen recognized by an antigen-binding molecule (such as bispecific polypeptide agent described herein), and more particularly, by the antigen-binding site of said molecule.

As used herein, an "epitope" can be formed both from contiguous amino acids, or noncontiguous amino acids juxtaposed by folding of a protein. Epitopes formed from contiguous amino acids are typically retained on exposure to denaturing solvents, whereas epitopes formed by folding are typically lost on treatment with denaturing solvents. An epitope typically includes at least 3, and more usually, at least 5, about 9, or about 8-10 amino acids in a unique spatial conformation. An "epitope" includes the unit of structure conventionally bound by an immunoglobulin V_{H}/V_{L} pair. Epitopes define the minimum binding site for an antibody, and thus represent the target of specificity of an antibody. In the case of a single domain antibody, an epitope represents the unit of structure bound by a variable domain in isolation. The terms "antigenic determinant" and "epitope" can also be used interchangeably herein.

As used herein, "immunoglobulin" refers to a family of polypeptides that retain the immunoglobulin fold characteristic of antibody molecules, which comprise two β sheets and, usually, a conserved disulphide bond. Members of the immunoglobulin superfamily are involved in many aspects of cellular and non-cellular interactions *in vivo,* including widespread roles in the immune system (for example, antibodies, T-cell receptor molecules and the like), involvement in cell adhesion (for example the ICAM molecules) and intracellular signaling (for example, receptor molecules, such as the PDGF receptor).

A "target site" or "ligand interaction site" on the target molecule means a site, epitope, antigenic determinant, part, domain or stretch of amino acid residues on the target that is a site for binding to a ligand, receptor or other binding partner, a catalytic site, a cleavage site, a site for allosteric interaction, a site involved in multimerisation (such as homodimerization or heterodimerization) of the target; or any other site, epitope, antigenic determinant, part, domain or stretch of amino acid residues on the target or antigen that is involved in a biological action or mechanism of the target, e.g., PD1, LAP and/or CEACAM1. More generally, a "ligand interaction site" can be any site, epitope, antigenic determinant, part, domain or stretch of amino acid residues on a target or antigen to which a binding site of an agent, or a bispecific or multispecific agent described herein can bind such that the target (and/or any pathway, interaction, signaling, biological mechanism or biological effect in which the target or antigen is involved), e.g., the signaling of any of CEACAM1, PD1, and/or LAP is inhibited.

As used herein, a "blocking" antibody or an antibody "antagonist" is one which inhibits or reduces biological activity of the antigen(s) it binds. For example, a CEACAM1/PD1 bispecific antagonist antibody binds CEACAM1 and PD1 and inhibits the ability of CEACAM1 and PD1 to induce or maintain T-cell tolerance.

As used herein "domain" refers to a folded protein structure which retains its tertiary structure independently of the rest of the protein. Generally, domains are responsible for discrete functional properties of proteins, and in many cases can be added, removed or transferred to other proteins without loss of function or properties of the remainder of the protein to which it is added or transferred and/or of the domain itself. In the context of an antibody, or a portion thereof, the term "binding domain" refers to such a domain that is directed against an antigenic determinant. By "single antibody variable domain" is meant a folded polypeptide domain comprising sequences characteristic of antibody variable domains. It therefore includes complete antibody variable domains and modified variable domains, for example in which one or more loops have been replaced by sequences which are not characteristic of antibody variable domains, or antibody variable domains which have been truncated or comprise N- or C-terminal extensions, as well as folded fragments of variable domains which retain at least in part the binding activity and specificity of the full-length domain. Thus, a bispecific polypeptide agent will comprise at least two different binding domains

Polyclonal antibodies specific for CEACAM1, LAP, and/or PD1 can be produced by various procedures well known in the art. For example, PD1 polypeptides or fragments thereof, such as a fragment comprising amino acid residues 42-136 of SEQ ID NO:1, can be administered to various host animals including, but not limited to, rabbits, mice, rats, etc. to induce the production of sera containing polyclonal antibodies specific for the protein. Polyclonal antibodies are preferably raised in animals by multiple subcutaneous (sc) or intraperitoneal (ip) injections of the relevant antigen, e.g., a PD1 fragment and an adjuvant. It can be useful to conjugate the antigen to a protein that is immunogenic in the species to be immunized, e.g., keyhole limpet hemocyanin, serum albumin, bovine thyroglobulin, or soy-bean trypsin inhibitor using a bifunctional or derivatizing agent, for example, maleimidobenzoyl sulfosuccinimide ester (conjugation through cysteine residues), N-hydroxy-succinimide (through lysine residues), glutaraldehyde, succinic anhydride, SOCl₂, or R ¹N=C=NR, where R and R¹ are different alkyl groups.

Animals can be immunized against the antigen, immunogenic conjugates, or derivatives by combining, e.g., the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Various other adjuvants can be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum.

Monoclonal antibodies can be prepared using a wide variety of techniques known in the art including the use of hybridoma, recombinant, and phage display technologies, or a combination thereof. Various methods for making monoclonal antibodies described herein are available in the art. For example, the monoclonal antibodies can be made using the hybridoma method first described by Kohler et al., Nature, 256:495 (1975) or adaptations thereof, or by recombinant DNA methods (U.S. Pat. No. 4,816,567). For example, monoclonal antibodies can be produced using hybridoma techniques including those known in the art and taught, for example, in Harlow et al., Antibodies: A Laboratory Manual, (Cold Spring Harbor Laboratory Press, 2nd ed., 1988); Hammerling, et al., in: Monoclonal Antibodies and T-Cell Hybrido-mas 563-681 (Elsevier, N.Y., 1981). The term "monoclonal antibody" as used herein is not limited to antibodies produced through hybridoma technology. It is to be understood that the term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art. In a non-limiting example, mice can be immunized with, e.g. PD1, or a fragment or derivative thereof, such as a fragment comprising amino acid residues 42-136 of SEQ ID NO:1, or a cell expressing PD1 or a fragment thereof. Once an immune response is detected, e.g., antibodies specific for PD1 are detected in the mouse serum, the mouse spleen is harvested and splenocytes isolated. The splenocytes are then fused by well-known techniques to any suitable myeloma cells, for example cells from cell line SP20 available from the ATCC. Hybridomas are selected and cloned by limiting dilution. The hybridoma clones are then assayed by methods known in the art for cells that secrete antibodies capable of binding PD1 and exerting a cytotoxic or cytostatic effect on activated lymphocytes. Ascites fluid, which generally contains high levels of antibodies, can be generated by injecting mice with positive hybridoma clones.

In the hybridoma method, a mouse or other appropriate host animal, such as a hamster or macaque monkey, is immunized as described above to elicit lymphocytes that produce or are capable of producing antibodies that will specifically bind to the protein used for immunization. Alternatively, lymphocytes can be immunized *in vitro.* Lymphocytes then are fused with myeloma cells using a suitable fusing agent, such as polyethylene glycol, to form a hybridoma cell (Goding, Monoclonal Antibodies: Principles and Practice, pp. 59-103 (Academic Press, 1986).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, Calif. USA, and SP-2 or X63-Ag8-653 cells available from the American Type Culture Collection, Rockville, Md. USA. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

After hybridoma cells are identified that produce antibodies of the desired specificity, affinity, and/or activity, the clones can be subcloned by limiting dilution procedures and grown by standard methods (Goding, Monoclonal Antibodies: Principles and Practice, pp. 59-103 (Academic Press, 1986)). Suitable culture media for this purpose include, for example, D-MEM or RPMI-1640 medium. In addition, the hybridoma cells can be grown *in vivo* as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies can be readily isolated and sequenced using conventional procedures (e.g., by PCR or by using oligonucleotide probes that are capable of binding specifically to genes encoding the heavy and light chains of the monoclonal antibodies). The hybridoma cells serve as a preferred source of such DNA. Once isolated, the DNA can be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells. Recombinant production of antibodies is described in more detail below.

In another example, antibodies useful in the methods and compositions described herein can also be generated using various phage display methods known in the art, such as isolation from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

In phage display methods, functional antibody domains are displayed on the surface of phage particles which carry the nucleic acid sequences encoding them. In a particular embodiment, such phage can be utilized to display antigen binding domains expressed from a repertoire or combinatorial antibody library (e.g. human or murine). In phage display methods, functional antibody domains are displayed on the surface of phage particles which carry the nucleic acid sequences encoding them. In particular, DNA sequences encoding V_{H} and V_{L} domains are amplified from animal cDNA libraries (e.g., human or murine cDNA libraries of lymphoid tissues). The DNA encoding the V_{H} and V_{L} domains are recombined together with an scFv linker by PCR and cloned into a phagemid vector (*e.g.,* p CANTAB 6 or pComb 3 HSS). The vector is electroporated into *E. coli* and the *E. coli* is infected with helper phage. Phage used in these methods are typically filamentous phage including fd and M13 binding domains expressed from phage with Fab, Fv or disulfide stabilized Fv antibody domains recombinantly fused to either the phage gene III or gene VIII protein. Phage expressing an antigen binding domain that binds to PD1, CEACAM1, or LAP or portions thereof can be selected or identified with antigen e.g., using labeled antigen or antigen bound or captured to a solid surface or bead. Examples of phage display methods that can be used to make the antibodies described herein include those disclosed in Brinkman et al, 1995, J. Immunol. Methods 182:41-50; Ames et al., 1995, J. Immunol. Methods 184:177-186; Kettleborough et al, 1994, Eur. J. Immunol. 24:952-958; Persic et al., 1997, Gene 187:9-18; Burton et al., 1994, Advances in Immunology, 191-280; PCT Publications WO 90/02809; WO 91/10737; WO 92/01047; WO 92/18619; WO 93/1 1236; WO 95/15982; WO 95/20401; and U.S. Pat. Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As is described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')₂ fragments can also be employed using methods known in the art such as those disclosed in PCT publication WO 92/22324; Mullinax et al, BioTechniques 1992, 12(6):864-869; and Sawai et al, 1995, AJRI 34:26-34; and Better et al., 1988, Science 240:1041-1043.

Simple binding assays can be used to screen for or detect agents that bind to any of CEACAM1, PD1, and/or LAP, and/or reduce the signaling activity of any of the foregoing. Because CEACAM1 and PD1 are transmembrane proteins, assays that use the soluble forms of these proteins rather than full-length protein can be used, in some embodiments. Soluble forms include, for example, those lacking the transmembrane domain and/or those comprising the IgV domain or fragments thereof which retain their ability to bind their cognate binding partners.

Further, agents that inhibit CEACAM1, PD1, and/or LAP for use in the compositions and methods described herein, can be identified by, for example, measuring the activation of the signaling pathways controlled by CEACAM1, PD1 and/or LAP. By way of non-limiting example, the expression of genes induced or suppressed by the activation of CEACAM1, PD1, and/or LAP signaling can be measured (e.g. by quantitative RT-PCR) or the level of phosphorylation of downstream signaling partners can be measured (e.g. by immunochemistry). Such markers for each of these pathways, and methods of measuring their levels are known in the art. Exemplary markers include but are not limited to: ZAP-70 phosphorylation and the level of SHP1 bound to CEACAM1 as markers for CEACAM1 signaling (see, e.g. Chen et al. Journal of Immunology 2008 6085-6093), SHP2 phosophorylation levels as markers for PD1 signaling (see, e.g. Yamamoto et al. Blood 2008 111:3220-4), and the level of phosphorylated SMAD2 and SMAD3 as markers for LAP signaling (see, e.g. Fleisch et al. Endocrine-Related Cancer 2006 13:379-400).

Another variation of assays to determine binding of an inhibitory agent to, e.g. a CEACAM1 protein is through the use of affinity biosensor methods. Such methods may be based on the piezoelectric effect, electrochemistry, or optical methods, such as ellipsometry, optical wave guidance, and surface plasmon resonance (SPR).

As another approach, efficacy of, e.g. an siRNA on the expression of PD1, LAP, and/or CEACAM1 can be monitored using methods known in the art, such as quantitative RT-PCR with specific oligonucleotide primers for each gene respectively, or ELISA for PD1, LAP, and/or CEACAM1. Alternatively, the expression of PD1, LAP and/or CEACAM1 in a population of T cells can be determined by FACS analysis using the markers characteristic of particular populations and subpopulations known in the art or disclosed herein.

Also described herein are methods for enhancing an immune response *in vivo,* comprising administering to the subject a therapeutically effective amount of one or more agents that inhibit CEACAM1, LAP, and/or PD1, as described herein. Modulation of T-cell tolerance by such agents is useful for specifically enhancing an immune response *in vivo,* which can be useful for the treatment of conditions related to immune function including cancer and chronic infections. Modulation of T-cell tolerance also is useful in *in vitro* or non-therapeutic applications including determining whether T-cells of a subject are functional (e.g., proliferation and/or cytotoxic functions), to determine if a treatment has rendered T-cells non-functional, in experimental models of cancer, e.g., to determine the effects of increases or decreases in T-cell function on particular organs or physiological processes, and to test for agents which increase or decrease T-cell activity. Other uses will be apparent to one of ordinary skill in the art. The agents that inhibit PD1, LAP and/or CEACAM1 can be used alone as a primary therapy or in combination with other therapeutics as a combination therapy to enhance the therapeutic benefits of other medical treatments.

CEACAM1, PD1, and/or LAP activity is "decreased" if one or more signaling activities or downstream read-outs of CEACAM1, PD1, and/or LAP activity is reduced or altered by a statistically significant amount, such as by at least 10%, at least 20%, at least 30%, at least 40%, at least 50%,a t least 60%, at least 70%, at least 80%, at least 90%, at least 95%, at least 97%, at least 98%, or more, up to and including 100%, in the presence of an agent or stimulus relative to the absence of such modulation. As will be understood by one of ordinary skill in the art, in some embodiments, if CEACAM1, PD1, and/or LAP activity is decreased, some downstream read-outs will decrease but others can increase (i.e. things that are normally suppressed by CEACAM1, PD1, and/or LAP activity).

As will be clear to the skilled person, a decrease in activity of a target can also involve effecting a change (which can either be an increase or a decrease) in affinity, avidity, specificity and/or selectivity of a target or antigen, such as CEACAM1, PD1, or LAP, for one or more of its ligands, binding partners, partners for association into a homomultimeric or heteromultimeric form, or substrates; and/or effecting a change (which can either be an increase or a decrease) in the sensitivity of the target or antigen, such as CEACAM1, PD1, or LAP, for one or more conditions in the medium or surroundings in which the target or antigen is present (such as pH, ion strength, the presence of cofactors, etc.), compared to the same conditions but without the presence of an inhibitory agent. Again, this can be determined in any suitable manner and/or using any suitable assay known per se or described herein, depending on the target involved. A decrease in the activity of a target can also mean effecting a change (i.e., in an activity as an agonist, as an antagonist or as a reverse agonist, respectively, depending on the target or antigen, such as CEACAM1, PD1, and/or LAP, and the desired biological or physiological effect) with respect to one or more biological or physiological mechanisms, effects, responses, functions, pathways or activities in which the target or antigen (or in which its substrate(s), ligand(s) or pathway(s) are involved, such as its signaling pathway or metabolic pathway and their associated biological or physiological effects) is involved. Again, as will be clear to the skilled person, such an action as an agonist or an antagonist can be determined in any suitable manner and/or using any suitable (*in vitro* and usually cellular or in assay) assay known per se or described herein, depending on the target or antigen involved.

A decrease in the activity of a target can, for example, also involve allosteric modulation of the target, (such as CEACAM1, PD1, and/or LAP); and/or reducing or inhibiting the binding of the target to one of its substrates or ligands and/or competing with a natural ligand, substrate for binding to the target. A decrease in the activity of a target can, for example, also involve effecting a change in respect of the folding or confirmation of the target, or with respect to the ability of the target to fold, to change its conformation (for example, upon binding of a ligand), to associate with other (sub)units, or to disassociate. A decrease in the activity of a target can, for example, also involve effecting a change in the ability of the target to signal, phosphorylate, dephosphorylate, and the like.

Immunosuppression of a host immune response plays a role in a variety of chronic immune conditions, such as in persistent infection and tumor immunosuppression. Recent evidence indicates that this immunosuppression can be mediated by immune inhibitory receptors expressed on the surface of an immune cell, and their interactions with their ligands. For example, cytotoxic CD8 T cells can enter a state of "functional exhaustion," or "unresponsiveness" whereby they express inhibitory receptors that prevent antigen-specific responses, such as proliferation and cytokine production. Accordingly, by inhibiting the activity and/or expression of such inhibitory receptors, an immune response to a persistent infection or to a cancer or tumor that is suppressed, inhibited, or unresponsive, can be enhanced or uninhibited.

As used herein, an "immune response" refers to a response by a cell of the immune system, such as a B cell, T cell (CD4 or CD8), regulatory T cell, antigen-presenting cell, dendritic cell, monocyte, macrophage, NKT cell, NK cell, basophil, eosinophil, or neutrophil, to a stimulus. In some embodiments, the response is specific for a particular antigen (an "antigen-specific response"), and refers to a response by a CD4 T cell, CD8 T cell, or B cell via their antigen-specific receptor. In some embodiments, an immune response is a T cell response, such as a CD4+ response or a CD8+ response. Such responses by these cells can include, for example, cytotoxicity, proliferation, cytokine or chemokine production, trafficking, or phagocytosis, and can be dependent on the nature of the immune cell undergoing the response.

As used herein, "unresponsiveness" or "functional exhaustion" with regard to immune cells includes refractivity of immune cells to stimulation, such as stimulation via an activating receptor or a cytokine. Unresponsiveness can occur, for example, because of exposure to immunosuppressants, exposure to high or constant doses of antigen, or through the activity of inhibitor receptors or factors, such as CEACAM1, PD1, and/or LAP. As used herein, the term "unresponsiveness" includes refractivity to activating receptor-mediated stimulation. Such refractivity is generally antigen-specific and persists after exposure to the antigen has ceased. Unresponsive immune cells can have a reduction of at least 10%, 20%, 30%, 40%, 50%, 60%, 70%, 80%, 90%, 95%, or even 100% in cytotoxic activity, cytokine production, proliferation, trafficking, phagocytotic activity, or any combination thereof, relative to a corresponding control immune cell of the same type.

As used herein, the term "reduces T cell tolerance" means that a given treatment or set of conditions leads to reduced T cell tolerance, i.e. greater T cell activity, responsiveness, and/or ability or receptiveness with regards to activation. Methods of measuring T cell activity are known in the art. By way of non-limiting example, T cell tolerance can be induced by contacting T cells with recall antigen, anti-CD3 in the absence of costimulation, and/or ionomycin. Levels of, e.g. LDH-A, RAB10, and/or ZAP70 (both intracellular or secreted) can be monitored, for example, to determine the extent of T cell tolerogenesis (with levels of IL-2, interferon-y and TNF correlating with increased T cell tolerance). The response of cells pre-treated with, e.g. ionomycin, to an antigen can also be measured in order to determine the extent of T cell tolerance in a cell or population of cells, e.g. by monitoring the level of secreted and/or intracellular IL-2 and/or TNF-α (see, e.g. Macian et al. Cell 2002 109:719-731. A compound known to reduce T cell tolerance which can be useful as a control, but which has extremely limited applicability in the clinic can include CTLA4. In one embodiment, a compound known to reduce T cell tolerance is not CTLA4.

Other characteristics of T cells having undergone adaptive tolerance is that they have increased levels of Fyn and ZAP-70/Syk, Cbl-b, GRAIL, Ikaros, CREM (cAMP response element modulator), B lymphocyte-induced maturation protein-1 (Blimp-1), PD1, CD5, and SHP2; increased phosphorylation of ZAP-70/Syk, LAT, PLCγ1/2, ERK, PKC-Θ/IKBA; increased activation of intracellular calcium levels; decreased histone acetylation or hypoacetylation and/or increased CpG methylation at the IL-2 locus. Thus, in some embodiments, reduction of one or more of any of these parameters can be assayed to determine whether one or more agents that inhibits CEACAM1, LAP, and/or PD-1 enhances an immune response in vivo or decreases immune tolerance.

Reduction of T cell tolerance can also be measured by determining the proliferation of T cells in the presence of a relevant antigen assayed, e.g. by a ³H-thymidine incorporation assay or cell number. Markers of T cell activation after exposure to the relevant antigen can also be assayed, e.g. flow cytometry analysis of cell surface markers indicative of T cell activation (e.g. CD69, CD30, CD25, and HLA-DR). Reduced T cell activation in response to antigen-challenge is indicative of tolerance induction. Conversely, increased T cell activation in response to antigen-challenge is indicative of reduced tolerance.

Reduction of T cell tolerance can also be measured, in some embodiments, by determining the degree to which the inhibitory agent inhibits the activity of its target. By way of a non-limiting example, the activity of a CEACAM1 inhibitory agent can be determined using the staphylococcal enterotoxin B (SEB) model. SEB typically stimulates CEACAM1, resulting in upregulation of TIM3. In the presence of a CEACAM1 inhibitory agent, the upregulation of TIM3 in response to SEB will be reduced.

The SEB model can also be adapted to measure T cell tolerance and modulation thereof. In normal mice, neonatal injection of staphylococcal enterotoxin B (SEB) induces tolerance in T cells that express reactive T cell receptor (TCR) V beta regions. If, in the presence of a CEACAM1, PD1, and/or LAP inhibitory agent, T cells expressing reactive TCR V beta regions (e.g., Vbeta8) display a statistically significant greater T cell activity than T cells not contacted with the inhibitory agent, the inhibitory agent is one that reduces T cell tolerance.

Other *in vivo* models of peripheral tolerance that can be used to measure reduction(s) in T cell tolerance using the inhibitory agents described herein include, for example, models for peripheral tolerance in which homogeneous populations of T cells from TCR transgenic and double transgenic mice are transferred into hosts that constitutively express the antigen recognized by the transferred T cells, e.g., the H-Y antigen TCR transgenic; pigeon cytochrome C antigen TCR transgenic; or hemagglutinin (HA) TCR transgenic. In such models, T cells expressing the TCR specific for the antigen constitutively or inducibly expressed by the recipient mice typically undergo an immediate expansion and proliferative phase, followed by a period of unresponsiveness, which is reversed when the antigen is removed and/or antigen expression is inhibited. Accordingly, if, in the presence of a CEACAM1, PD1, and/or LAP inhibitory agent, in such models if the T cells proliferate or expand, show cytokine activity, etc. significantly more than T cells in the absence of the inhibitory agent, than that agent is one that reduces T cell tolerance. Such measurements of proliferation can occur in vivo using T cells labeled with BrDU, CFSE or another intravital dye that allows tracking of proliferation prior to transferring to a recipient animal expressing the antigen, or cytokine reporter T cells, or using ex vivo methods to analyze cellular proliferation and/or cytokine production, such as thymidine proliferation assays, ELISA, cytokine bead assays, and the like.

Reduction of T cell tolerance can also be assessed by examination of tumor infiltrating lymphocytes or T lymphocytes within lymph nodes that drain from an established tumor. Such T cells exhibit features of "exhaustion" through expression of cell surface molecules such as PD1, TIM-3 or LAG-3, for example, and decreased secretion of cytokines such as interferon-γ. Accordingly, if, in the presence of a CEACAM1, PD1, and/or LAP inhibitory agent, increased quantities of T cells with 1) antigen specificity for tumor associated antigens are observed (e.g. as determined by major histocompatibility complex class I or class II tetramers which contain tumor associated peptides) and 2 that are capable of secreting high levels of interferon-y and cytolytic effector molecules such as granzyme-B, relative to that observed in the absence of the inhibitory agent, this would be evidence that T cell tolerance had been reduced. In one example, the technology described herein relates to methods for treating a chronic disease. Such methods can comprise administering a composition as described herein (i.e. a composition comprising at least two of (i) a CEACAM1 inhibitory agent, (ii) a PD1 inhibitory agent, and (iii) a LAP inhibitory agent) to a subject in need of treatment for a chronic disease. In some embodiments, a chronic disease can be selected from the group consisting of cancer; a persistent infection; and a chronic viral infection. In some embodiments, the subject in need of treatment for a chronic disease can be a subject suffering from undesirably high levels of T-cell tolerance, a subject diagnosed as suffering from undesirably high levels of T-cell tolerance, or a subject in need of a reduction or inhibition of T-cell tolerance. As used herein an "undesirably high level of T-cell tolerance" can be a level of T-cell tolerance which does not allow a subject's immune system to respond to and/or clear an infection or chronic disease. A subject in need of a reduction or inhibition of T-cell tolerance using the methods described herein includes but is not limited to a subject having cancer or a pre-cancerous condition (e.g. cytological changes indicating a shift from a normal phenotype that is not yet neoplastic but which marks the tendency to progress to a neoplastic phenotype), a subject with a chronic infection, or a subject suffering from infection by a pathogen.

In some examples of the methods described herein, the subject being administered an agent(s) for inhibiting at least two of CEACAM1, PD1, and/or LAP has a persistent infection with a bacterium, virus, fungus, or parasite. "Persistent infections" refer to those infections that, in contrast to acute infections, are not effectively cleared by the induction of a naturally occurring host immune response. During such persistent infections, the infectious agent and the immune response reach equilibrium such that the infected subject remains infectious over a long period of time without necessarily expressing symptoms. Persistent infections often involve stages of both silent and productive infection without rapidly killing or even producing excessive damage of the host cells. Persistent infections include for example, latent, chronic and slow infections. Persistent infection occurs with viruses including, but not limited to, human T-Cell leukemia viruses, Epstein-Barr virus, cytomegalovirus, herpesviruses, varicella-zoster virus, measles, papovaviruses, prions, hepatitis viruses, adenoviruses, parvoviruses and papillomaviruses.

In a "chronic infection," the infectious agent is present in the subject at all times. However, the signs and symptoms of the disease can be present or absent for an extended period of time. Non-limiting examples of chronic infection include hepatitis B (caused by hepatitis B virus (HBV)) and hepatitis C (caused by hepatitis C virus (HCV)) adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyomavirus BK, polyomavirus JC, measles virus, rubella virus, human immunodeficiency virus (HIV), human T cell leukemia virus I, and human T cell leukemia virus II. Parasitic persistent infections can arise as a result of infection by, for example, Leishmania, Toxoplasma, Trypanosoma, Plasmodium, Schistosoma, and Encephalitozoon.

In some embodiments, a chronic infection can be a latent infection. In some embodiments, a chronic infection can include periods in which the infection is a latent infection. In a "latent infection," the infectious agent (such as a virus) is seemingly inactive and dormant such that the subject does not always exhibit signs or symptoms. In a latent viral infection, the virus remains in equilibrium with the host for long periods of time before symptoms again appear; however, the actual viruses cannot typically be detected until reactivation of the disease occurs. Non-limiting examples of latent infections include infections caused by herpes simplex virus (HSV)-1 (fever blisters), HSV-2 (genital herpes), and varicella zoster virus VZV (chickenpox-shingles).

In a "slow infection," the infectious agents gradually increase in number over a very long period of time during which no significant signs or symptoms are observed. Non-limiting examples of slow infections include AIDS (caused by HIV-1 and HIV-2), lentiviruses that cause tumors in animals, and prions.

In addition, persistent infections that can be treated using the methods described herein include those infections that often arise as late complications of acute infections. For example, subacute sclerosing panencephalitis (SSPE) can occur following an acute measles infection or regressive encephalitis can occur as a result of a rubella infection.

The mechanisms by which persistent infections are maintained can involve modulation of virus and cellular gene expression and modification of the host immune response. Reactivation of a latent infection can be triggered by various stimuli, including changes in cell physiology, superinfection by another virus, and physical stress or trauma. Host immunosuppression is often associated with reactivation of a number of persistent virus infections. In some of the methods described herein, the subject being administered an agent(s) for inhibiting CEACAM1 and PD1 or CEACAM1 and LAP, for example, has an infection with a pathogen, such as a bacterium, virus, fungus, or parasite.

Examples of infectious viruses include, but are not limited to: *Retroviridae* (for example, HIV); *Picornaviridae* (for example, polio viruses, hepatitis A virus; enteroviruses, human coxsackie viruses, rhinoviruses, echoviruses); *Calciviridae* (such as strains that cause gastroenteritis); *Togaviridae* (for example, equine encephalitis viruses, rubella viruses); *Flaviridae* (for example, dengue viruses, encephalitis viruses, yellow fever viruses); *Coronaviridae* (for example, coronaviruses); *Rhabdoviridae* (for example, vesicular stomatitis viruses, rabies viruses); *Filoviridae* (for example, ebola viruses); *Paramyxoviridae* (for example, parainfluenza viruses, mumps virus, measles virus, respiratory syncytial virus); *Orthomyxoviridae* (for example, influenza viruses); *Bungaviridae* (for example, Hantaan viruses, bunga viruses, phleboviruses and Nairo viruses); *Arena viridae* (hemorrhagic fever viruses); *Reoviridae* (*e.g.,* reoviruses, orbiviurses and rotaviruses); *Birnaviridae; Hepadnaviridae* (Hepatitis B virus); *Parvoviridae* (parvoviruses); *Papovaviridae* (papilloma viruses, polyoma viruses); *Adenoviridae* (most adenoviruses); *Herpesviridae* (herpes simplex virus (HSV) 1 and HSV-2, varicella zoster virus, cytomegalovirus (CMV), herpes viruses); *Poxviridae* (variola viruses, vaccinia viruses, pox viruses); and *Iridoviridae* (such as African swine fever virus); and unclassified viruses (for example, the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (*i.e.*, Hepatitis C); Norwalk and related viruses, and astroviruses). The compositions and methods described herein are contemplated for use in treating infections with these and other viral agents.

Examples of fungal infections include but are not limited to: aspergillosis; thrush (caused by *Candida albicans*); cryptococcosis (caused by *Cryptococcus*); and histoplasmosis. Thus, examples of infectious fungi include, but are not limited to, *Cryptococcus neoformans, Histoplasma capsulatum, Coccidioides immitis, Blastomyces dermatitidis, Chlamydia trachomatis, Candida albicans.* The compositions and methods described herein are contemplated for use in treating infections with these and other fungal agents.

Examples of infectious bacteria include, but are not limited to: *Helicobacterpyloris, Borelia burgdorferi, Legionella pneumophilia, Mycobacteria* sps (such as *M. tuberculosis, M. avium, M. intracellulare, M. kansaii, M. gordonae*), *Staphylococcus aureus, Neisseria gonorrhoeae, Neisseria meningitidis, Listeria monocytogenes, Streptococcus pyogenes* (Group A Streptococcus), *Streptococcus agalactiae* (Group B Streptococcus), *Streptococcus* (viridans group), *Streptococcus faecalis, Streptococcus bovis, Streptococcus* (anaerobic sps.), *Streptococcus pneumoniae,* pathogenic *Campylobacter* sp., *Enterococcus* sp., *Haemophilus influenzae, Bacillus anthracis, corynebacterium diphtheriae, corynebacterium* sp., *Erysipelothrix rhusiopathiae, Clostridium perfringens, Clostridium tetani, Enterobacter aerogenes, Klebsiella pneumoniae, Pasturella multocida, Bacteroides* sp., *Fusobacterium nucleatum, Streptobacillus moniliformis, Treponema pallidium, Treponema pertenue, Leptospira,* and *Actinomyces israelli.* The compositions and methods described herein are contemplated for use in treating infections with these and other bacterial agents. Other infectious organisms (such as protists) include: *Plasmodium falciparum* and *Toxoplasma gondii.* The compositions and methods described herein are contemplated for use in treating infections with these and other agents.

In some examples, the methods further comprise administering an effective amount of a viral, bacterial, fungal, or parasitic antigen in conjunction with an agent(s) for inhibiting PD1, LAP and/or CEACAM1. Non-limiting examples of suitable viral antigens include: influenza HA, NA, M, NP and NS antigens; HIV p24, pol, gp41 and gpl20; Metapneumovirus (hMNV) F and G proteins; Hepatitis C virus (HCV) E1, E2 and core proteins; Dengue virus (DEN1-4) E1, E2 and core proteins; Human Papilloma Virus L1 protein; Epstein Barr Virus gp220/350 and EBNA-3A peptide; Cytomegalovirus (CMV) gB glycoprotein, gH glycoprotein, pp65, IE1 (exon 4) and pp 150; Varicella Zoster virus (VZV) IE62 peptide and glycoprotein E epitopes; Herpes Simplex Virus Glycoprotein D epitopes, among many others. The antigenic polypeptides can correspond to polypeptides of naturally occurring animal or human viral isolates, or can be engineered to incorporate one or more amino acid substitutions as compared to a natural (pathogenic or non-pathogenic) isolate.

In other examples of the methods described herein, the subject being administered an agent for inhibiting PD1, LAP and/or CEACAM1 has a cancer or tumor. Accordingly, described herein, are methods of treating a subject having a cancer or tumor comprising administering an effective amount of an agent(s) for inhibiting PD1, LAP and/or CEACAM1.

A "cancer" or "tumor" as used herein refers to an uncontrolled growth of cells which interferes with the normal functioning of the bodily organs and systems. A subject that has a cancer or a tumor is a subject having objectively measurable cancer cells present in the subject's body. Included in this definition are benign and malignant cancers, as well as dormant tumors or micrometastases. Cancers which migrate from their original location and seed vital organs can eventually lead to the death of the subject through the functional deterioration of the affected organs. Hemopoietic cancers, such as leukemia, are able to out-compete the normal hemopoietic compartments in a subject, thereby leading to hemopoietic failure (in the form of anemia, thrombocytopenia and neutropenia) ultimately causing death.

By "metastasis" is meant the spread of cancer from its primary site to other places in the body. Cancer cells can break away from a primary tumor, penetrate into lymphatic and blood vessels, circulate through the bloodstream, and grow in a distant focus (metastasize) in normal tissues elsewhere in the body. Metastasis can be local or distant. Metastasis is a sequential process, contingent on tumor cells breaking off from the primary tumor, traveling through the bloodstream, and stopping at a distant site. At the new site, the cells establish a blood supply and can grow to form a life-threatening mass. Both stimulatory and inhibitory molecular pathways within the tumor cell regulate this behavior, and interactions between the tumor cell and host cells in the distant site are also significant. Metastases are most often detected through the sole or combined use of magnetic resonance imaging (MRI) scans, computed tomography (CT) scans, blood and platelet counts, liver function studies, chest X-rays and bone scans in addition to the monitoring of specific symptoms.

Examples of cancer include but are not limited to, carcinoma, lymphoma, blastoma, sarcoma, and leukemia. More particular examples of such cancers include, but are not limited to, basal cell carcinoma, biliary tract cancer; bladder cancer; bone cancer; brain and CNS cancer; breast cancer; cancer of the peritoneum; cervical cancer; choriocarcinoma; colon and rectum cancer; connective tissue cancer; cancer of the digestive system; endometrial cancer; esophageal cancer; eye cancer; cancer of the head and neck; gastric cancer (including gastrointestinal cancer); glioblastoma; hepatic carcinoma; hepatoma; intra-epithelial neoplasm; kidney or renal cancer; larynx cancer; leukemia; liver cancer; lung cancer (e.g., small-cell lung cancer, non-small cell lung cancer, adenocarcinoma of the lung, and squamous carcinoma of the lung); lymphoma including Hodgkin's and non-Hodgkin's lymphoma; melanoma; myeloma; neuroblastoma; oral cavity cancer (*e.g.,* lip, tongue, mouth, and pharynx); ovarian cancer; pancreatic cancer; prostate cancer; retinoblastoma; rhabdomyosarcoma; rectal cancer; cancer of the respiratory system; salivary gland carcinoma; sarcoma; skin cancer; squamous cell cancer; stomach cancer; testicular cancer; thyroid cancer; uterine or endometrial cancer; cancer of the urinary system; vulval cancer; as well as other carcinomas and sarcomas; as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

In some examples described herein, the methods further comprise administering a tumor or cancer antigen to a subject being administered an agent(s) for inhibiting PD1, LAP and/or CEACAM1 described herein. A number of tumor antigens have been identified that are associated with specific cancers. As used herein, the terms "tumor antigen" and "cancer antigen" are used interchangeably to refer to antigens which are differentially expressed by cancer cells and can thereby be exploited in order to target cancer cells. Cancer antigens are antigens which can potentially stimulate apparently tumor-specific immune responses. Some of these antigens are encoded, although not necessarily expressed, by normal cells. These antigens can be characterized as those which are normally silent *(i.e.,* not expressed) in normal cells, those that are expressed only at certain stages of differentiation and those that are temporally expressed such as embryonic and fetal antigens. Other cancer antigens are encoded by mutant cellular genes, such as oncogenes (*e.g*., activated ras oncogene), suppressor genes (*e.g*., mutant p53), fusion proteins resulting from internal deletions or chromosomal translocations. Still other cancer antigens can be encoded by viral genes such as those carried on RNA and DNA tumor viruses. Many tumor antigens have been defined in terms of multiple solid tumors: MAGE 1, 2, & 3, defined by immunity; MART-1/Melan-A, gp100, carcinoembryonic antigen (CEA), HER-2, mucins *(i.e.,* MUC-1), prostate-specific antigen (PSA), and prostatic acid phosphatase (PAP). In addition, viral proteins such as proteins of hepatitis B (HBV), Epstein-Barr (EBV), and human papilloma (HPV) have been shown to be important in the development of hepatocellular carcinoma, lymphoma, and cervical cancer, respectively. However, due to the immunosuppression of patients diagnosed with cancer, the immune systems of these patients often fail to respond to the tumor antigens.

In some of the methods described herein, the methods further comprise administering a chemotherapeutic agent to the subject being administered an agent(s) for inhibiting PD1, LAP and/or CEACAM1. Non-limiting examples of chemotherapeutic agents can include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (*e.g*., calicheamicin, especially calicheamicin gamma1I and calicheamicin omegaI1 (see, *e.g*., Agnew, Chem. Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® paclitaxel (Bristol-Myers Squibb Oncology, Princeton, N.J.), ABRAXANE® Cremophor-free, albumin-engineered nanoparticle formulation of paclitaxel (American Pharmaceutical Partners, Schaumberg, Ill.), and TAXOTERE® doxetaxel (Rhone-Poulenc Rorer, Antony, France); chloranbucil; GEMZAR® gemcitabine; 6-thioguanine; mercaptopurine; methotrexate; platinum analogs such as *cis*platin, oxaliplatin and carboplatin; vinblastine; platinum; etoposide (VP-16); ifosfamide; mitoxantrone; vincristine; NAVELBINE; vinorelbine; novantrone; teniposide; edatrexate; daunomycin; aminopterin; xeloda; ibandronate; irinotecan (Camptosar, CPT-11) (including the treatment regimen of irinotecan with 5-FU and leucovorin); topoisomerase inhibitor RFS 2000; difluoromethylornithine (DMFO); retinoids such as retinoic acid; capecitabine; combretastatin; leucovorin (LV); oxaliplatin, including the oxaliplatin treatment regimen (FOLFOX); lapatinib (TYKERB); inhibitors of PKC-alpha, Raf, H-Ras, EGFR (*e.g.*, erlotinib (TARCEVA®)) and VEGF-A that reduce cell proliferation, and pharmaceutically acceptable salts, acids or derivatives of any of the above. In addition, the methods of treatment can further include the use of radiation.

As used herein, the terms "treat," "treatment," "treating," or "amelioration" refer to therapeutic treatments, wherein the object is to reverse, alleviate, ameliorate, inhibit, slow down or stop the progression or severity of a condition associated with, a disease or disorder. The term "treating" includes reducing or alleviating at least one adverse effect or symptom of a condition, disease or disorder, such as an autoimmune disease, a chronic infection or a cancer. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of at least slowing of progress or worsening of symptoms that would be expected in absence of treatment. Beneficial or desired clinical results include, but are not limited to, alleviation of one or more symptom(s), diminishment of extent of disease, stabilized *(i.e.,* not worsening) state of disease, delay or slowing of disease progression, amelioration or palliation of the disease state, and remission (whether partial or total), whether detectable or undetectable. The term "treatment" of a disease also includes providing relief from the symptoms or side-effects of the disease (including palliative treatment).

The term "effective amount" as used herein refers to the amount of an agent(s) for inhibiting PD1, LAP and/or CEACAM1 needed to alleviate at least one or more symptom of the disease or disorder, and relates to a sufficient amount of pharmacological composition to provide the desired effect, *i.e.,* inhibit T cell tolerance, for example. The term "therapeutically effective amount" therefore refers to an amount of an agent(s) for inhibiting PD1, LAP and/or CEACAM1 using the methods as disclosed herein, that is sufficient to effect a particular effect when administered to a typical subject. An effective amount as used herein would also include an amount sufficient to delay the development of a symptom of the disease, alter the course of a symptom of disease (for example but not limited to, slow the progression of a symptom of the disease), or reverse a symptom of disease. Thus, it is not possible to specify the exact "effective amount". However, for any given case, an appropriate "effective amount" can be determined by one of ordinary skill in the art using only routine experimentation.

Effective amounts, toxicity, and therapeutic efficacy can be determined by standard pharmaceutical procedures in cell cultures or experimental animals, *e.g.,* for determining the LD50 (the dose lethal to 50% of the population) and the ED50 (the dose therapeutically effective in 50% of the population). The dosage can vary depending upon the dosage form employed and the route of administration utilized. The dose ratio between toxic and therapeutic effects is the therapeutic index and can be expressed as the ratio LD50/ED50. Compositions and methods that exhibit large therapeutic indices are preferred. A therapeutically effective dose can be estimated initially from cell culture assays. Also, a dose can be formulated in animal models to achieve a circulating plasma concentration range that includes the IC50 (*i.e.,* the concentration of the agent(s) for inhibiting PD1, LAP and/or CEACAM1), which achieves a half-maximal inhibition of symptoms) as determined in cell culture, or in an appropriate animal model. Levels in plasma can be measured, for example, by high performance liquid chromatography. The effects of any particular dosage can be monitored by a suitable bioassay. The dosage can be determined by a physician and adjusted, as necessary, to suit observed effects of the treatment.

The agents useful according to the compositions and methods described herein, including antibodies and other polypeptides, are isolated agents, meaning that the agents are substantially pure and are essentially free of other substances with which they may be found in nature or *in vivo* systems to an extent practical and appropriate for their intended use. In particular, the agents are sufficiently pure and are sufficiently free from other biological constituents of their host cells so as to be useful in, for example, producing pharmaceutical preparations. Because an isolated agent may be admixed with a pharmaceutically acceptable carrier in a pharmaceutical preparation, the agents may comprise only a small percentage by weight of the preparation.

The agents described herein for inhibiting PD1, LAP and/or CEACAM1 can be administered to a subject in need thereof by any appropriate route which results in an effective treatment in the subject. As used herein, the terms "administering," and "introducing" are used interchangeably and refer to the placement of a polypeptide agent into a subject by a method or route which results in at least partial localization of such agents at a desired site, such as a site of inflammation, such that a desired effect(s) is produced.

In some examples, the agents described herein for inhibiting PD1, LAP and/or CEACAM1 are administered to a subject by any mode of administration that delivers the agent systemically or to a desired surface or target, and can include, but is not limited to, injection, infusion, instillation, and inhalation administration. To the extent that polypeptide agents can be protected from inactivation in the gut, oral administration forms are also contemplated. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. In some examples, the agents for inhibiting PD1, LAP and/or CEACAM1 for use in the methods described herein are administered by intravenous infusion or injection.

The phrases "parenteral administration" and "administered parenterally" as used herein, refer to modes of administration other than enteral and topical administration, usually by injection. The phrases "systemic administration," "administered systemically", "peripheral administration" and "administered peripherally" as used herein refer to the administration of an agent for inhibiting PD1, LAP and/or CEACAM1 other than directly into a target site, tissue, or organ, such as a tumor site, such that it enters the subject's circulatory system and, thus, is subject to systemic metabolism and other like processes.

For the clinical use of the methods described herein, administration of an agent for inhibiting PD1, LAP and/or CEACAM1 can include formulation into pharmaceutical compositions or pharmaceutical formulations for parenteral administration, *e.g.,* intravenous; mucosal, *e.g.,* intranasal; ocular, or other mode of administration. In some examples, an agent for inhibiting PD1, LAP and/or CEACAM1 described herein can be administered along with any pharmaceutically acceptable carrier compound, material, or composition which results in an effective treatment in the subject. Thus, a pharmaceutical formulation for use in the methods described herein can contain an agent for inhibiting PD1, LAP and/or CEACAM1 as described herein in combination with one or more pharmaceutically acceptable ingredients.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" as used herein means a pharmaceutically acceptable material, composition or vehicle, such as a liquid or solid filler, diluent, excipient, solvent, media, encapsulating material, manufacturing aid (*e.g.*, lubricant, talc magnesium, calcium or zinc stearate, or steric acid), or solvent encapsulating material, involved in maintaining the stability, solubility, or activity of, an agent for inhibiting PD1, LAP and/or CEACAM1. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) excipients, such as cocoa butter and suppository waxes; (8) oils, such as peanut oil, cottonseed oil, safflower oil, sesame oil, olive oil, corn oil and soybean oil; (9) glycols, such as propylene glycol; (10) polyols, such as glycerin, sorbitol, mannitol and polyethylene glycol (PEG); (11) esters, such as ethyl oleate and ethyl laurate; (12) agar; (13) buffering agents, such as magnesium hydroxide and aluminum hydroxide; (14) alginic acid; (15) pyrogen-free water; (16) isotonic saline; (17) Ringer's solution; (19) pH buffered solutions; (20) polyesters, polycarbonates and/or polyanhydrides; (21) bulking agents, such as polypeptides and amino acids (22) serum components, such as serum albumin, HDL and LDL; (23) C2-C12 alcohols, such as ethanol; and (24) other non-toxic compatible substances employed in pharmaceutical formulations. Release agents, coating agents, preservatives, and antioxidants can also be present in the formulation. The terms such as "excipient", "carrier", "pharmaceutically acceptable carrier" or the like are used interchangeably herein.

The agents for inhibiting PD1, LAP and/or CEACAM1 described herein can be specially formulated for administration of the compound to a subject in solid, liquid or gel form, including those adapted for the following: (1) parenteral administration, for example, by subcutaneous, intramuscular, intravenous or epidural injection as, for example, a sterile solution or suspension, or sustained-release formulation; (2) topical application, for example, as a cream, ointment, or a controlled-release patch or spray applied to the skin; (3) intravaginally or intrarectally, for example, as a pessary, cream or foam; (4) ocularly; (5) transdermally; (6) transmucosally; or (79) nasally. Additionally, a bispecific or multispecific polypeptide agent can be implanted into a patient or injected using a drug delivery system. See, for example, Urquhart, et al., Ann. Rev. Pharmacol. Toxicol. 24: 199-236 (1984); Lewis, ed. "Controlled Release of Pesticides and Pharmaceuticals" (Plenum Press, New York, 1981); U.S. Pat. No. 3,773,919; and U.S. Pat. No. 35 3,270,960.

Further embodiments of the formulations and modes of administration of an agent for inhibiting PD1, LAP and/or CEACAM1 that can be used in the methods described herein are illustrated below.

Parenteral Dosage Forms. Parenteral dosage forms of an agent for inhibiting PD1, LAP and/or CEACAM1 can also be administered to a subject by various routes, including, but not limited to, subcutaneous, intravenous (including bolus injection), intramuscular, and intraarterial. Since administration of parenteral dosage forms typically bypasses the patient's natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, controlled-release parenteral dosage forms, and emulsions.

Suitable vehicles that can be used to provide parenteral dosage forms of the disclosure are well known to those skilled in the art. Examples include, without limitation: sterile water; water for injection USP; saline solution; glucose solution; aqueous vehicles such as but not limited to, sodium chloride injection, Ringer's injection, dextrose Injection, dextrose and sodium chloride injection, and lactated Ringer's injection; water-miscible vehicles such as, but not limited to, ethyl alcohol, polyethylene glycol, and propylene glycol; and non-aqueous vehicles such as, but not limited to, corn oil, cottonseed oil, peanut oil, sesame oil, ethyl oleate, isopropyl myristate, and benzyl benzoate.

Aerosol formulations. An agent for inhibiting PD1, LAP and/or CEACAM1 can be packaged in a pressurized aerosol container together with suitable propellants, for example, hydrocarbon propellants like propane, butane, or isobutane with conventional adjuvants. An agent for inhibiting PD1, LAP and/or CEACAM1 can also be administered in a non-pressurized form such as in a nebulizer or atomizer. An agent for inhibiting PD1, LAP and/or CEACAM1 can also be administered directly to the airways in the form of a dry powder, for example, by use of an inhaler.

Suitable powder compositions include, by way of illustration, powdered preparations of an agent for inhibiting PD1, LAP and/or CEACAM1 thoroughly intermixed with lactose, or other inert powders acceptable for intrabronchial administration. The powder compositions can be administered via an aerosol dispenser or encased in a breakable capsule which can be inserted by the subject into a device that punctures the capsule and blows the powder out in a steady stream suitable for inhalation. The compositions can include propellants, surfactants, and co-solvents and can be filled into conventional aerosol containers that are closed by a suitable metering valve.

Aerosols for the delivery to the respiratory tract are known in the art. See for example, Adjei, A. and Garren, J. Pharm. Res., 1: 565-569 (1990); Zanen, P. and Lamm, J.-W. J. Int. J. Pharm., 114: 111-115 (1995); Gonda, I. "Aerosols for delivery of therapeutic an diagnostic agents to the respiratory tract," in Critical Reviews in Therapeutic Drug Carrier Systems, 6:273-313 (1990); Anderson et al., Am. Rev. Respir. Dis., 140: 1317-1324 (1989)) and have potential for the systemic delivery of peptides and proteins as well (Patton and Platz, Advanced Drug Delivery Reviews, 8:179-196 (1992)); Timsina et. al., Int. J. Pharm., 101: 1-13 (1995); and Tansey, I. P., Spray Technol. Market, 4:26-29 (1994); French, D. L., Edwards, D. A. and Niven, R. W., Aerosol Sci., 27: 769-783 (1996); Visser, J., Powder Technology 58: 1-10 (1989)); Rudt, S. and R. H. Muller, J. Controlled Release, 22: 263-272 (1992); Tabata, Y, and Y. Ikada, Biomed. Mater. Res., 22: 837-858 (1988); Wall, D. A., Drug Delivery, 2: 10 1-20 1995); Patton, J. and Platz, R., Adv. Drug Del. Rev., 8: 179-196 (1992); Bryon, P., Adv. Drug. Del. Rev., 5: 107-132 (1990); Patton, J. S., et al., Controlled Release, 28: 15 79-85 (1994); Damms, B. and Bains, W., Nature Biotechnology (1996); Niven, R. W., et al., Pharm. Res., 12(9); 1343-1349 (1995); and Kobayashi, S., et al., Pharm. Res., 13(1): 80-83 (1996).

The formulations of the agents for inhibiting PD1, LAP and/or CEACAM1 described herein further encompass anhydrous pharmaceutical compositions and dosage forms comprising the disclosed compounds as active ingredients, since water can facilitate the degradation of some compounds. For example, the addition of water (*e.g.,* 5%) is widely accepted in the pharmaceutical arts as a means of simulating long-term storage in order to determine characteristics such as shelf life or the stability of formulations over time. See, *e.g.,* Jens T. Carstensen, Drug Stability: Principles & Practice, 379-80 (2nd ed., Marcel Dekker, NY, N.Y.: 1995). Anhydrous pharmaceutical compositions and dosage forms of the disclosure can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected. Anhydrous compositions are preferably packaged using materials known to prevent exposure to water such that they can be included in suitable formulary kits. Examples of suitable packaging include, but are not limited to, hermetically sealed foils, plastics, unit dose containers (e.g., vials) with or without desiccants, blister packs, and strip packs.

Controlled and Delayed Release Dosage Forms. In some examples of the methods described herein, an agent for inhibiting PD1, LAP and/or CEACAM1 can be administered to a subject by controlled- or delayed-release means. Ideally, the use of an optimally designed controlled-release preparation in medical treatment is characterized by a minimum of drug substance being employed to address or control the condition in a minimum amount of time. Advantages of controlled-release formulations include: 1) extended activity of the drug; 2) reduced dosage frequency; 3) increased patient compliance; 4) usage of less total drug; 5) reduction in local or systemic side effects; 6) minimization of drug accumulation; 7) reduction in blood level fluctuations; 8) improvement in efficacy of treatment; 9) reduction of potentiation or loss of drug activity; and 10) improvement in speed of control of diseases or conditions. (Kim, Cherng-ju, Controlled Release Dosage Form Design, 2 (Technomic Publishing, Lancaster, Pa.: 2000)). Controlled-release formulations can be used to control a compound of formula (I)'s onset of action, duration of action, plasma levels within the therapeutic window, and peak blood levels. In particular, controlled- or extended-release dosage forms or formulations can be used to ensure that the maximum effectiveness of a compound of formula (I) is achieved while minimizing potential adverse effects and safety concerns, which can occur both from under-dosing a drug (*i.e.,* going below the minimum therapeutic levels) as well as exceeding the toxicity level for the drug.

A variety of known controlled- or extended-release dosage forms, formulations, and devices can be adapted for use with the agents for inhibiting PD1, LAP and/or CEACAM1 described herein. Examples include, but are not limited to, those described in U.S. Pat. Nos.: 3,845,770; 3,916,899; 3,536,809; 3,598,123; 4,008,719; 5674,533; 5,059,595; 5,591 ,767; 5,120,548; 5,073,543; 5,639,476; 5,354,556; 5,733,566; and 6,365,185 B1. These dosage forms can be used to provide slow or controlled-release of one or more active ingredients using, for example, hydroxypropylmethyl cellulose, other polymer matrices, gels, permeable membranes, osmotic systems (such as OROS® (Alza Corporation, Mountain View, Calif. USA)), multilayer coatings, microparticles, liposomes, or microspheres or a combination thereof to provide the desired release profile in varying proportions. Additionally, ion exchange materials can be used to prepare immobilized, adsorbed salt forms of the disclosed compounds and thus effect controlled delivery of the drug. Examples of specific anion exchangers include, but are not limited to, Duolite® A568 and Duolite® AP143 (Rohm&Haas, Spring House, Pa. USA).

In some examples, an agent for inhibiting PD1, LAP and/or CEACAM1 for use in the methods described herein is administered to a subject by sustained release or in pulses. Pulse therapy is not a form of discontinuous administration of the same amount of a composition over time, but comprises administration of the same dose of the composition at a reduced frequency or administration of reduced doses. Sustained release or pulse administrations are particularly preferred when the disorder occurs continuously in the subject, for example where the subject has continuous or chronic symptoms of a viral infection. Each pulse dose can be reduced and the total amount of the agent for inhibiting PD1, LAP and/or CEACAM1 administered over the course of treatment to the patient is minimized.

The interval between pulses, when necessary, can be determined by one of ordinary skill in the art. Often, the interval between pulses can be calculated by administering another dose of the composition when the composition or the active component of the composition is no longer detectable in the subject prior to delivery of the next pulse. Intervals can also be calculated from the *in vivo* half-life of the composition. Intervals can be calculated as greater than the *in vivo* half-life, or 2, 3, 4, 5 and even 10 times greater than the composition half-life. Various methods and apparatus for pulsing compositions by infusion or other forms of delivery to the patient are disclosed in U.S. Pat. Nos. 4,747,825; 4,723,958; 4,948,592; 4,965,251 and 5,403,590.

It should be understood that this invention is not limited to the particular methodology, protocols, and reagents, etc., described herein, and as such may vary. The terminology used herein is for the purpose of describing particular embodiments only, and is not intended to limit the scope of the present invention, which is defined solely by the claims.

Unless defined otherwise, all technical and scientific terms used herein have the same meaning as those commonly understood to one of ordinary skill in the art to which this invention pertains. Although any known methods, devices, and materials may be used in the practice or testing of the invention, the methods, devices, and materials in this regard are described herein.

### EXAMPLES

### EXAMPLE 1: Tumor infiltrating lymphocytes co-express CEACAM1 together with other accessory molecules that are associated with T cell tolerance and exhaustion.

Expression of inhibitory molecules on lymphocytes plays a major role in limiting immune defenses against cancers. Inhibition of these types of molecules is increasingly being shown to permit natural cancer defenses in association with the eradication of tumors. Molecules such as TIM3, CTLA4, LAP, PD1 and more recently CEACAM1 have been demonstrated to be increased on tumor infiltrating lymphocytes and, in some cases, to be associated with poor prognosis. In turn, blockade of these pathways has been demonstrated to be associated with increased cancer responses in mouse models and more recently in humans at least with respect to studies with CTLA4 and PD1. However, blockade of these pathways individually is associated with only limited benefit in humans.

Demonstrated herein is the upregulation of CEACAM1 and PD1 as well as CEACAM1 and LAP together on tumor-infiltrating lymphocytes in models of colon cancer; a previously unreported observation. Accordingly, blockade of both CEACAM1 and PD1 or CEACAM1 and LAP together is more effective than either one alone.

Described herein are results indicating that CEACAM1 and PD1 positive (double-positive) T cells are increased in tumor-infiltrating lymphocytes relative to that which is observed on lymphocytes under physiologic circumstances and increased association between CEACAM1 and LAP expression on T cells in vivo. Inhibition of two or more of CEACAM1, PD1, and/or LAP together in vivo is a superior therapeutic approach to inhibition of a single factor alone.

The expression of a tolerance associated phenotype through combinations of cell surface molecules such as PD-1, TIM-3, LAP and CEACAM1 was examined using the AOM-DSS model in wild-type mice (see, e.g. De Robertis et al. J Carcinog 2011 10:9. Briefly, a dose of azoxymethane (AOM) with subsequent doses of the inflammatory agent dextran sodium sulphate (DSS) (Figure 1) increases the onset of colorectal cancer, and accordingly, T cell tolerance of the associated tumor infiltrating lymphocytes in approximation to the colorectal cancer cells.

Subpopulations of T cells obtained from these mice at Day 44 of the AOM-DSS protocol were subjected to FACS analysis. Tumor infiltrating lymphocytes, tumor intra-epithelial lymphocytes, adjacent tumor infiltrating lymphocytes, adjacent tumor intraepithelial lymphocytes, and cells from the mesenteric lymph nodes were examined for cell surface expression of CD4, CD8, PD1, CEACAM1, and TIM3 (Figures 2-4). The results of these analyses are summarized in Figures 5A-5B, demonstrating that tumor infiltrating lymphocytes, adjacent tumor infiltrating lymphocytes and adjacent tumor intraepithelial lymphocytes contain significant subpopulations of CEACAM1⁺ PD1⁺ cells.

Figures 5A-5B depict graphs demonstrating CEACAM1 and PD1 are co-expressed on the cell surface of chronically activated CD4⁺ (Figure 5A) and CD8⁺ (Figure 5B) tumor infiltrating T lymphocytes.

### EXAMPLE 2: T lymphocytes with CEACAM1 expression are characterized by co-expression of accessory molecules associated with T cell tolerance and a regulatory phenotype.

**CEACAM1 is preferentially expressed on specific subsets of circulating and tissue-resident CD4+ T cells.** Attention was focused on defining the accessory molecules expressed by the small subset of CEACAM1+ T cells detected under physiologic conditions in blood (data not shown) and mesenteric lymph node (MLN) (see Figure 6A) and which were increased in frequency in PP and LP of WT mice (Figure 9A and 9B). It was observed that CD4+CEACAM1+ T cells from the blood, MLN, PP and LP expressed higher amounts of both CD44 and CD62L than did CD4+CEACAM1- T cells (Figure 6A and 9A and 9B). This is consistent with CEACAM1 being associated with effector and/or central memory T cells (Boulton et al., 2002). Additionally, it was noted that CD4+CEACAM1+ T cells from each of these tissue compartments expressed more α4β7-integrin than their CD4+CEACAM1-counterparts, suggesting that CEACAM1 expression on T cells, even those within the peripheral immune system, may be associated with enhanced potential to home to intestinal tissues. Interestingly, it was also noted that CEACAM1 positivity was associated with preferential expression of PD1, LAP (TGFβ Latency Associated Peptide) and B220, all of which have previously been associated with T cell subsets possessing regulatory or accessory potential in the production of IgA (Fagarasan et al., 2001; Good-Jacobson et al., 2010; Ochi et al., 2006; Oida et al., 2003).

These observations led to investigation of specific cell subsets on which the identified molecules are known to be enriched. In particular, since the data described herein showed CEACAM1 expression to be strongly co-segregating with surface LAP on CD4+ T cells from naive WT animals (Figures 6A and Figure 9A and 9Bµ), CD4+CD25-LAP+ T cells, a subset of Treg that are concentrated in intestinal tissues although functioning throughout the body and provide TGF-β dependent regulatory function and, potentially, support for secretory IgA immunity were carefully examined (Niemir et al., 1995; Ochi et al., 2006; Oida et al., 2003). Whereas on average 43.6 ± 10.9 % of CD4+CD25-LAP+ T cells within MLN co-expressed CEACAM1, only 2.07 ± 0.8 % of CD4+CD25-LAP- cells were also CEACAM1+ under steady-state conditions in WT mice (Figure 6B). This was in stark contrast to the CEACAM1 expression in another regulatory T cell subset wherein similar low levels of CEACAM1 were observed on CD4+Foxp3+ and CD4+Foxp3- Treg cells from the MLN (Figure 6B). The relative expression levels of the CEACAM1-L and -S isoforms in CD4+CEACAM1-LAP-, CD4+CEACAM1+LAP-, or CD4+CEACAM1+LAP+ cells flow-cytometrically sorted from pooled spleen and MLN of WT mice was examined using the real-time PCR assay described below (Figure 8). Consistent with the findings in the total population of peripheral CD4+ T cells, the ratio of L:S isoforms was 2.78 ± 0.07 in CD4+CEACAM1+LAP- cells, indicating a predominance of the L isoform in this group of T cells (Figure 6C). In contrast, the L:S ratio was decreased to 1.16± 0.27 (P < 0.05) in CD4+CEACAM1+LAP+ cells indicating an enrichment of the CEACAM1-S isoform in this regulatory cell subset (Figure 6C). No differences were observed in the L:S ratio between CD4+CEACAM1+CD25- cells (1.52 ± 0.29) and CD4+CEACAM1+CD25+ cells (1.52 ± 0.11) isolated from the spleen and LN (data not shown). The relative expression of the L and S isoforms does not differ between CD4+CEACAM1+CD25- and CD4+CEACAM1+CD25+ regulatory T cells with the L isoform predominating in both populations. The L:S ratio was 1.52 for CD4+CEACAM1+CD25- cells and 1.52 for CD4+CEACAM1+CD25+ cells. Cells were isolated by flow cytometric sorting from pooled spleen and MLN. These data demonstrate the existence of a close association between the expression of CEACAM1 and LAP.

### EXAMPLE 3: CEACAM1-long and CEACAM1-short isoforms are regulated by tolerogenic signals provided by anti-CD3 stimulation.

Carcinoembryonic antigen cell adhesion molecule 1 (CEACAM1), a member of the carcinoembryonic antigen family, is engaged in intercellular binding interactions that affect signal transduction by a number of cell surface receptors. CEACAM1 is constitutively expressed by a variety of cell types including those of endothelial, epithelial and hematopoietic origin such as B cells, polymorphonuclear leukocytes, monocytes, and dendritic cells. In addition, CEACAM1 is expressed at minimal levels in circulating natural killer (NK) and T cells but is upregulated in these cell types following a variety of different forms of activation (Azuz-Lieberman et al., 2005; Boulton et al., 2002; Coutelier et al., 1994; Nakajima et al., 2002; Singer et al., 2002). On the cell surface, CEACAM1 can associate with and modify the function of numerous signaling receptors that are dictated by the CEACAM1 isoforms expressed. The individual CEACAM1 isoforms are generated by alternative splicing and consist of transmembrane proteins that possess a characteristic membrane distal, IgV-like domain (the N-domain) which functions in homophilic binding but differs with respect to the number of membrane proximal extracellular IgC2-like domains and the length of their cytoplasmic (cyt) tail (Gray-Owen et al., 2006). Specifically, the 11 human and 4 mouse CEACAM1 splice variants encode either a short (S) cyt tail about which little is known or a long (L) cyt tail containing immune receptor tyrosine-based inhibitory motifs (ITIM). In the latter case, consistent with the presence of ITIM motifs in their cyt tail, the CEACAM1-L isoforms of mice and humans have been linked to inhibition of many different signaling receptors (Chen et al., 2001; Chen et al., 2008; Kammerer et al., 1998; Pan et al., 2010). In each example, the inhibitory function of CEACAM1-L isoforms is triggered by the phosphorylation of the ITIM tyrosine residues by receptor tyrosine kinases or Src-related kinases, resulting in recruitment of the Src homology 2 (SH2) domain-containing protein-tyrosine phosphatases (SHP)-1 or -2 (Huber et al., 1999; Nagaishi et al., 2006). Thus, CEACAM1-L-mediated recruitment of these phosphatases results in the dephosphorylation of critical tyrosines contained within activating motifs associated with a variety of cell surface receptors such as the B cell receptor, epidermal growth factor receptor and T cell receptor (TCR)/CD3 complex (Abou-Rjaily et al., 2004; Boulton et al., 2002; Chen et al., 2008; Lobo et al., 2009). As such, CEACAM1-L isoforms are functionally inhibitory and are the major isoforms described in mouse and human lymphocytes including NK cells, B cells and T cells.

CEACAM1-L and CEACAM1-S isoforms are expressed at varying ratios in different cell types with recent evidence showing that such expression is under the influence of cytokines (e.g. interferon-y), transcription factors (e.g. IRF-1) and splicing regulators (e.g. members of the heterogeneous nuclear ribonucleoprotein family) (Chen et al., 1996; Dery et al. 2011; Gencheva et al., 2010). Although not directly demonstrated, this is believed to be the case for T lymphocytes as well. It has been shown that while mouse and human T cells predominantly express CEACAM1-L isoforms, there is evidence that CEACAM1-S isoforms can be expressed (Donda et al., 2000; Singer et al., 2002). In vitro transfection studies in T cells suggest that CEACAM1 is a 'tunable' receptor system given that CEACAM1-S isoforms may possess weak co-stimulatory function that is capable of ameliorating the inhibitory signals provided by CEACAM1-L isoforms in the context of TCR/CD3 complex signaling when expressed together or novel signals (Chen et al., 2004a; Chen et al., 2008; Lee et al., 2008). However, there is no information available on CEACAM1 isoform regulation in T cells in vivo and the physiologic function(s) that this might confer.

By establishing a novel PCR method for quantifying mouse CEACAM1-S and CEACAM1-L variants, it was found that T cells within the intestines, relative to peripheral tissues such as the spleen, not only exhibit increased overall CEACAM1 expression but also uniquely possess a relative overabundance of CEACAM1-S relative to CEACAM1-L isoforms. Moreover, in this environment, it is shown herein that CEACAM1-S isoforms have special physiological functions which at a cellular level enhance T cell activation independently of CEACAM1-L isoforms and are preferentially expressed in cells performing common mucosal immune functions including T cell regulation and the induction of secretory immunity. Specifically, it is demonstrated herein that CEACAM1-S acts as an individual signaling unit which promotes the induction of CD4+LAP+ T regulatory (Treg) cells (Figure 7A-7D), CD4+PD1+CXCR5+ follicular helper T cells (T_{FH}) (Figure 13A-13C), and the development of IgA committed B cells within Peyer's patches (PP) (data not shown). Consistent with this, T cell-associated CEACAM1-S isoforms lead to enhanced mucosal IgA secretion which influences both the composition of commensal microbiota and host defense against exposure to pathogens such as Listeria monocytogenes (data not shown). In the absence of CEACAM1 expression, these barrier-protective functions of IgA are diminished. Together, these studies provide tissue-specific evidence for CEACAM1 isoform regulation and function.

The relative expression of CEACAM1 isoforms was assessed by first establishing a novel, quantitative assay for analyzing expression of mouse CEACAM1-L and -S variants as currently available antibodies are not able to distinguish between these two general classes of CEACAM1 in a quantitative fashion. Two pairs of real-time PCR primers were designed to specifically quantify these in mouse T cells. The specificity and efficiency of these primers was verified using mouse (m) CEACAM1-4L (4L) and CEACAM1-4S (4S) plasmids (Figure 8). Quantitative (q)PCR analysis of mouse primary CD3+ T cells isolated from spleen and lymph nodes (LN) demonstrated that CEACAM1-L isoforms predominated in resting peripheral T cells with increased expression upon anti-CD3 stimulation as previously described (Boulton et al., 2002; Nakajima et al., 2002) (data not shown). In addition, although CEACAM1-S isoforms were detected, CEACAM1-L isoforms predominated in peripheral T cells such that the CEACAM1-L:CEACAM1-S (L:S) ratio was 1.95 ± 0.34, consistent with previous studies (Singer et al., 2002). Having validated this qPCR assay with splenic T cells, a broad survey of CEACAM1 isoform expression in primary T cells isolated from several different tissues was performed. It was noted that, unlike CD3+ T cells isolated from spleen or MLN wherein CEACAM1 expression was low and CEACAM1-L isoforms predominated over CEACAM1-S isoforms, CD3+ T cells from the intestinal LP of the duodenum, jejunum, ileum or colon and Peyer's patches (PP) were observed to express an overall increase in CEACAM1 transcript levels with significantly greater quantities of CEACAM1-S isoforms relative to CEACAM1-L isoforms. Thus, an average L:S ratio of <1.0 (PP: 0.53 ± 0.08; small intestine: 0.36 ± 0.11; and colon; 0.19 ± 0.04) was observed, which was reversed from peripheral T cells (data not shown). Accordingly, it was noted that the quantity and L:S ratio of CEACAM1 expression varied cephalocaudally along the axis of the gut. Specifically, the amounts of CEACAM1 and L:S ratio were more similar to spleen, MLN and peripheral blood in the proximal intestines. Furthermore, the levels of CEACAM1 increased and the L:S ratio progressively decreased from the distal small intestine (ileum) to the colon. As a result, LP T cells of the PP and colon contained the highest levels of CEACAM1 and the lowest L:S ratios relative to all other compartments examined. This was verified by immunoblotting with the CC1 mAb, further confirming that T cells isolated from PP and colonic LP expressed higher CEACAM1 amounts relative to peripheral blood and especially CEACAM1-S isoforms which was most notable in the colonic LP (data not shown). T cells were purified from spleen (SP), PP, colon lamina propria (LP) of WT mice and spleen of 4S Tg and 4L Tg mice as well as B cells from spleen of WT mice. These studies both confirm the increased expression of CEACAM1 within intestinal T cells and further indicate that CEACAM1-S isoforms are not only expressed by primary T cells but establish that they are a major isoform within intestinal T cells and potentially other tissue compartments.

Pan T cells from the spleen were stimulated with plate-bound coated CD3, and CD3/CD8 for 2 and subsequently 4 days. CEACAM1-S, -L and total CEACAM1 quantitative PCR were analyzed. It was observed that CEACAM1 expression, which is low on resting T cells, is transcriptionally regulated by ligation of T cells through the TCR/CD3 complex (signal 1) but negatively regulated when the TCR/CD3 complex is co-engaged by the classical co-stimulatory signal (signal 2) provided by CD28 (Figure 11). These studies show that CEACAM1-S and -L isoforms are positively regulated by tolerogenic signals.

### EXAMPLE 4: Forced expression of CEACAM1 on T cells promotes the generation of cells that co-express LAP (Treg) and PD-1 (Tfollicular helper or T_{FH}).

**CEACAM1-S independently regulates the expansion and function of CD4+ T cells in which it is preferentially expressed.** The observations described herein, of preferential CEACAM1 expression on CD4+CD25-LAP+ from WT mice led to a question of the ability of CEACAM1 to regulate these cells and, further, to interrogate the relative contribution of CEACAM1-L and CEACAM1-S isoforms in this process. Utilizing CEACAM1 transgenic (Tg) mice that over-express CEACAM1-specifically in T cells, the frequency of CD4+CD25-LAP+ T cells in mucosal tissues was examined under conditions of forced over-expression of the CEACAM1-4L or -4S isoforms. These data demonstrated a significant increase in the percentage of CD4+CD25-LAP+ T cells in the MLN from CEACAM1-4S (4S Tg) mice relative to WT controls (Figure 7A and 7B) and in *CEACAM1*^{*-*/*-*}-4S Tg mice compared to *CEACAM1*^{*-*/*-*} controls (Figure 7C and 7D), wherein the CEACAM1-4S isoform is the only isoform expressed by T cells. This increase in CD4+CD25-LAP+ cells was specifically detected under conditions of over-expression of the CEACAM1-4S isoform in both WT and *CEACAM1*^{*-*/*-*} animals (Figure 7A and 7C) and was notably absent in CEACAM1-4L transgenic mice in which the CEACAM1 isoform was overexpressed (Figure 7B and 7D). In stark contrast, the analysis did not reveal any differences in the frequency of CD4+CD25+Foxp3+ T cells upon forced expression of CEACAM1-4S in T cells relative to that observed in WT mice (data not shown). In order to ascertain whether enrichment of activating CEACAM1-S was functionally relevant to CD4+CD25-LAP+ T cells, the in vitro suppressive function of CD4+LAP+ T regulatory cells obtained from the mesenteric lymph node of 4S Tg mice was examined. It was found that these cells possessed enhanced regulatory activity when compared to CD4+CD25-LAP+ T cells from WT controls (Figure 7E). In contrast, no differences were observed in the regulatory potential between 4S Tg and WT CD4+CD25+ Treg cells obtained from the MLN in similar assays (Figure 7E). Taken together, these studies indicate that the relative increase of CEACAM1-4S, in the absence of any contribution from the inhibitory CEACAM1-L isoforms, specifically enhances both the development and functional capacity of regulatory CD4+CD25-LAP+ T cells.

Previous studies have shown that CD4+CD25-LAP+ T cells accumulate in intestinal tissues under steady-state and inflammatory conditions and that orally administered CD3-specific antibodies induce CD4+CD25-LAP+ regulatory T cells in MLN (Boirivant et al., 2008; Ochi et al., 2006). This approach was used in order to determine whether CEACAM1 is specifically involved in the induction phase of CD4+CD25-LAP+ T cell development under conditions of physiological CEACAM1 expression. Oral administration of anti-CD3 antibody significantly increased the percentage of CD4+CD25-LAP+ T cells in the MLN of WT but not CEACAM1^{-/-} mice (Figure 7F). No significant differences were identified in the proportions of CD3+CD25+/-LAP+ Treg cells in spleen, MLN, PP or blood of untreated or isotype-treated *CEACAM1*^{*-*/*-*} and WT mice (data not shown). In contrast, oral administration of a CD3-specific antibody was not observed to alter the percentage of CD4+Foxp3+ T regulatory cells in the MLN of WT or *CEACAM1*^{*-*/*-*} mice (data not shown). Mice were fed anti-CD3 (5 µg/mouse/day) or an isotype control for 5 consecutive days and tissues were analyzed after 7 days for analysis of regulatory T cell frequency. These studies confirm a physiological role played by CEACAM1 in regulating the expansion of CD4+CD25-LAP+ T cells in mucosal tissues as well as in promoting their suppressive function.

Given the co-expression of CEACAM1 and PD1 on T cells in multiple tissue compartments, the impact of high levels of CEACAM1-4S on the frequency of cells that co-express PD-1, or so-called T_{FH} cells, in the spleen, MLN and PP of 4S Tg mice was examined. Flow cytometric analysis of each of these tissues revealed an enrichment not only of PD1 itself but also of the PD1+CXCR5+ population of T_{FH} cells together with CEACAM1 in the 4S Tg mice compared to their WT littermate controls (Figure 13A-13B). Importantly, CEACAM1-4S-mediated enrichment of T_{FH} cells occurred independently of CEACAM1-L isoforms since *CEACAM1*^{*-*/*-*}-4S Tg mice also contained higher levels of T_{FH} cells than their *CEACAM1*^{*-*/*-*} counterparts (Figure 13C). Collectively, these data are consistent with a role for CEACAM1 and especially the CEACAM1-S isoforms in promoting the development of two types of accessory cells that express LAP (CD4+CD25+LAP+ T cells) and PD1 (T_{FH} cells) which are not only enriched in organized lymphoid structures and mucosal tissues but are also known to be involved in immune tolerance and B cell switching (Cerutti, 2008; Good-Jacobson et al.).

### EXAMPLE 5: CEACAM1-S isoforms provide unique activating signals that sensitize T cells to activation induced cell death and promote the development of regulatory populations of T cells.

Carcinoembryonic antigen cell adhesion molecule like I (CEACAM1) is expressed on activated T cells and signals through either a long (L) cytoplasmic tail containing immune receptor tyrosine based inhibitory motifs, which provide inhibitory function, or a short (S) cytoplasmic tail that signals at least in part via NFAT (data not shown). Previous studies on T cells from the periphery have shown that CEACAM1-L isoforms predominate with little CEACAM1-S isoforms in mouse and human. It is demonstrated herein that this is not the case in tissue resident T cells such as in intestines and gut associated lymphoid tissues which demonstrate predominant expression of CEACAM1-S isoforms relative to CEACAM1-L isoforms in both human and mouse. Thus primary mouse and human T cells express both isoforms of CEACAM1.

**CEACAM1-4S functions as an independent signaling unit in the induction of T cell activation.** Although CEACAM1-L isoform expression in T cells is well known to provide important negative regulation of TCR/CD3 complex function after ITIM phosphorylation by the src-related kinase, p56^{lck}, and recruitment of SHP1 resulting in dephosphorylation of CD3-ζ and ZAP70 (Chen et al., 2008), very little is known about CEACAM1-S function in general or in T cells in particular. Previous results in T cells are limited to transfected in vitro model systems and have been controversial with both stimulatory and inhibitory functions assigned to CEACAM1-S splice variants (Chen et al., 2004a; Chen et al., 2004b). Given the impossibility of generating a knockout mouse for a specific CEACAM1-S splice variant without also deleting the alternative CEACAM1-L variant, CEACAM1-4S (4S) transgenic (Tg) mice were generated, overexpressing the mouse 4S isoform specifically in T cells under the human CD2 (hCD2) promoter in order to explore the function of CEACAM1-S isoforms in T cells in vivo. The overexpression of 4S in T cells was confirmed by flow cytometry using the CC1 mAb. These studies confirmed that the T cells in the 4S Tg mice uniformly expressed increased CEACAM1 as shown by flow cytometry using the CC1 monoclonal antibody and anti-CD3 antibody in cells isolated from spleen (data not shown). Overexpression of CEACAM1-S isoforms on T cells in 4S Tg mice as measured by qPCR analysis using the assay described in Figure 8 confirming the dominance of this isoform in the Tg mice (data not shown).

When examined by flow cytometry, 4S Tg mice exhibited greater quantities of activated CD4+ and CD8+ T cells as shown by lower percentages of CD62LhiCD44lo naive T cells, and a higher percentage of CD62LloCD44hi or CD62LhiCD44hi cells consistent with effector memory and potentially central memory T cells (Seder et al., 2003) respectively, in spleen, MLN, PP and colon LP (data not shown). It was noted that 4S Tg mice exhibited increased numbers of apoptotic CD3+ T cells (annexin V+7-AAD-CD3+ cells) within the peripheral blood, spleen, MLN and PP which correlated inversely with decreased levels of CD3+ cells (Figure 12A). In spite of this, total PP and MLN cell counts were found to be very similar between WT and CEACAM1-4S Tg mice (data not shown) indicating that overexpression of CEACAM1-4S was selectively depleting T cells. Intracellular cytokine staining of the CD4+ T cells isolated from spleen, MLN and PP also revealed that IL-2, IL-4, IL-10, IL-17, IFN-γ and TGF-β were all increased in the T cells of 4S Tg mice when compared to that observed in WT littermates (data not shown). CEACAM1-S induction of cytokines (interferon-y) influenced the total CD4+ T cell population (CD4+ T cells isolated from PP of WT or 4S Tg mice) consistent with a broad activating function of the 4S isoform when expressed at high levels (data not shown). CD3+ T cells isolated from the spleen of 4S Tg mice also exhibited a higher proliferation rate and more IFN-γ and IL-2 secretion upon anti-CD3 stimulation ex vivo in comparison to that associated with T cells from WT littermate controls (data not shown). However, with longer time-periods of culture, the proliferation rate of splenic CD3+ T cells, as measured by [³H]-thymidine incorporation as well as the production of IFN-γ and IL-2, from the 4S Tg mice was observed to be lower than that detected with the WT littermates after stimulation, consistent with in vitro T cell collapse due to increased activation-induced cell death (AICD) in the presence of the CEACAM1-4S isoform (Figure 12B). Consistent with this, splenic 4S Tg T cells exhibited increased annexin V staining (Figure 12C) and cleavage of caspase 3 (Figure 12D) after 3 hours of anti-CD3 stimulation. These results show that the mouse 4S isoform both co-stimulates primary T cells and sensitizes them to activation induced cell death (AICD), supporting previous in vitro transfection studies that have ascribed activating functions to this CEACAM1 isoform in contrast to the inhibitory functions of the L isoform (Chen et al., 2004a; Chen et al., 2004b). Moreover, these observations further support the idea that both the increased total CEACAM1 expression and the enrichment of the uniquely activating CEACAM1-S isoform observed on T cells in intestinal tissues is associated with the effector memory T cell phenotype which predominates in the normal intestinal LP under steady-state conditions (Hurst et al., 1999) as well as the selective expansion of T cells with a regulatory phenotype and focused on the promotion of secretory IgA.

**The activating function of CEACAM1-4S in T cells is observed independently of CEACAM1-L isoform expression.** Since the studies performed in 4S Tg mice were undertaken in the context of endogenous CEACAM1 expression, it was possible that the apparent activating and inhibiting functions of the 4S isoform were either the consequence of CEACAM1-mediated neutralization of CEACAM1-L inhibitory function and/or the result of a direct activating signal by CEACAM1-S. To examine this question, 4S Tg mice that lacked expression of any other CEACAM1 isoforms were generated by crossing 4S Tg mice with *CEACAM1*^{*-*/*-*} mice on a C57BL/6 background (*CEACAM1*^{*-*/*-*}-4S Tg). Although less than that observed in 4S Tg T cells containing endogenous levels of CEACAM1 in all cell types, *Ceacam1*-1^{*-*/*-*}-4S Tg mice still contained more activated T cells in vivo as revealed by lower percentages of CD4+(or CD8+) CD62LhiCD44lo naive T cells, and higher percentages of CD4+(or CD8+) CD62LloCD44hi effector memory T cells in spleen, MLN, PP and colon LP (data not shown) in comparison to *CEACAM1*^{*-*/*-*} littermates. *CEACAM1*^{*-*/*-*}-4S Tg mice also exhibited more apoptotic, Annexin+ CD3+ T cells within the MLN, blood and PP (Figure 12E) inversely correlating with decreased levels of CD3+ cells (data not shown). CD4+ T cells isolated from the spleens of *CEACAM1*^{*-*/*-*}-4S Tg mice also proliferated more vigorously and secreted more IL-2 and IFN-γ upon anti-CD3 stimulation. Notably, splenic CD4+ T cells from *CEACAM1*^{*-*/*-*} mice exhibited increased activation upon anti-CD3 stimulation that was intermediate to the anti-CD3 induced stimulation observed with WT and *CEACAM1*^{*-*/*-*}-4S Tg T cells (data not shown). On the other hand, splenic T cells from mice in which only the CEACAM1-4L (4L) isoform was expressed by T cells, generated by crossing previously established 4L Tg mice (Nagaishi et al., 2006) with *CEACAM1*^{*-*/*-*} mice, exhibited a blunted response to anti-CD3 stimulation which was less than that observed with WT T cells and is consistent with the direct inhibitory functions of this isoform. Taken together, these studies show that loss of dominant endogenous CEACAM1-L inhibitory function in *CEACAM1*^{*-*/*-*}mice or gain of CEACAM1-S expression independently of CEACAM1-L isoforms, as observed in *CEACAM1*^{*-*/*-*}-4S Tg T cells, results in enhanced TCR/CD3 complex signaling and sensitization to activation induced cell death in the latter case. In comparison, gain of CEACAM1-L isoforms independently of CEACAM1-S isoforms leads directly to inhibition of T cell function. Thus CEACAM1-S and CEACAM1-L are able to function as independent activating and inhibitory signaling units in T cells that are in the end both associated with immune tolerance.

In the case of CEACAM1-S, this activating function is associated ultimately with regulation of immune responses as it results in sensitization to activation induced cell death and the promotion of T cells with regulatory and thus inhibitory function such as CD4+CEACAM1+LAP+T cells. In contrast, the CEACAM1-L isoforms directly deliver inhibitory signals to T cells.

### Discussion

Described herein is evidence for tissue and cell-type specific expression of CEACAM1, the association of CEACAM1 with specific types of cell surface molecules that are associated with immune tolerance such as TIM3, PD-1 and LAP as well as the regulation of CEACAM1 splicing in T cells which demonstrates the important role played by CEACAM1-S isoforms and the unique predominance of CEACAM1-S isoforms in intestinal resident T cells and specific subsets of T cells associated with immune regulation and humoral immunity. Although CEACAM1 is an activation-induced molecule on T cells, the observed tissue-specific splicing was not due to activation of the T cells per se as it was not observed when T cells were stimulated ex vivo. Adoptive transfer experiments demonstrated that CEACAM1-S dominance was gained by naive peripheral T cells upon entry into the intestines, and was lost when mucosal T cells were removed from the intestines and re-stimulated ex vivo. This indicates that the specific mucosal milieu of the intestines promotes CEACAM1-S expression in T cells. Moreover, the results in 4S Tg mice indicate CEACAM1-S facilitates T cell activation, sensitization to activation induced cell death and the induction of secretory IgA immunity and cells with regulatory function. Consistent with this, T cell subsets which bear a memory phenotype characteristic of mucosal tissues (Gibbons et al., 2011) and specific subsets of T cells such as CD4+CD25-LAP+ and T_{FH} cells which possess regulatory functions and encourage secretory immunity display an enrichment upon CEACAM1-S expression and are driven by its presence. Thus, CEACAM1-S expression is promoted and maintained by the mucosal environment and facilitates T cell activation linked to the generation of cell types responsible for mucosal regulation, tolerance and protection.

Importantly, the studies described herein also demonstrate that CEACAM1-S is not a minor isoform in primary T cells as was currently thought (Gray-Owen et al., 2006). In transgenic mice with conditional expression of CEACAM1 in T cells, it was found that CEACAM1-S provides unique co-stimulatory signals which promote both activation induced cell death and an effector memory phenotype, whereas CEACAM1-L is directly co-inhibitory for TCR/CD3 complex signaling (Nagaishi et al., 2006). Moreover, it is now shown that CEACAM1-S in T cells can elicit this stimulation as an independent signaling unit without CEACAM1-L assistance in vivo. Through these properties, CEACAM1-S regulates specific subsets of T cells. These cell populations include CD4+LAP+ T cells and T_{FH} cells which are an integral component of mucosal tissues and organized lymphoid structures, respectively, and are known to regulate immune tolerance and secretory immunity (Linterman et al., 2011). Accumulation of such cell surface molecules such as CEACAM1 together with PD1, TIM3 and LAP as well as cell types associated with these cell surface molecules (e.g. CD4+CEACAM1+LAP+ or CD4+CEACAM+PD1+ T cells) amongst tumor infiltrating lymphocytes would be predicted to be deleterious for the host resulting in either direct suppression of effector T cell function or inhibition of the effector T cells through the effects of the accumulated regulatory T cells. In view of CEACAM1-S enrichment in these latter cell types irrespective of their localization to peripheral or mucosal tissues as well as with the observations that the intestinal tissues facilitate CEACAM1-S expression, these studies also raise that possibility that CD4+LAP+ T cells and T_{FH} cells may be born in and/or modified remotely by mucosal factors.

Although little is known about CEACAM1-S signaling in T cells, in epithelial cells it has been shown to associate with the cytoskeleton (actin and tropomyosin), calmodulin and annexin II (Edlund et al., 1996; Kirshner et al., 2003; Schumann et al., 2001). Such intracellular associations have interestingly been linked to the promotion of apoptosis of mammary epithelial cells during acini morphogenesis (Kirshner et al., 2003). In addition, consistent with previous transfection studies in Jurkat T cells, CEACAM1-S signaling was able to activate NFAT in vivo (Chen et al., 2004b). As shown here, this was associated with the upregulation of NFAT-dependent soluble and cell surface molecules such as PD1 and CD40L (data not shown). Thus these studies not only demonstrate the importance of CEACAM1-S in T cells under physiological conditions but also their role in delivering independent signals that may oppose those provided by CEACAM1-L isoforms and drive the cell to a unique destination that is associated with either cell death or a regulatory function.

### Experimental Procedures

*Animals.* Wild-type (WT) C57BL/6 (B6) mice were purchased from The Jackson Laboratory. hCD2 promoter controlled CEACAM1-4S transgenic mice were generated by a similar strategy as previously described in the of Brigham and Women's Hospital Transgenic Core Facility (Nagaishi et al., 2006). *CEACAM1*^{*-*/*-*} mice were previously described (Leung et al., 2006). Swiss Webster germ-free (SWGF) mice and C57BL/6 germ-free mice (B6GF) were maintained in germ-free isolators at Taconic. All other mice were maintained under specific pathogen-free conditions at the Harvard Center for Comparative Medicine at Harvard Medical School. All mice were used between 8 and 12 weeks of age.

*Flow Cytometry.* For CEACAM1 expression assays, cells were incubated with the CC1 antibody for 20 min followed by FITC-conjugated rat anti-mouse IgG1. For other cell surface proteins, cells were stained with the indicated fluorescence-conjugated antibodies for 20 minutes, washed and resuspended with 1% BSA/PBS FACS staining buffer containing DAPI (Invitrogen). Cell apoptosis assays were performed according to the Annexin V-FITC Apoptosis Detection Kit manual (BD Bioscience). For intracellular cytokine staining, cells were stimulated with ionomycin (Sigma, 2 tg/ml), PMA (Sigma, 30 ng/ml) and GOLGISTOP™ (BD Bioscience) for 4 hours followed by intracellular cytokine staining using CYTOFIX/CYTOPERM ™ solution kit according to the manufacturer's instructions (BD Bioscience). IgA staining was performed according to the instructions provided with the rat anti-mouse IgA antibody (C10-3). CD4+LAP+ T cells were stained for 20 min with biotinylated anti-LAP antibody (R&D Systems) followed by APC-conjugated streptavidin (BD Biosciences) as described previously (Ochi et al., 2006). T_{FH} cells were stained for CXCR5 and PD1 as described previously (Linterman et al., 2011). All stained cells were analyzed on an LSRII™ (BD Biosciences) or a MACSQUANT™ (Miltenyi Biotech) flow cytometer, and data were analyzed using FLOWJO™ software.

*Oral feeding of anti-CD3.* Mice were fed with anti-CD3 antibody using a protocol described previously (Ochi et al., 2006). Specifically, groups of paired mice were fed anti-CD3 or IgG isotype control antibody at a dose of 5 µg/mouse/day for 5 days. After one day's rest, mice were sacrificed at day 7 and the percentage of CD4+LAP+ regulatory T cells was determined in specific lymphoid tissues.

*Suppression assays.* Regulatory T cell suppression assays were performed as previously described (Fantini et al., 2007). Briefly, 1x105 freshly isolated splenic CD4+CD25- T cells were stained with CFSE and mixed with an equal number of either CD4+CD25+ or CD4+LAP+ T regulatory cells isolated via magnetic sorting (Miltenyi). T cell mixtures were then activated in the presence of soluble anti-CD3 (1 µg/ml) and 4x10⁵ irradiated total splenocytes as antigen presenters. After 4 days of culture, CFSE dilution of the responder CD4+CD25- T cells was measured as an indication of proliferation.

*Total Serum and secretion Immunoglobulin ELISA.* The sera, intestinal washes or fecal extracts were collected for ELISA as previously described (Hapfelmeier et al., 2010). Data are presented as mean ± S.D.

*Immunohistochemical analysis.* PP were isolated and embedded in OCT compound (Sakura Finetechnical) and snap frozen in dry ice. Frozen sections were prepared on micro slides using a cryostat (Leica), and stored at - 70°C until use. The slides were fixed in 100% acetone for 5 min at 4°C and air dried at room temperature. Cryostat sections were blocked with 10% normal goat serum for 1 hour at room temperature (RI). For B220 and IgA staining, the slides were stained with purified rat anti-mouse IgA antibody for 1 hour at RI, followed by incubation with ALEXA FLURO™ 568 conjugated goat anti-rat IgG secondary antibody (Invitrogen) and FITC-conjugated anti-mouse B220 antibody (BD Bioscience) for 1 hour at RI. For NFAI staining, the slides were stained with biotin conjugated-anti-mouse CD4 antibody and rabbit anti-NFAIl antibodies for 2 hours at RI, followed by incubation with rhodamine red-X-streptavidin (Jackson ImmunoResearch) and ALEXA FLUOR® 488 F(ab')2 fragment of goat anti-rabbit IgG (H+L) (Invitrogen) for 1 hour at RI. The counterstaining was performed using DAPI (Invitrogen). The specimens were analyzed using a NIKON ECLIPSE II™ microscope.

*Statistics.* The Mann-Whitney U-test (two-tailed) was used to assess differences in log10 colony forming units (c.f.u.) in quantitative culture assays. The t-test (two-tailed) was used to assess differences between means for other data analyzed. Tests with a P-value less than 0.05 were considered statistically significant. *Antibodies and Reagents.* A mAb specific for mouse CEACAM1, CC1, was previously described (Dveksler et al., 1993). 5F4 is a mouse anti-human CEACAM1 specific mAb (Morales et al., 1999). Caspase-3 and NFAT1 antibodies were purchased from Cell Signaling. GAPDH and 13-actin antibodies were obtained from Sigma-Aldrich. Biotinylated *anti-LAP* antibody was from R&D Systems (Ochi et al., 2006). All other antibodies used for flow cytometry or immunohistochemistry staining were purchased from BD Biosciences, eBioscience or Biolegend. Human rIL-2 for the stimulation of mouse primary T cells was provided by the National Institutes of Health.

*Lymphocyte Isolation.* Single cell suspensions of mononuclear cells from spleen, lymph nodes, blood, Peyer's patches (PP) (Iwasaki and Kelsall, 1999) or intestinal lamina propria (LP) were prepared as previously described (Nakajima et al., 2002). PP or intestines (removed PP, rinsed with PBS and cut in small pieces) were treated with media containing dithiothreitol 100 µg/ml (Sigma), 25 mM Hepes, 10% fetal bovine serum (FBS), and 5 mM EDTA in RPMI1640 for 30 min at 37 °C to remove epithelial cells, and were washed extensively with RPMI1640. Tissues were further digested in RPMI 1640 containing 2% FBS, collagenase D (400 U/ml, Roche) and DNase I (0.1 mg/ml, Sigma) in a shaking incubator at 37 °C for 30 min. Cells were then layered on a 40-75% Percoll gradient (GE Healthcare) and lymphocyte-enriched populations were isolated from the cells at the 40-75% interface. After washing, single cell suspensions were prepared. Cells were directly used for flow staining, or were stained with PE conjugated anti-CD3 antibody and followed by anti-PE MicroBeads according to the instructions provided by Miltenyi Biotech to further isolate CD3+ T cells.

Single-cell isolations from spleen and mesenteric lymph nodes were performed with a 40 tm cell strainer (BD Bioscience) and red blood lysis buffer (Sigma) for depletion of red blood cells. CD3+ or CD4+ T cells were isolated from spleen and/or lymph nodes of mice by pan T cell isolation kit or CD4+ T cell isolation kit for mouse (Miltenyi Biotech) according to the manufacturer's instructions. *Quantification of Peyer's patch.* The entire small intestines were removed from mice. Lipid on the surface was carefully removed, and then the number and size of PPs were determined by macroscopic observation. The PP score was established by combining the number and size, and calculated in a blinded fashion, as described previously (Barreau et al., 2007). For absolute quantification of PP numbers, SI were opened along the mesenteric border, washed by shaking in dH2O for one hour and fixed in 5% glacial acetic acid overnight. After washing in dH2O for another hour, tissues were stained by briefly incubating in Unna's polychrome methylene blue stain (31.3mM methylene blue, 72.4mM potassium carbonate). Tissues were washed once more, then left in dH2O for 48h to allow differentiation of the stain.

*Cell Culture, Proliferation and Determination of Cytokine Production.* Cells were resuspended in complete RPMI 1640 medium containing 10% FBS, 1% penicillin and streptomycin, 2 mM glutamine, 1 mM sodium pyruvate, 20 mM HEPES, and 1% nonessential amino acids (all from Invitrogen) at a density of 1 x 106 cells/ml, and cultured in 96-well flat bottomed plates with the indicated concentrations of plate-bound anti-CD3 antibodies (eBioscience). Culture supernatants were harvested at 24 h and cytokine production determined by ELISA (BD Biosciences) according to the manufacturer's instructions. Proliferation assays were performed by addition of [3H]-thymidine (1.0 µCi/well) at 48 hours for 8 hours.

*Protein Isolation and Western Blotting.* Spleen and MLN mononuclear cells were washed twice in cold PBS. Total protein was isolated with RIPA buffer as previously described (Chen et al., 2008). The nuclear and cytoplasmic protein was isolated using NE-PER Nuclear and Cytoplasmic Extraction Kit (Thermo Scientific). The concentration of proteins in the supernatants was assessed using the BAC Protein Assay (Thermo Scientific). Immunoblotting was performed as previously described by using specific antibodies as indicated in the results and legends (Chen et al., 2008).

*Real-time Quantitative PCR.* RNA was extracted using RNEASY™ Mini kit (Qiagen) according to the manufacturer's instructions, and genomic DNA was removed with DNase I. qPCR was performed as described previously using SYBR GREEN™ quantitative PCR master mix (Roche) (Nagaishi et al., 2006). All runs were accompanied by two internal control genes, 13-actin and GAPDH. Samples were normalized using a ΔΔ cycle threshold-based algorithm to provide arbitrary units representing relative expression levels between samples. The following primers were used for mouse CEACAM1:
CEACAM1-L, 5'-GCGAGATCTCACAGAGCACA-3' (forward; SEQ ID NO: 35) and 5'-GCTGGGAATTGAAGTTCAGG-3' (reverse; SEQ ID NO: 36); CEACAM1-S, 5'-CTGGCATCGTGATTGGAGTT-3' (forward; SEQ ID NO:37) and 5'-CAGAAGGAGCCAGATCCG-3' (reverse; SEQ ID NO: 38). hnRNP expression was determined by qPCR, as described previously (Dery et al., 2011).

### References

Abou-Rjaily, G. A., Lee, S. J., May, D., Al-Share, Q. Y., Deangelis, A. M., Ruch, R. J., Neumaier, M., Kalthoff, H., Lin, S. H., and Najjar, S. M. (2004). CEACAM1 modulates epidermal growth factor receptor--mediated cell proliferation. J. Clin. Invest. 114, 944-952.
Azuz-Lieberman, N., Markel, G., Mizrahi, S., Gazit, R., Hanna, J., Achdout, H., Gruda, R., Katz, G., Arnon, T. I., Battat, S., et al. (2005). The involvement of NK cells in ankylosing spondylitis. Int. Immunol. 17, 837-845.
Barreau, F., Meinzer, U., Chareyre, F., Berrebi, D., Niwa-Kawakita, M., Dussaillant, M., Foligne, B., Ollendorff, V., Heyman, M., Bonacorsi, S., et al. (2007). CARD15/NOD2 is required for Peyer's patches homeostasis in mice. PLoS One 2, e523.
Baumgart, M., Dogan, B., Rishniw, M., Weitzman, G., Bosworth, B., Yantiss, R., Orsi, R. H., Wiedmann, M., McDonough, P., Kim, S. G., et al. (2007). Culture independent analysis of ileal mucosa reveals a selective increase in invasive Escherichia coli of novel phylogeny relative to depletion of Clostridiales in Crohn's disease involving the ileum. ISME J. 1, 403-418.
Bergqvist, P., Stensson, A., Lycke, N. Y., and Bemark, M. (2010). T cell-independent IgA class switch recombination is restricted to the GALT and occurs prior to manifest germinal center formation. J. Immunol. 184, 3545-3553.
Bjorneklett, A., Fausa, O., and Midtvedt, T. (1983). Bacterial overgrowth in jejunal and ileal disease. Scand. J. Gastroenterol. 18, 289-298.
Boirivant, M., Amendola, A., Butera, A., Sanchez, M., Xu, L., Marinaro, M., Kitani, A., Di Giacinto, C., Strober, W., and Fuss, I. J. (2008). A transient breach in the epithelial barrier leads to regulatory T-cell generation and resistance to experimental colitis. Gastroenterology 135, 1612-1623 e1615.
Borsutzky, S., Cazac, B. B., Roes, J., and Guzman, C. A. (2004). TGF-beta receptor signaling is critical for mucosal IgA responses. J. Immunol. 173, 3305-3309.
Boullier, S., Tanguy, M., Kadaoui, K. A., Caubet, C., Sansonetti, P., Corthesy, B., and Phalipon, A. (2009). Secretory IgA-mediated neutralization of Shigella flexneri prevents intestinal tissue destruction by down-regulating inflammatory circuits. J. Immunol. 183, 5879-5885.
Boulton, I. C., and Gray-Owen, S. D. (2002). Neisserial binding to CEACAM1 arrests the activation and proliferation of CD4+ T lymphocytes. Nat. Immunol. 3, 229-236.
Cazac, B. B., and Roes, J. (2000). TGF-beta receptor controls B cell responsiveness and induction of IgA in vivo. Immunity 13, 443-451.
Cerutti, A. (2008). The regulation of IgA class switching. Nat. Rev. Immunol. 8, 421-434.
Chen, C. J., Lin, T. T., and Shively, J. E. (1996). Role of interferon regulatory factor-1 in the induction of biliary glycoprotein (cell CAM-1) by interferon-gamma. J. Biol. Chem. 271, 28181-28188.
Chen, C. J., and Shively, J. E. (2004a). The cell-cell adhesion molecule carcinoembryonic antigen-related cellular adhesion molecule 1 inhibits IL-2 production and proliferation in human T cells by association with Src homology protein-1 and down-regulates IL-2 receptor. J. Immunol. 172, 3544-3552.
Chen, D., Iijima, H., Nagaishi, T., Nakajima, A., Russell, S., Raychowdhury, R., Morales, V., Rudd, C. E., Utku, N., and Blumberg, R. S. (2004b). Carcinoembryonic antigen-related cellular adhesion molecule 1 isoforms alternatively inhibit and costimulate human T cell function. J. Immunol. 172, 3535-3543.
Chen, T., Zimmermann, W., Parker, J., Chen, I., Maeda, A., and Bolland, S. (2001). Biliary glycoprotein (BGPa, CD66a, CEACAM1) mediates inhibitory signals. J. Leukoc. Biol. 70, 335340. Chen, Z., Chen, L., Qiao, S. W., Nagaishi, T., and Blumberg, R. S. (2008). Carcinoembryonic antigen-related cell adhesion molecule 1 inhibits proximal TCR signaling by targeting ZAP-70. J. Immunol. 180, 6085-6093.
Cong, Y., Wang, L., Konrad, A., Schoeb, T., and Elson, C. O. (2009). Curcumin induces the tolerogenic dendritic cell that promotes differentiation of intestine-protective regulatory T cells. Eur. J. Immunol. 39, 3134-3146.
Coutelier, J. P., Godfraind, C., Dveksler, G. S., Wysocka, M., Cardellichio, C. B., Noel, H., and Holmes, K. V. (1994). B lymphocyte and macrophage expression of carcinoembryonic antigen-related adhesion molecules that serve as receptors for murine coronavirus. Eur. J. Immunol. 24, 1383-1390.
Dery, K. J., Gaur, S., Gencheva, M., Yen, Y., Shively, J. E., and Gaur, R. K. (2011). Mechanistic control of carcinoembryonic antigen-related cell adhesion molecule-1 (CEACAM1) splice isoforms by the heterogeneous nuclear ribonuclear proteins hnRNP L, hnRNP A1, and hnRNP M. J. Biol. Chem. 286, 16039-16051.
Donda, A., Mori, L., Shamshiev, A., Carena, I., Mottet, C., Heim, M. H., Beglinger, C., Grunert, F., Rochlitz, C., Terracciano, L., et al. (2000). Locally inducible CD66a (CEACAM1) as an amplifier of the human intestinal T cell response. Eur. J. Immunol. 30, 2593-2603.
Dveksler, G. S., Pensiero, M. N., Dieffenbach, C. W., Cardellichio, C. B., Basile, A. A., Elia, P. E., and Holmes, K. V. (1993). Mouse hepatitis virus strain A59 and blocking antireceptor monoclonal antibody bind to the N-terminal domain of cellular receptor. Proc. Natl. Acad. Sci. U.S.A. 90, 1716-1720.
Edlund, M., Blikstad, I., and Obrink, B. (1996). Calmodulin binds to specific sequences in the cytoplasmic domain of C-CAM and down-regulates C-CAM self-association. J. Biol. Chem. 271, 1393-1399.
Fagarasan, S. (2008). Evolution, development, mechanism and function of IgA in the gut. Curr. Opin. Immunol. 20, 170-177.
Fagarasan, S., Kinoshita, K., Muramatsu, M., Ikuta, K., and Honjo, T. (2001). In situ class switching and differentiation to IgA-producing cells in the gut lamina propria. Nature 413, 639.
Fagarasan, S., Muramatsu, M., Suzuki, K., Nagaoka, H., Hiai, H., and Honjo, T. (2002). Critical roles of activation-induced cytidine deaminase in the homeostasis of gut flora. Science 298, 1424-1427.
Fantini, M. C., Dominitzki, S., Rizzo, A., Neurath, M. F., and Becker, C. (2007). In vitro generation of CD4+ CD25+ regulatory cells from murine naive T cells. Nat Protoc 2, 1789-1794. Fazilleau, N., Mark, L., McHeyzer-Williams, L. J., and McHeyzer-Williams, M. G. (2009a). Follicular helper T cells: lineage and location. Immunity 30, 324-335.
Fazilleau, N., McHeyzer-Williams, L. J., Rosen, H., and McHeyzer-Williams, M. G. (2009b). The function of follicular helper T cells is regulated by the strength of T cell antigen receptor binding. Nat. Immunol. 10, 375-384.
Gencheva, M., Chen, C. J., Nguyen, T., and Shively, J. E. (2010). Regulation of CEACAM1 transcription in human breast epithelial cells. BMC Mol. Biol. 11, 79.
Gibbons, D. L., and Spencer, J. (2011). Mouse and human intestinal immunity: same ballpark, different players; different rules, same score. Mucosal Immunol. 4, 148-157.
Good-Jacobson, K. L., Szumilas, C. G., Chen, L., Sharpe, A. H., Tomayko, M. M., and Shlomchik, M. J. (2010). PD1 regulates germinal center B cell survival and the formation and affinity of long-lived plasma cells. Nat. Immunol. 11, 535-542.
Gray-Owen, S. D., and Blumberg, R. S. (2006). CEACAM1: contact-dependent control of immunity. Nat. Rev. Immunol. 6, 433-446.
Greenwald, R. J., Freeman, G. J., and Sharpe, A. H. (2005). The B7 family revisited. Annu. Rev. Immunol. 23, 515-548.
Hapfelmeier, S., Lawson, M. A., Slack, E., Kirundi, J. K., Stoel, M., Heikenwalder, M., Cahenzli, J., Velykoredko, Y., Balmer, M. L., Endt, K., et al. (2010). Reversible microbial colonization of germ-free mice reveals the dynamics of IgA immune responses. Science 328, 1705-1709.
Hashizume, T., Togawa, A., Nochi, T., Igarashi, O., Kweon, M. N., Kiyono, H., and Yamamoto, M. (2008). Peyer's patches are required for intestinal immunoglobulin A responses to Salmonella spp. Infect. Immun. 76, 927-934.
Hermann-Kleiter, N., and Baier, G. (2010). NFAT pulls the strings during CD4+ T helper cell effector functions. Blood 115, 2989-2997.
Huber, M., Izzi, L., Grondin, P., Houde, C., Kunath, T., Veillette, A., and Beauchemin, N. (1999). The carboxyl-terminal region of biliary glycoprotein controls its tyrosine phosphorylation and association with protein-tyrosine phosphatases SHP-1 and SHP-2 in epithelial cells. J. Biol. Chem. 274, 335-344.
Hurst, S. D., Cooper, C. J., Sitterding, S. M., Choi, J., Jump, R. L., Levine, A. D., and Barrett, T. A. (1999). The differentiated state of intestinal lamina propria CD4+ T cells results in altered cytokine production, activation threshold, and costimulatory requirements. J. Immunol. 163, 5937-5945.
Iijima, H., Neurath, M. F., Nagaishi, T., Glickman, J. N., Nieuwenhuis, E. E., Nakajima, A., Chen, D., Fuss, I. J., Utku, N., Lewicki, D. N., et al. (2004). Specific regulation of T helper cell 1-mediated murine colitis by CEACAM1. J. Exp. Med. 199, 471-482.
Kammerer, R., Hahn, S., Singer, B. B., Luo, J. S., and von Kleist, S. (1998). Biliary glycoprotein (CD66a), a cell adhesion molecule of the immunoglobulin superfamily, on human lymphocytes: structure, expression and involvement in T cell activation. Eur. J. Immunol. 28, 3664-3674.
Kaser, A., Lee, A. H., Franke, A., Glickman, J. N., Zeissig, S., Tilg, H., Nieuwenhuis, E. E., Higgins, D. E., Schreiber, S., Glimcher, L. H., et al. (2008). XBP1 links ER stress to intestinal inflammation and confers genetic risk for human inflammatory bowel disease. Cell 134, 743756.
Kirshner, J., Schumann, D., and Shively, J. E. (2003). CEACAM1, a cell-cell adhesion molecule, directly associates with annexin II in a three-dimensional model of mammary morphogenesis. J. Biol. Chem. 278, 50338-50345.
Kuespert, K., Pils, S., and Hauck, C. R. (2006). CEACAMs: their role in physiology and pathophysiology. Curr Opin Cell Biol 18, 565-571.
Lee, H. S., Ostrowski, M. A., and Gray-Owen, S. D. (2008). CEACAM1 dynamics during neisseria gonorrhoeae suppression of CD4+ T lymphocyte activation. J. Immunol. 180, 68276835.
Leung, N., Turbide, C., Olson, M., Marcus, V., Jothy, S., and Beauchemin, N. (2006). Deletion of the carcinoembryonic antigen-related cell adhesion molecule 1 (CEACAM1) gene contributes to colon tumor progression in a murine model of carcinogenesis. Oncogene 25, 5527-5536.
Linterman, M. A., Pierson, W., Lee, S. K., Kallies, A., Kawamoto, S., Rayner, T. F., Srivastava, M., Divekar, D. P., Beaton, L., Hogan, J. J., et al. (2011). Foxp3+ follicular regulatory T cells control the germinal center response. Nat Med 17, 975-982.
Lobo, E. O., Zhang, Z., and Shively, J. E. (2009). Pivotal advance: CEACAM1 is a negative coreceptor for the B cell receptor and promotes CD19-mediated adhesion of B cells in a PI3K-dependent manner. J. Leukoc. Biol. 86, 205-218.
Macpherson, A. J., Hunziker, L., McCoy, K., and Lamarre, A. (2001). IgA responses in the intestinal mucosa against pathogenic and non-pathogenic microorganisms. Microbes Infect. 3, 1021-1035. Macpherson, A. J., and Slack, E. (2007). The functional interactions of commensal bacteria with intestinal secretory IgA. Curr. Opin. Gastroenterol. 23, 673-678.
Macpherson, A. J., and Uhr, T. (2004). Induction of protective IgA by intestinal dendritic cells carrying commensal bacteria. Science 303, 1662-1665.
Manohar, M., Baumann, D. O., Bos, N. A., and Cebra, J. J. (2001). Gut colonization of mice with actA-negative mutant of Listeria monocytogenes can stimulate a humoral mucosal immune response. Infect. Immun. 69, 3542-3549.
Massacand, J. C., Kaiser, P., Ernst, B., Tardivel, A., Burki, K., Schneider, P., and Harris, N. L. (2008). Intestinal bacteria condition dendritic cells to promote IgA production. PLoS One 3, e2588.
Morales, V. M., Christ, A., Watt, S. M., Kim, H. S., Johnson, K. W., Utku, N., Texieira, A. M., Mizoguchi, A., Mizoguchi, E., Russell, G. J., et al. (1999). Regulation of human intestinal intraepithelial lymphocyte cytolytic function by biliary glycoprotein (CD66a). J. Immunol. 163, 1363-1370.
Nagaishi, T., Pao, L., Lin, S. H., Iijima, H., Kaser, A., Qiao, S. W., Chen, Z., Glickman, J., Najjar, S. M., Nakajima, A., et al. (2006). SHP1 phosphatase-dependent T cell inhibition by CEACAM1 adhesion molecule isoforms. Immunity 25, 769-781.
Nakajima, A., Iijima, H., Neurath, M. F., Nagaishi, T., Nieuwenhuis, E. E., Raychowdhury, R., Glickman, J., Blau, D. M., Russell, S., Holmes, K. V., et al. (2002). Activation-induced expression of carcinoembryonic antigen-cell adhesion molecule 1 regulates mouse T lymphocyte function. J. Immunol. 168, 1028-1035.
Niemir, Z. I., Stein, H., Noronha, I. L., Kruger, C., Andrassy, K., Ritz, E., and Waldherr, R. (1995). PDGF and TGF-[beta] contribute to the natural course of human IgA glomerulonephritis. Kidney Int 48, 1530-1541.
Ochi, H., Abraham, M., Ishikawa, H., Frenkel, D., Yang, K., Basso, A. S., Wu, H., Chen, M. L., Gandhi, R., Miller, A., et al. (2006). Oral CD3-specific antibody suppresses autoimmune encephalomyelitis by inducing CD4+ CD25- LAP+ T cells. Nat Med 12, 627-635.
Oestreich, K. J., Yoon, H., Ahmed, R., and Boss, J. M. (2008). NFATc1 regulates PD1 expression upon T cell activation. J. Immunol. 181, 4832-4839.
Oida, T., Zhang, X., Goto, M., Hachimura, S., Totsuka, M., Kaminogawa, S., and Weiner, H. L. (2003). CD4+CD25- T cells that express latency-associated peptide on the surface suppress CD4+CD45RBhigh-induced colitis by a TGF-beta-dependent mechanism. J. Immunol. 170, 2516-2522.
Pan, H., and Shively, J. E. (2010). Carcinoembryonic antigen-related cell adhesion molecule-1 regulates granulopoiesis by inhibition of granulocyte colony-stimulating factor receptor. Immunity 33, 620-631.
Schumann, D., Chen, C. J., Kaplan, B., and Shively, J. E. (2001). Carcinoembryonic antigen cell adhesion molecule 1 directly associates with cytoskeleton proteins actin and tropomyosin. J. Biol. Chem. 276, 47421-47433.
Seder, R. A., and Ahmed, R. (2003). Similarities and differences in CD4+ and CD8+ effector and memory T cell generation. Nat. Immunol. 4, 835-842.
Shannon, C. E. (1997). The mathematical theory of communication. 1963. MD Comput. 14, 306317. Singer, B. B., Scheffrahn, I., Heymann, R., Sigmundsson, K., Kammerer, R., and Obrink, B. (2002). Carcinoembryonic antigen-related cell adhesion molecule 1 expression and signaling in human, mouse, and rat leukocytes: evidence for replacement of the short cytoplasmic domain isoform by glycosylphosphatidylinositol-linked proteins in human leukocytes. J. Immunol. 168, 5139-5146.
Suzuki, K., Meek, B., Doi, Y., Muramatsu, M., Chiba, T., Honjo, T., and Fagarasan, S. (2004). Aberrant expansion of segmented filamentous bacteria in IgA-deficient gut. Proc. Natl. Acad. Sci. U.S.A. 101, 1981-1986.
Taylor, J. M., Ziman, M. E., Fong, J., Solnick, J. V., and Vajdy, M. (2007). Possible correlates of long-term protection against Helicobacter pylori following systemic or combinations of mucosal and systemic immunizations. Infect. Immun. 75, 3462-3469.
van der Waaij, L. A., Limburg, P. C., Mesander, G., and van der Waaij, D. (1996). In vivo IgA coating of anaerobic bacteria in human faeces. Gut 38, 348-354.
Wijburg, O. L., Uren, T. K., Simpfendorfer, K., Johansen, F. E., Brandtzaeg, P., and Strugnell, R. A. (2006). Innate secretory antibodies protect against natural Salmonella typhimurium infection. J. Exp. Med. 203, 21-26.
Wollert, T., Pasche, B., Rochon, M., Deppenmeier, S., van den Heuvel, J., Gruber, A. D., Heinz, D. W., Lengeling, A., and Schubert, W. D. (2007). Extending the host range of Listeria monocytogenes by rational protein design. Cell 129, 891-902.
Iwasaki, A., and Kelsall, B.L. (1999). Freshly isolated Peyer's patch, but not spleen, dendritic cells produce interleukin 10 and induce the differentiation of T helper type 2 cells. J Exp Med 190, 229-239.

### EXAMPLE 6

The expression of CEACAM1 and PD1 on cells was examined under physiological conditions. Lamina propria lymphocytes were isolated from colon, ileum, jejunum and duodenum as well as mesenteric lymph nodes (MLN) and spleen and after gating on CD3-positive cells stained for co-expression of PD1 and CEACAM1 (Figure 14).

Colitis was induced in mice by adoptive transfer of naive CD4+ T cells in Rag-deficient and CEACAM1-deficient animals and expression of CEACAM1 and PD1 was examined in lamina propria lymphocytes infiltrating the colon. Co-expression of CEACAM1 and PD1 is shown in Figure 15, after gating on CD3+, in lymphocytes obtained from proximal, middle and distal colon.

Tumor infiltrating lymphocytes (TILs) were isolated from subcutaneous tumors associated with CT26 colorectal carcinoma cell line. TILs were stimulated with anti-CD3 and anti-CD28 monoclonal antibodies in the presence of isotype control antibodies or antibodies that block different co-inhibitory cell surface molecules. This blockade of CEACAM1 and PD1 increases TNF-a production from tumor infiltrating lymphocytes (TIL). The data demonstrates a synergistic increase of TNF-a production by TILs treated with anti-CEACAM1 and anti-PD1 antibodies.

## Claims

1. A therapeutically effective amount of an anti-CEACAM1 antibody and an anti-PD1 antibody for use in therapy.

2. A therapeutically effective amount according to claim 1, wherein therapy comprises (i) enhancing an immune response and/or reducing T cell tolerance in a subject, or (ii) the treatment of a chronic disease.

3. A therapeutically effective amount of claim 2, wherein the chronic disease is selected from the group consisting of: cancer; a persistent infection; and a chronic viral infection.

4. A therapeutically effective amount of any preceding claim, wherein the anti-CEACAM1 antibody or the anti-PD1 antibody inhibits signalling mediated by the target.

5. A therapeutically effective amount of any preceding claim, wherein the anti-CEACAM1 antibody binds a CEACAM1 molecule having the sequence set forth in SEQ ID NO:32, or an allelic or splice variant of SEQ ID NO: 32.

6. A therapeutically effective amount of any preceding claim, wherein the anti-CEACAM1 antibody binds a CEACAM1 ligand interaction site, optionally wherein the anti-CEACAM1 antibody binds to an amino acid residue corresponding to any of amino acid residues 1-429 of SEQ ID NO: 32.

7. A therapeutically effective amount of any preceding claim, wherein the anti-CEACAM1 antibody binds an extracellular domain of CEACAM1, and/or wherein the anti-CEACAM1 antibody binds the homophilic interaction domain of CEACAM1 and inhibits homophilic interaction of CEACAM1 molecules.

8. A therapeutically effective amount of any preceding claim, wherein the anti-PD1 antibody binds a PD1 molecule having the sequence set forth in SEQ ID NO:1, or an allelic or splice variant of SEQ ID NO: 1.

9. A therapeutically effective amount of any preceding claim, wherein the anti-PD1 antibody binds a PD1 ligand interaction site, optionally wherein the PD1 antibody binds to an amino acid residue corresponding to any of amino acid residues 41-136 of SEQ ID NO: 1.

10. A composition comprising an anti-CEACAM1 antibody and an anti-PD1 antibody, for use in therapy.

11. A composition according to claim 10, wherein therapy comprises (i) enhancing an immune response and/or reducing T cell tolerance in a subject, or (ii) the treatment of a chronic disease, optionally wherein the chronic disease is selected from the group consisting of: cancer; a persistent infection; and a chronic viral infection.

## Patentansprüche

1. Therapeutisch wirksame Menge eines Anti-CEACAM1-Antikörpers und eines Anti-PD1-Antikörpers zur Verwendung bei der Therapie.

2. Therapeutisch wirksame Menge nach Anspruch 1, wobei die Therapie (i) das Verstärken einer Immunreaktion und/oder das Reduzieren einer T-Zell-Toleranz bei einem Subjekt oder (ii) die Behandlung einer chronischen Krankheit umfasst.

3. Therapeutisch wirksame Menge nach Anspruch 2, wobei die chronische Krankheit aus der Gruppe ausgewählt ist, bestehend aus: Krebs, einer persistierenden Infektion; und einer chronischen Virusinfektion.

4. Therapeutisch wirksame Menge nach einem der vorstehenden Ansprüche, wobei der Anti-CEACAM1-Antikörper oder der Anti-PD1-Antikörper eine durch das Ziel vermittelte Signalgebung inhibiert.

5. Therapeutisch wirksame Menge nach einem der vorstehenden Ansprüche, wobei der Anti-CEACAM1-Antikörper ein CEACAM1-Molekül mit der in SEQ ID NO:32 oder einer Allel- oder Spleißvariante von SEQ ID NO:32 dargelegten Sequenz bindet.

6. Therapeutisch wirksame Menge nach einem der vorstehenden Ansprüche, wobei der Anti-CEACAM1-Antikörper eine CEACAM1-Ligandeninteraktionsstelle bindet, wobei der Anti-CEACAM1-Antikörper wahlweise einen Aminosäurerest bindet, der einem der Aminosäurereste 1-429 von SEQ ID NO:32 entspricht.

7. Therapeutisch wirksame Menge nach einem der vorstehenden Ansprüche, wobei der Anti-CEACAM1-Antikörper eine extrazelluläre Domäne von CEACAM1 bindet und/oder wobei der Anti-CEACAM1-Antikörper die homophile Interaktionsdomäne des CEACAM1-Antikörpers bindet und die homophile Interaktion von CEACAM1-Molekülen inhibiert.

8. Therapeutisch wirksame Menge nach einem der vorstehenden Ansprüche, wobei der Anti-PD1-Antikörper ein PD1-Molekül mit der in SEQ ID NO:1 oder einer Allel- oder Spleißvariante von SEQ ID NO:1 dargelegten Sequenz bindet.

9. Therapeutisch wirksame Menge nach einem der vorstehenden Ansprüche, wobei der Anti-PD1-Antikörper eine PD1-Ligandeninteraktionsstelle bindet, wobei der PD1-Antikörper wahlweise einen Aminosäurerest bindet, der einem der Aminosäurereste 41-136 von SEQ ID NO:1 entspricht.

10. Zusammensetzung, umfassend einen Anti-CEACAM1-Antikörper und einen Anti-PD1-Antikörper zur Verwendung bei der Therapie.

11. Zusammensetzung nach Anspruch 10, wobei die Therapie (i) das Verstärken einer Immunreaktion und/oder das Reduzieren einer T-Zell-Toleranz bei einem Subjekt oder (ii) die Behandlung einer chronischen Krankheit umfasst, optional wobei die chronische Krankheit aus der Gruppe ausgewählt ist, bestehend aus: Krebs, einer persistierenden Infektion; und einer chronischen Virusinfektion.

## Revendications

1. Quantité thérapeutiquement efficace d'un anticorps anti-CEACAM1 et d'un anticorps anti-PD1 pour une utilisation en thérapie.

2. Quantité thérapeutiquement efficace selon la revendication 1, dans laquelle la thérapie comprend (i) l'amélioration d'une réponse immunitaire et/ou la réduction de la tolérance des lymphocytes T chez un sujet, ou (ii) le traitement d'une maladie chronique.

3. Quantité thérapeutiquement efficace selon la revendication 2, dans laquelle la maladie chronique est sélectionnée à partir du groupe constitué par : le cancer; une infection persistante ; et une infection virale chronique.

4. Quantité thérapeutiquement efficace selon l'une quelconque des revendications précédentes, dans laquelle l'anticorps anti-CEACAM1 ou l'anticorps anti-PD1 empêche le signalement diffusé par la cible.

5. Quantité thérapeutiquement efficace selon l'une quelconque des revendications précédentes, dans laquelle l'anticorps anti-CEACAM1 se lie à une molécule de CEACAM1 présentant la séquence exposée dans la SEQ ID N° : 32, ou un variant allélique ou d'épissage de la SEQ ID N° : 32.

6. Quantité thérapeutiquement efficace selon l'une quelconque des revendications précédentes, dans laquelle l'anticorps anti-CEACAM1 se lie à un site d'interaction de ligand de CEACAM1, facultativement dans laquelle l'anticorps anti-CEACAM1 se lie à un résidu d'acide aminé correspondant à l'un quelconque de résidus d'acide aminé de 1 à 429 de la SEQ ID N° : 32.

7. Quantité thérapeutiquement efficace selon l'une quelconque des revendications précédentes, dans laquelle l'anticorps anti-CEACAM1 se lie à un domaine extracellulaire de CEACAM1, et/ou dans laquelle l'anticorps anti-CEACAM1 se lie au domaine d'interaction homophile de CEACAM1 et empêche une interaction homophile de molécules de CEACAM1.

8. Quantité thérapeutiquement efficace selon l'une quelconque des revendications précédentes, dans laquelle l'anticorps anti-PD1 se lie à une molécule de PD1 présentant la séquence exposée dans la SEQ ID N° : 1, ou un variant allélique ou d'épissage de la SEQ ID N° : 1.

9. Quantité thérapeutiquement efficace selon l'une quelconque des revendications précédentes, dans laquelle l'anticorps anti-PD1 se lie à un site d'interaction de ligand de PD1, facultativement dans laquelle l'anticorps anti-PD1 se lie à un résidu d'acide aminé correspondant à l'un quelconque de résidus d'acide aminé de 41 à 136 de la SEQ ID N° : 1.

10. Composition comprenant un anticorps anti-CEACAM1 et un anticorps anti-PD1, pour une utilisation en thérapie.

11. Composition selon la revendication 10, dans laquelle la thérapie comprend (i) l'amélioration d'une réponse immunitaire et/ou la réduction de la tolérance des lymphocytes T chez un sujet, ou (ii) le traitement d'une maladie chronique, facultativement dans laquelle la maladie chronique est sélectionnée à partir du groupe constitué par : le cancer ; une infection persistante ; et une infection virale chronique.
